(19) Europäisches Patentamt
European Patent Office
Office européen des brevets

(11) Veröffentlichungsnummer: **0 024 282**
**B1**

# (12) EUROPÄISCHE PATENTSCHRIFT

(45) Veröffentlichungstag der Patentschrift: **18.05.83**

(21) Anmeldenummer: **80103099.0**

(22) Anmeldetag: **04.06.80**

(51) Int. Cl.³: **C 07 C 101/62,** C 07 C 103/76, C 07 C 149/18, C 07 D 295/14, C 07 D 295/08, C 07 D 209/28, C 07 D 213/74, A 61 K 31/215, A 61 K 31/395

## (54) Neue Benzoylderivate, deren Herstellung und deren Verwendung als Arzneimittel.

(30) Priorität: **30.06.79 DE 2926472**

(43) Veröffentlichungstag der Anmeldung: **04.03.81 Patentblatt 81/9**

(45) Bekanntmachung des Hinweises auf die Patenterteilung: **18.05.83 Patentblatt 83/20**

(84) Benannte Vertragsstaaten: **AT BE CH DE FR GB IT LI LU NL SE**

(56) Entgegenhaltungen:
**DE-A-2 535 799**
**FR-A-2 225 165**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 720, D-7950 Biberach (Riss) (DE)**

(72) Erfinder: **Keck, Johannes, Dr., Dipl.-Chem., Talfeldstrasse 27, D-7950 Biberach 1 (DE)**
Erfinder: **Krüger, Gerd, Dr., Dipl.-Chem., Ginsterhalde 30, D-7950 Biberach 1 (DE)**
Erfinder: **Pieper, Helmut, Dr., Dipl.-Chem., Kapellenweg 5, D-7950 Biberach 1 (DE)**
Erfinder: **Noll, Klaus, Dr., Dipl.-Chem., Im Schönblick 3, D-7951 Warthausen 1 (DE)**
Erfinder: **Engelhardt, Günther, Dr., Unterer Bühl 18, D-7950 Biberach 1 (DE)**
Erfinder: **Promberger, Norbert, Dr., Talfeldstrasse 57, D-7950 Biberach 1 (DE)**
Erfinder: **Zimmermann, Rainer, Dr., Dipl.-Biochem., Laurenbühlstrasse 17, D-7951 Mittelbiberach (DE)**

EP 0 024 282 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Benzoylderivate, deren Herstellung und deren Verwendung als Arzneimittel

In der DE-A Nr. 2535799 werden entzündungshemmende und antirheumatisch wirkende Verbindungen beschrieben, wobei als bevorzugte Verbindung N'-2-[1-(p-Chlorbenzoyl)-5-methoxy-2-methyl-3-indolacetoxy]äthyl-N-3-(N-benzoyl-N',N'-di-n-propyl-DL-isoglutaminyl)oxypropylpiperazin erwähnt wird.

Es wurde nun gefunden, dass die neuen Benzoylderivate der allgemeinen Formel

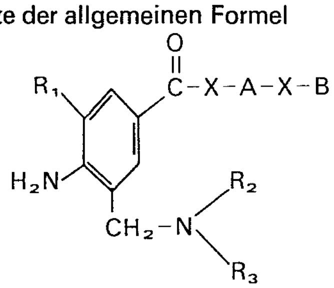

(I)

in welcher die Symbole die unten angegebenen Bedeutungen haben, und deren physiologisch verträgliche Säureadditionssalze mit anorganischen oder organischen Säuren bei überraschend geringen Nebenwirkungen ebenfalls eine entzündungshemmende Wirkung besitzen und somit überlegene therapeutische Eigenschaften aufweisen. Gegenstand der vorliegenden Erfindung sind somit die neuen Benzoylderivate der obigen allgemeinen Formel I, deren physiologisch verträgliche Säureadditionssalze mit anorganischen oder organischen Säuren und Verfahren zu ihrer Herstellung sowie die Verwendung der neuen Benzoylderivate und deren Salze als Arzneimittel.

In der obigen allgemeinen Formel I bedeuten

X ein Sauerstoffatom oder die Iminogruppe, wobei die beiden Reste X gleich oder verschieden sein können,

$R_1$ ein Chlor- oder Bromatom,

$R_2$ und $R_3$, die gleich oder verschieden sein können, Wasserstoffatome, geradkettige oder verzweigte Alkylgruppen mit 1 bis 6 Kohlenstoffatomen, welche durch eine Phenylgruppe oder eine Dialkylaminogruppe mit 1 bis 3 Kohlenstoffatomen in jedem Alkylteil substituiert sein können, Pyridyl- oder Cycloalkylgruppen mit 5 bis 7 Kohlenstoffatomen oder $R_2$ und $R_3$ zusammen mit dem dazwischenliegenden Stickstoffatom eine Pyrrolidino-, Piperidino-, Hexamethylenimino-, Morpholino-, N-Arylpiperazino- oder N-Alkylpiperazinogruppe, wobei die Alkylgruppe 1 bis 3 Kohlenstoffatome enthalten kann,

A eine Cycloalkylengruppe mit 5 bis 7 Kohlenstoffatomen oder eine Alkylengruppe mit 2 bis 10 Kohlenstoffatomen, die durch eine oder zwei Alkylgruppen mit jeweils 1 bis 3 Kohlenstoffatomen, durch eine oder zwei Carbalkoxygruppen mit insgesamt je 2 bis 4 Kohlenstoffatomen, durch eine oder zwei Phenylgruppen, durch 1 bis 4 Hydroxylgruppen, durch eine Halogenmethyl-, Hydroxymethyl-, Alkanoyloxy- oder Alkanoyloxymethylgruppe mit jeweils 1 bis 18 Kohlenstoffatomen im Alkanoylteil oder durch eine Gruppe der Formel

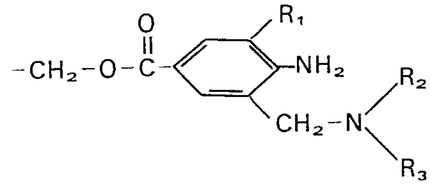

wobei $R_1$, $R_2$ und $R_3$ wie eingangs definiert sind, substituiert oder durch ein Sauerstoff- oder Schwefelatom, durch eine Sulfinyl-, Sulfonyl-, Phenylen-, Cyclohexylen-, Pyridindiyl-, Piperazindiyl- oder Iminogruppe unterbrochen sein kann, wobei die Iminogruppe durch eine Alkylgruppe mit 1 bis 6 Kohlenstoffatomen, durch eine Phenyl- oder Phenylalkylgruppe mit 1 bis 3 Kohlenstoffatomen im Alkylteil substituiert sein kann, und

B die 2-Acetoxybenzoyl-, 2-[(2,6-Dichlorphenyl)amino]phenylacetyl-, 1-(4-Chlorbenzoyl)-5-methoxy-2-methyl-[1H]-indol-3-acetyl-, 3-Benzoyl-α-methylphenylacetyl-, α-Methyl-4-(2-methylpropylphenylacetyl-, 2-Fluor-α-methyl-[1,1'-biphenyl]-4-acetyl- und (+)-6-Methoxy-α-methyl-2-naphthalinacetylgruppe.

Unter den bei der Definition der Reste $R_1$, $R_2$, $R_3$ und A erwähnten Bedeutungen kommt somit für

$R_1$ die Bedeutung des Chlor- oder Bromatoms, für die Gruppe

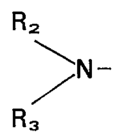

die Bedeutung der Amino-,
Methylamino-,
Äthylamino-,
n-Propylamino-,
Isopropylamino-,
n-Butylamino-,
tert.-Butylamino-,
n-Pentylamino-,
n-Hexylamino-,
Dimethylamino-,
Diäthylamino-,
Di-n-propylamino-,
Diisopropylamino-,
Di-n-butylamino-,
Di-n-pentylamino-,
Di-n-hexylamino-,
Äthylmethylamino-,
Methylpropylamino-,
Äthylpropylamino-,
Äthylbutylamino-,
Propylisopropylamino-,
Propylbutylamino-,
Methylcyclopentylamino-,
Methylcyclohexylamino-,
Methylcycloheptylamino-,
Äthylcyclopentylamino-,
Äthylcyclohexylamino-,
Äthylcycloheptylamino-,
Propylcyclopentylamino-,

Propylcyclohexylamino-,
Propylcycloheptylamino-,
Cyclopentylamino-,
Cyclohexylamino-,
Cycloheptylamino-,
Dicyclohexylamino-,
Benzylamino-,
Methylbenzylamino-,
Äthylbenzylamino-,
Cyclohexylbenzylamino-,
Dibenzylamino-,
Phenyläthylamino-,
Methylphenyläthylamino-,
Propylphenyläthylamino-,
Phenylpropylamino-,
2-(Dimethylamino)äthylamino-,
2-(Diäthylamino)äthylamino-,
2-(Dipropylamino)äthylamino-,
3-(Dimethylamino)propylamino-,
3-(Dipropylamino)propylamino-,
N-Methyl-2-(dimethylamino)äthylamino-,
N-Äthyl-2-(diäthylamino)äthylamino-,
Pyridylamino-,
Pyrrolidino-,
Piperidino-,
Hexamethylenimino-,
Morpholino-,
N-Methylpiperazino-,
N-Äthylpiperazino-,
N-Propylpiperazino-,
N-Isopropylpiperazino- oder
N-Phenylpiperazinogruppe, und

für A die der 1,2-Cyclopentylen-,
1,3-Cyclopentylen-,
1,2-Cyclohexylen-,
1,3-Cyclohexylen-,
1,4-Cyclohexylen-,
1,2-Cycloheptylen-,
1,3-Cycloheptylen-,
1,4-Cycloheptylen-,
Äthylen-,
n-Propylen-,
n-Butylen-,
n-Pentylen-,
n-Hexylen-,
n-Heptylen-,
n-Octylen-,
n-Nonylen-,
n-Decylen-,
1-Methyläthylen-,
2-Methyläthylen-,
1,2-Dimethyläthylen-,
1-Methylpropylen-,
2-Methylpropylen-,
3-Methylpropylen-,
1,2-Dimethylpropylen-,
1,3-Dimethylpropylen-,
2-Methyl-2-n-propylpropylen-,
1-Methylbutylen-,
1-Methylpentylen-,
1-Phenyläthylen-,
2-Phenyläthylen-,
1,2-Diphenyläthylen-,
1-Phenyl-n-propylen-,
1-Methoxycarbonyläthylen-,
1-Äthoxycarbonyläthylen-,
2-Äthoxycarbonyläthylen-,
1,2-Diäthoxycarbonyläthylen-,
1-Äthoxycarbonylpropylen-,
2-Äthoxycarbonylpropylen-,
3-Äthoxycarbonylpropylen-,
1-Propoxycarbonyläthylen-,
2-Propoxycarbonyläthylen-,
2-Hydroxypropylen-,
2-Hydroxybutylen-,
3-Hydroxybutylen-,
2,3-Dihydroxybutylen-,
2-Hydroxypentylen-,
2,3,4-Trihydroxypentylen-,
2,3,4,5-Tetrahydroxy-n-hexylen-,
1-Chlormethyläthylen-,
2-Chlormethyläthylen-,
1-Hydroxymethyläthylen-,
2-Hydroxymethyläthylen-,
2-Acetoxymethyläthylen-,
2-Pentanoyloxymethyläthylen-,
2-Decanoyloxymethyläthylen-,
2-Tetradecanoyloxymethyläthylen-,
2-Octadecanoyloxymethyläthylen-,
2-[4-Amino-3-brom-5-(N-cyclohexyläthyl-aminomethyl)benzoyloxymethyl]äthylen-,
2-Acetoxypropylen-,
2-Pentanoyloxypropylen-,
2-Decanoyloxypropylen-,
2-Tetradecanoyloxypropylen-,
2-Octadecanoyloxypropylen-,
Diäthylenoxid-,
Diäthylensulfid-,
Diäthylensulfoxid-,
Diäthylensulfonyl-,
N,N-Diäthylenamino-,
N,N-Diäthylenmethylamino-,
N,N-Diäthylenäthylamino-,
N,N-Diäthylenpropylamino-,
N,N-Diäthylenisopropylamino-,
N,N-Diäthylenbutylamino-,
N,N-Diäthylen-tert.-butylamino-,
N,N-Diäthylenhexylamino-,
N,N-Diäthylenphenylamino-,
N,N-Diäthylenbenzylamino-,
N,N-Diäthylenphenylpropylamino-,
Äthylenpropylenoxid-,
N-Äthylen-N-Propylenmethylamino-,
N-Äthylen-N-butylenäthylamino-,
Cyclohexandimethylen-,
Xylylen-,
Pyridindimethylen-,
N-N'-Diäthylenpiperazino- oder
N-Äthylen-N'-propylenpiperazinogruppe in Betracht.

Bevorzugte Verbindungen der allgemeinen Formel I sind jedoch diejenigen, in der

X ein Sauerstoffatom oder die Iminogruppe, wobei die beiden Reste X gleich oder verschieden sein können,

$R_1$ ein Chlor- oder Bromatom,

$R_2$ und $R_3$, die gleich oder verschieden sein können, Wasserstoffatome, Alkylgruppen mit 1 bis 4 Kohlenstoffatomen, Cycloalkylgruppen mit 5 bis 7 Kohlenstoffatomen, Benzyl- oder 2-Diäthyl-

aminoäthylgruppen oder $R_2$ und $R_3$ zusammen mit dem dazwischenliegenden Stickstoffatom die Pyrrolidino-, Piperidino-, Hexamethylenimino-, Morpholino-, N-Methylpiperazino- oder N-Phenylpiperazinogruppe, und

A eine geradkettige Alkylengruppe mit 2 bis 10 Kohlenstoffatomen, eine Äthylengruppe, die durch 1 oder 2 Methylgruppen, durch 1 oder 2 Alkoxycarbonylgruppen mit insgesamt je 2 oder 3 Kohlenstoffatomen, durch ein oder zwei Phenylgruppen, durch eine Hydroxy-, Chlormethyl-, Hydroxymethyl-, 4-Amino-3-brom-5-(N-äthylcyclohexylaminomethyl)benzoyloxymethyl- oder Alkanoyloxymethylgruppe mit 1 bis 18 Kohlenstoffatomen substituiert ist, eine Propylengruppe, die in 2-Stellung durch eine Hydroxygruppe oder eine Alkanoyloxygruppe mit 1 bis 18 Kohlenstoffatomen oder durch zwei Alkylgruppen mit jeweils 1 bis 3 Kohlenstoffatomen substituiert ist, eine durch 4 Hydroxygruppen substituierte n-Hexylengruppe, eine Cyclohexylengruppe oder eine geradkettige Alkylengruppe mit 4 bis 6 Kohlenstoffatomen, welche zwischen den C-Atomen 2 und 3 oder 3 und 4 durch ein Sauerstoff- oder Schwefelatom, durch eine Sulfinyl-, Sulfonyl-, Imino-, Phenylimino-, Benzylimino-, Piperazindiyl- oder eine Alkyliminogruppe mit 1 bis 4 Kohlenstoffatomen unterbrochen ist, eine 1,4-Cyclohexandimethylen-, p-Xylylen- oder 2,6-Pyridindimethylengruppe, und

B die 2-Acetoxybenzoyl-, 2-[(2,6-Dichlorphenyl)amino]phenylacetyl-, 1-(4-Chlorbenzoyl)-5-methoxy-2-methyl-[1H]-indol-3-acetyl-, 3-Benzoyl-α-methylphenylacetyl-, α-Methyl-4-(2-methylpropyl)phenylacetyl-, 2-Fluor-α-methyl-[1,1'-biphenyl]-4-acetyl- und (+)-6-Methoxy-α-methyl-2-naphthalinacetylgruppe bedeuten, und deren physiologisch verträgliche Säureadditionssalze mit anorganischen oder organischen Säuren.

Besonders bevorzugte Verbindungen der allgemeinen Formel I sind diejenigen, in der

X je ein Sauerstoffatom,

$R_1$ ein Bromatom,

$R_2$ die Methyl- oder Äthylgruppe,

$R_3$ die Äthyl- oder Cyclohexylgruppe oder $R_2$ und $R_3$ zusammen mit dem Stickstoffatom die Hexamethyleniminogruppe,

A eine geradkettige Alkylengruppe mit 2 bis 6 Kohlenstoffatomen oder eine geradkettige Alkylengruppe mit 4 bis 6 Kohlenstoffatomen, welche zwischen den C-Atomen 2 und 3 oder 3 und 4 durch ein Sauerstoff- oder Schwefelatom, durch eine Äthylimino- oder Propyliminogruppe unterbrochen ist, und

B die 1-(4-Chlorbenzoyl)-5-methoxy-2-methyl-[1H]-indol-3-acetyl-, 2-[(2,6-Dichlorphenyl)amino]phenylacetyl-, 3-Benzoyl-α-methylphenylacetyl-, (+)-6-Methoxy-α-methyl-2-naphthalinacetyl-, α-Methyl-4-(2-methylpropyl)phenylacetyl- oder 2-Fluor-α-methyl-[1,1'-biphenyl]-4-acetylgruppe bedeuten, und deren physiologisch verträgliche Salze mit anorganischen oder organischen Säuren.

Erfindungsgemäss erhält man die neuen Verbindungen der allgemeinen Formel I nach folgenden Verfahren:

Umsetzung einer Carbonsäure der allgemeinen Formel

$$B - OH \qquad (II)$$

bzw. ihrer Salze oder reaktionsfähigen Derivate mit einer Verbindung der allgemeinen Formel

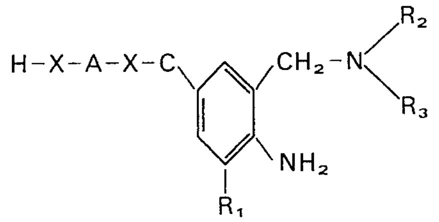

(III)

bzw. ihrer Alkalisalze oder reaktionsfähigen Derivate, oder

Umsetzung einer Carbonsäure der allgemeinen Formel

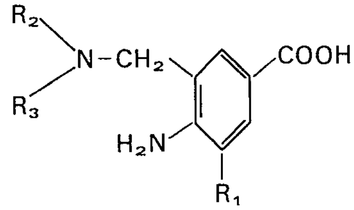

(IV)

bzw. ihrer Salze oder reaktionsfähigen Derivate mit einer Verbindung der allgemeinen Formel

$$H - X - A - X - B \qquad (V)$$

ihrer Alkalisalze oder reaktionsfähigen Derivate, in denen $R_1$ bis $R_3$, A, B und X wie eingangs definiert sind und die Gruppe HX zusammen mit den benachbarten α- und β-ständigen C-Atomen des Restes A auch eine Oxiranylgruppe darstellen kann.

Die Umsetzung betrifft somit die Acylierung einer Verbindung der allgemeinen Formel III oder V mit einer Carbonsäure der allgemeinen Formel II oder IV oder mit deren funktionellen Derivaten, gegebenenfalls in Gegenwart eines säureaktivierenden und/oder wasserentziehenden Mittels, die Umsetzung einer Carbonsäure der allgemeinen Formel II oder IV mit einem reaktionsfähigen Derivat einer Verbindung der allgemeinen Formel III oder V oder die Alkylierung eines Salzes einer Carbonsäure der allgemeinen Formel II oder IV mit einem entsprechenden funktionellen Derivat einer Verbindung der allgemeinen Formel III oder V.

Als funktionelle Derivate einer Carbonsäure der allgemeinen Formel II oder IV kommen beispielsweise deren Alkyl-, Aryl- oder Aralkylester wie der Methyl-, Äthyl-, Phenyl- oder Benzylester, deren Imidazolide, deren Säurehalogenide wie das Säurechlorid oder Säurebromid, deren Anhydride, deren gemischte Anhydride mit aliphatischen oder aromatischen Carbonsäuren oder Kohlensäureestern, z.B. der Essigsäure, Propionsäure oder des Kohlensäureäthylesters, deren Acyloxytriphenylphosphoniumsalze, deren N-Acyloxyimide oder, falls die Aminogruppe in 2-Stellung einer entsprechenden Carbonsäure der allgemeinen Formel IV steht, auch deren Isatosäureanhydride, als reaktionsfähige Derivate einer Verbindung der allgemeinen Formel III oder V deren Phosphazoderivate, falls X eine Iminogruppe und ein Sauerstoffatom darstellt, deren Epoxide, Halogenide wie das Chlorid, Bromid oder Jodid, deren Sulfonsäureester wie die der Methansulfonsäure oder p-Toluolsulfonsäure oder deren Ester mit aliphatischen oder aromatischen Carbonsäuren wie die der Essigsäure, Propionsäure oder Benzoesäure und als Salze einer Carbonsäure der allgemeinen Formel II oder IV deren Alkali- und Erdalkalisalze wie das Natrium-, Kalium- oder Calciumsalz oder deren Silbersalz in Betracht.

Als wasserentziehende und/oder säureaktivierende Mittel kommen beispielsweise ein Chlorameisensäureester wie Chlorameisensäureäthylester, Thionylchlorid, Phosphortrichlorid, Phosphorpentoxid, N,N'-Dicyclohexylcarbodiimid, N,N'-Carbonyldiimidazol, N,N'-Thionyldiimidazol oder Bortrifluoridätherat in Betracht.

Die Umsetzung wird zweckmässigerweise in einem Lösungsmittel wie Dichlormethan, Chloroform, Tetrachlorkohlenstoff, Äther, Dioxan, Tetrahydrofuran, Benzol, Toluol, Dimethylformamid oder Methanol gegebenenfalls in Gegenwart einer anorganischen Base wie Natriumcarbonat oder einer tertiären organischen Base wie Triäthylamin, Pyridin und/oder 4-Dimethylaminopyridin, welche gleichzeitig auch als Lösungsmittel dienen können, gegebenenfalls in Gegenwart eines säureaktivierenden Mittels bei Temperaturen zwischen −25 und 250°C, vorzugsweise jedoch bei Temperaturen zwischen −10°C und der Siedetemperatur des verwendeten Lösungsmittels, durchgeführt. Hierbei braucht ein gegebenenfalls *in situ* entstandenes funktionelles Derivat einer Verbindung der allgemeinen Formeln II bis V nicht isoliert zu werden, ferner kann die Umsetzung auch ohne Lösungsmittel und/oder in Gegenwart eines Reaktionsbeschleunigers wie Natriumhydrid oder 4-Dimethylaminopyridin durchgeführt werden. Des weiteren kann während der Umsetzung entstehendes Wasser durch azeotrope Destillation, z.B. durch Erhitzen mit Toluol am Wasserabscheider, gegebenenfalls in Gegenwart eines Trockenmittels wie Magnesiumsulfat oder Molekularsieb, abgetrennt werden.

Besonders vorteilhaft lässt sich jedoch die Umsetzung eines Alkalisalzes einer Carbonsäure der allgemeinen Formel II oder IV mit einer Verbindung der allgemeinen Formel III oder V, in der HX ein Halogenatom wie das Chlor- oder Bromatom darstellt, in Gegenwart eines Reaktionsbeschleunigers wie ein Alkalijodid, z.B. Natrium- oder Kaliumjodid, oder in Gegenwart eines Phasentransferkatalysators wie ein sogenannter Kronenäther, z.B. 15-Krone-5, durchführen.

Die erfindungsgemäss erhaltenen Verbindungen der allgemeinen Formel I, in der die Gruppe A eine Hydroxylgruppe enthält, lassen sich anschliessend mittels Acylierung in die entsprechenden Acyloxyverbindungen der allgemeinen Formel I und die Verbindungen der allgemeinen Formel I, in der die Gruppe A eine durch ein Schwefelatom unterbrochene Alkylengruppe darstellt, mittels Oxidation in die entsprechenden Sulfinyl- oder Sulfonylverbindungen der allgemeinen Formel I und die Verbindungen der allgemeinen Formel I, in der die Gruppe A eine durch eine Sulfinylgruppe unterbrochene Alkylengruppe darstellt, mittels Oxidation in die entsprechenden Sulfonylverbindungen überführen.

Die nachträgliche Acylierung wird zweckmässigerweise in einem Lösungsmittel wie Dimethylformamid, Tetrahydrofuran, Toluol oder Methylenchlorid mit einer entsprechenden Carbonsäure oder deren reaktionsfähigen Derivaten wie deren Anhydride, Säurehalogenide, 1-Imidazolylderivate oder mit deren gemischten Anhydriden mit Carbonsäuren oder Kohlensäureestern gegebenenfalls in Gegenwart eines säureaktivierenden und/oder wasserentziehenden Mittels, z.B. Chlorameisensäureäthylester, Thionylchlorid, N,N'-Dicyclohexylcarbodiimid oder N,N'-Carbonyldiimidazol, und gegebenenfalls in Gegenwart einer anorganischen Base wie Natriumcarbonat oder tertiären organischen Base wie Triäthylamin oder Pyridin, welche gleichzeitig auch als Lösungsmittel dienen können, bei Temperaturen zwischen −25 und 250°C, vorzugsweise jedoch bei Temperaturen zwischen −10°C und der Siedetemperatur des verwendeten Lösungsmittels, durchgeführt.

Die nachträgliche Oxidation wird vorzugsweise in einem Lösungsmittel, z.B. Wasser, Wasser/Pyridin, Eisessig oder Methanol, je nach dem verwendeten Oxidationsmittel zweckmässigerweise bei Temperaturen zwischen −80 und 100°C durchgeführt.

Zur Herstellung der Sulfoxide der allgemeinen Formel I wird die nachträgliche Oxidation zweckmässigerweise mit einem Äquivalent des verwendeten Oxidationsmittels durchgeführt, z.B. mit Wasserstoffperoxid in Eisessig bei 0 bis 20°C, mit einer Persäure wie Peressigsäure, m-Chlorperbenzoesäure oder Peroxytrifluoressigsäure bei 0 bis 50°C, mit Natriummetaperjodat in wässerigem Methanol oder Äthanol bei 15 bis 25°C, mit tert.-Butylhypochlorit in Methanol bei −80 bis −30°C, mit Jodbenzoldichlorid in wässerigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei −70°C, der hierbei erhaltene Thioäther/Chlor-Komplex wird zweckmässigerweise mit wässerigem Äthanol hydrolysiert.

Zur Herstellung der Sulfone der allgemeinen Formel I wird die nachträgliche Oxidation zweckmässigerweise mit einem bzw. mit zwei Äquivalenten des verwendeten Oxidationsmittels durchgeführt, z.B. mit Wasserstoffperoxid in Eisessig bei 20 bis 100°C, mit einer Persäure wie Peressigsäure, m-Chlorperbenzoesäure oder Peroxytrifluoressigsäure bei Temperaturen zwischen 0 und 50°C, mit Salpetersäure in Eisessig bei 0 bis 20°C, mit Chromsäure oder Kaliumpermanganat in Eisessig, Wasser/Schwefelsäure oder in Aceton bei 0 bis 20°C.

Des weiteren lassen sich die neuen Benzoylderivate der allgemeinen Formel I mit anorganischen oder organischen Säuren in ihre physiologisch

verträglichen Säureadditionssalze mit 1 bis 3 Äquivalenten der betreffenden Säure überführen. Als Säuren haben sich beispielsweise Salzsäure, Bromwasserstoffsäure, Schwefelsäure, Phosphorsäure, Milchsäure, Zitronensäure, Weinsäure, Maleinsäure oder Fumarsäure als geeignet erwiesen.

Die als Ausgangsstoffe verwendeten Verbindungen der allgemeinen Formeln II bis V sind teilweise literaturbekannt bzw. können nach an sich bekannten Verfahren hergestellt werden. So werden beispielsweise die Benzoesäuren der allgemeinen Formel IV bzw. deren Herstellung in der GB-PS Nr. 1469187 beschrieben; die Verbindungen der allgemeinen Formeln III (siehe DE-OS Nr. 2926471, angemeldet am 30.06.1979) und V erhält man durch Veresterung bzw. Amidierung der entsprechenden Carbonsäuren mit entsprechenden Verbindungen.

Wie bereits eingangs erwähnt, weisen die neuen Verbindungen der allgemeinen Formel I sowie deren physiologisch verträgliche Salze mit anorganischen oder organischen Säuren bei überraschend geringen Nebenwirkungen wertvolle pharmakologische Eigenschaften auf, insbesondere eine entzündungshemmende Wirkung.

Beispielsweise wurden die Substanzen

A = 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid,

B = 1-(4-Amino-3-brom-5-diäthylaminomethylbenzoyloxy)-2-[1-(4-chlorbenzoyl)-5-methoxy-2-methyl-[1H]-indol-3-acetoxy]-äthanhydrochlorid,

C = 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid,

D = 1-[4-Amino-3-brom-5-(N-äthylcyclohexylaminomethyl)benzoyloxy]-6-{2-[(2,6-dichlorphenyl)amino]phenylacetoxy}-n-hexanhydrochlorid,

E = N-{2-[4-Amino-3-brom-5-(N-äthylcyclohexylaminomethyl)benzoyloxy]äthyl}-N-[2-{2-[(2,6-dichlorphenyl)amino]phenylacetoxy}-äthyl]äthylamindihydrochlorid,

F = 2-[4-Amino-3-brom-5-(N-äthylcyclohexylaminomethyl)benzoyloxy]-2'-{2-[(2,6-dichlorphenyl)amino]phenylacetoxy}diäthyloxidhydrochlorid,

G = 2-[4-Amino-3-brom-5-(N-äthylcyclohexylaminomethyl)benzoyloxy]-2'-{2-[(2,6-dichlorphenyl)amino]phenylacetoxy}diäthylsulfidhydrochlorid,

H = 1-[4-Amino-3-brom-5-(N-äthylcyclohexylaminomethyl)benzoyloxy]-2-(3-benzoyl-α-methylphenylacetoxy)äthanhydrochlorid,

I = N-{2-[4-Amino-3-brom-5-(N-äthylcyclohexylaminomethyl)benzoyloxy]äthyl}-N-[2-(3-benzoyl-α-methylphenylacetoxy)äthyl]-n-propylamin,

J = 1-[4-Amino-3-brom-5-(N-äthylcyclohexylaminomethyl)benzoyloxy]-3-[(+)-6-methoxy-α-methyl-2-naphtalinacetoxy]-n-propan,

K = 2-[4-Amino-3-brom-5-(N-äthylcyclohexylaminomethyl)benzoyloxy]-2'-[(+)-6-methoxy-α-methyl-2-naphthalinacetoxy]diäthylsulfid,

L = 2-[4-Amino-3-brom-5-(N-äthylcyclohexylaminomethyl)benzoyloxy]-2'-[α-methyl-4-(2-methylpropyl)phenylacetoxy]diäthyloxyd, und

M = N-{2-[4-Amino-3-brom-5-(N-äthylcyclohexylaminomethyl)benzoyloxy]äthyl}-N-{2-[α-methyl-4-(2-methylpropyl)phenylacetoxy]äthyl}-n-propylamin

auf ihre biologischen Eigenschaften wie folgt untersucht:

*1) Antiphlogistische Wirkung:*

*Methodik:*

An Ratten (eigener Zucht) beider Geschlechter mit einem Gewicht von 120-150 g wurde entsprechend den Angaben von Winter *et al.* [„J. Pharmacol.", *141*, 369 (1963)] durch die subplantare Injektion von 0,05 ml einer 1%igen Lösung von Karrageein in 0,85%iger NaCl-Lösung ein Ödem ausgelöst.

Die Prüfsubstanzen wurden 60 min vor Ödemprovokation als Verreibung in 1%iger Methylzellulose (1 ml/100 g Tier) per Schlundsonde beigebracht. Die Messung der Pfotenschwellung erfolgte 3 h nach der Ödemauslösung mit der von Doepfner und Cerletti [„Int. Arch. Allergy appl. Immunol.", *12*, 89 (1958)] angegebenen Technik.

Aus der mit den verschiedenen Dosen der Prüfsubstanzen erzielten Änderung der Schwellungswerte wurde nach linearer Regressionsanalyse nach Linder [„Statistische Methoden", 4. Aufl., S. 148-162, Birkhäuser, Basel (1964)] eine $ED_{35}$ nach Fieller [„Quart. J. Pharm. Pharmacol.", *17*, 117-123 (1944)] als die Dosis berechnet, die zu einer 35%igen Abschwächung des Schwellungswertes führte.

Die nachfolgende Tabelle enthält die gefundenen Werte:

| Substanz | $n_1$ | $n_2$ | $ED_{35}$ (mg/kg) |
|---|---|---|---|
| A | 4 | 10 | 84,7 |
| B | 4 | 10 | 35,5 |
| C | 4 | 10 | 9,9 |
| D | 3 | 10 | 29,9 |
| E | 4 | 10 | 18,9 |
| F | 3 | 10 | 49,2 |
| G | 3 | 10 | 6,7 |
| H | 5 | 10 | 15,8 |
| I | 3 | 10 | 2,7 |
| J | 3 | 10 | 36,1 |
| K | 3 | 10 | 36,2 |
| L | 4 | 10 | 24,7 |
| M | 3 | 10 | 53,3 |

$n_1$ = Anzahl der geprüften Dosen
$n_2$ = Anzahl der Tiere/Dosis

*2) Orientierende Toxizität:*

Ratten (eigener Zucht) beider Geschlechter erhielten die Prüfsubstanzen in einer Dosierung von 100 mg/kg als Verreibung in 1%iger Methylzellulose (1 ml/100 g Tieren) per Schlundsonde beigebracht. Die Tiere wurden 24 h nachbeobachtet. Die nachfolgende Tabelle enthält die gefundenen Werte:

| Substanz | Dosis (mg/kg) | n | Anteil der Tiere mit einer Symptomatik | Anteil der verstorbenen Tiere |
|---|---|---|---|---|
| A | 100 | 10 | 0/10 | 0/10 |
| B | 100 | 10 | 0/10 | 0/10 |
| C | 100 | 10 | 0/10 | 0/10 |
| D | 100 | 10 | 0/10 | 0/10 |
| E | 100 | 10 | 0/10 | 0/10 |
| F | 100 | 10 | 0/10 | 0/10 |
| G | 100 | 10 | 0/10 | 0/10 |
| H | 100 | 10 | 0/10 | 0/10 |
| I | 100 | 10 | 0/10 | 0/10 |
| J | 100 | 10 | 0/10 | 0/10 |
| K | 100 | 10 | 0/10 | 0/10 |
| L | 100 | 10 | 0/10 | 0/10 |
| M | 100 | 10 | 0/10 | 0/10 |

Aufgrund ihrer pharmakologischen Eigenschaften eignen sich die erfindungsgemäss hergestellten Verbindungen der allgemeinen Formel I sowie deren physiologisch verträgliche Salze mit anorganischen oder organischen Säuren zur Behandlung von entzündlichen Prozessen wie Periarthritis humeroscapularis, Bursitis, Synovitis, Tendinitis, Tendovaginitis, Gicht, Polyarthritis, Arthrosis deformans, Spondylitis ankylopoetica oder Lumbalgien und lassen sich hierzu, gegebenenfalls in Kombination mit anderen Wirksubstanzen, in die üblichen pharmazeutischen Zubereitungsformen wie Tabletten, Dragées, Kapseln, Suppositorien, Suspensionen, Salben oder Cremes einarbeiten. Die Einzeldosis am Menschen beträgt 100-600 mg, vorzugsweise jedoch 300-450 mg, 1 bis 3 × täglich.

Die nachfolgenden Vorschrifte und Beispiele sollen die Herstellung der Ausgangsstoffe bzw. die Erfindung näher erläutern:

*Vorschrift A*

*1-[4-Amino-3-brom-5-(N-äthylcyclohexylaminomethyl)benzoyloxy]-2,3-dihydroxy-n-propan*

70 g (0,185 mol) 4-Amino-3-brom-5-(N-äthylcyclohexylaminomethyl)benzoesäurenatriumsalz werden in 250 ml 1-Chlor-2,3-dihydroxy-n-propan suspendiert und unter Rühren 3 h lang auf 110-120°C erhitzt. Danach wird die Hauptmenge des überschüssigen 1-Chlor-2,3-dihydroxy-n-propans bei einem Druck von 0,02 mmHg abdestilliert, der Rückstand in einem Gemisch aus Methylenchlorid/Methanol/konz. Ammoniak (90/10/1) gelöst und über Kieselgel chromatographiert. Das erhaltene zähflüssige Öl wird in 130 ml Isopropanol gelöst und nach Verdünnen mit 200 ml Essigsäureäthylester mit ätherischer Salzsäure in das Hydrochlorid übergeführt, welches bei 0-5°C innerhalb von 24 h kristallisiert. Die Kristalle werden abgesaugt, mit wenig Äther gewaschen und getrocknet.

Schmelzpunkt des Hydrochlorids: 167-173°C

*Vorschrift B*

*1-[4-Amino-3-brom-5-(N-äthylcyclohexylaminomethyl)benzoyloxy]-2-hydroxyäthan*

41 g (0,1 mol) 4-Amino-3-brom-5-(N-äthylcyclohexylaminomethyl)benzoylchloridhydrochlorid werden in 250 ml Äthylenglykol gelöst und nach Zugabe von 17 g (0,22 mol) Pyridin 1 h auf 105°C erwärmt. Nach Abkühlen auf Raumtemperatur wird mit 1 l Wasser verdünnt, nach Zugabe von Natronlauge mit Äther extrahiert, die Ätherlösung über Natriumsulfat getrocknet und zur Trockene eingeengt. Der Rückstand wird über Kieselgel chromatographiert (Essigsäureäthylester). Durch Eindampfen des Eluats erhält man ein Harz, das beim Verreiben mit Petroläther kristallisiert und aus Äthanol umkristallisiert wird.

Schmelzpunkt: 72-75°C

*Vorschrift C*

*1-[4-Amino-3-brom-5-(N-äthylcyclohexylaminomethyl)benzamido]-3-hydroxy-n-propan*

30 g (0,073 mol) 4-Amino-3-brom-5-(N-äthylcyclohexylaminomethyl)benzoylchloridhydrochlorid werden in 400 ml Tetrahydrofuran gelöst und langsam zu einer Lösung von 15 g (0,2 mol) 3-Amino-n-propanol in 300 ml Tetrahydrofuran getropft. Das Gemisch wird ½ h bei 50°C gerührt und dann im Vakuum eingeengt. Der Rückstand wird zwischen Wasser und Chloroform verteilt, die Chloroformphase abgetrennt, getrocknet und eingedampft. Der Rückstand wird in Isopropanol gelöst, mit äthanolischer Salzsäure angesäuert und mit Äther verdünnt. Das Hydrochlorid wird abgesaugt und getrocknet.

Schmelzpunkt des Hydrochlorids: 110-113°C

*Vorschrift D*

*1-[4-Amino-3-brom-5-(N-äthylcyclohexylaminomethyl)benzamido]-4-hydroxy-n-butan*

27,5 g (0,067 mol) 4-Amino-3-brom-5-(N-äthylcyclohexylaminomethyl)benzoylchloridhydrochlorid werden in 400 ml Tetrahydrofuran gelöst und langsam zu einer Lösung von 12 g (0,135 mol) 4-Aminobutanol getropft. Das Gemisch wird ½ h bei 50°C gerührt und dann im Vakuum eingeengt. Der Rückstand wird zwischen Wasser und Chloroform verteilt, die Chloroformphase abgetrennt, getrocknet und eingedampft. Der Rückstand wird durch Chromatographie an Kieselgel [Chloroform/Methanol (9/1)] gereinigt. Beim Eindampfen des Eluats erhält man ein Öl.

IR-Spektrum (Methylenchlorid):
Amid-CO 1650 $cm^{-1}$
UV-Spektrum (Äthanol): $\lambda_{max.}$285 nm

*Vorschrift E*

*1-[4-Amino-3-brom-5-(N-äthylcyclohexylaminomethyl)benzoyloxy]-2,3-dihydroxy-n-propan*

2,1 g (0,05 mol) 1-[4-Amino-3-brom-5-(N-äthylcyclohexylaminomethyl)benzoyloxy-2,3-epoxy-n-propan werden in 100 ml Wasser und 100 ml Methanol gelöst. Die erhaltene Lösung wird mit Schwefelsäure auf pH 2-3 eingestellt und nach Zusatz von ca. 20 mg Eisen(III)chlorid 2 h am Rückfluss gekocht. Nach Abkühlen wird neutralisiert, im Vakuum zur Trockne eingeengt und der Rückstand durch Chromatographie an Kieselgel [Methylenchlorid/Methanol/konz. Ammoniak (90/10/1)] gereinigt. Das erhaltene Öl wird in Isopropanol gelöst und durch Zugabe von ätherischer Salzsäure und Essigsäureäthylester als Hydrochlorid zur Kristallisation gebracht.

Schmelzpunkt des Hydrochlorids: 167–173°C

*Vorschrift F*

*1-(4-Amino-3-brom-5-diäthylaminomethylbenzoyloxy)-6-chlor-n-hexan*

5,2 g (0,013 mol) 1-(4-Amino-3-brom-5-diäthylaminomethylbenzoyloxy)-6-hydroxy-n-hexan werden in 15 ml Chloroform gelöst und mit 15 ml Thionylchlorid 3 h bei 60°C gerührt. Das Reaktionsgemisch wird eingedampft und der Rückstand in Chloroform aufgenommen. Die organische Phase wird mit Natriumbicarbonatlösung gewaschen, getrocknet und eingedampft. Der erhaltene Rückstand wird in Isopropanol gelöst und das Hydrochlorid mit ätherischer Salzsäure ausgefällt.

Schmelzpunkt des Hydrochlorids: 121-122°C

*Vorschrift G*

*N-[4-Amino-3-brom-5-(N-äthylcyclohexylaminomethyl)benzoyl]-L-serinmethylester*

Eine Lösung von 27,4 g (0,07 mol) 4-Amino-3-brom-5-(N-äthylcyclohexylaminomethyl)-benzoesäurehydrochlorid in 250 ml Dimethylformamid wird unter Rühren und bei Raumtemperatur portionsweise mit 13 g (0,08 mol) N,N'-Carbonyldiimidazol versetzt. Nach beendeter Zugabe wird auf 50°C erwärmt und diese Temperatur 1 h lang beibehalten. Nach Abkühlen auf Raumtemperatur gibt man unter weiterem Rühren eine Lösung von 12,5 g (0,08 mol) L-Serinmethylesterhydrochlorid und 8,1 g (0,08 mol) Triäthylamin in 100 ml Dimethylformamid zu und rührt weitere 16 h. Anschliessend entfernt man das Lösungsmittel im Vakuum und verteilt den Rückstand zwischen Wasser und Chloroform. Die Chloroformphase wird mit Wasser gewaschen, über Natriumsulfat getrocknet und im Vakuum eingeengt. Es bleibt ein hellgelbes Öl zurück, das über Kieselgel chromatographiert [500 g Kieselgel, Chloroform/Essigsäureäthylester (3/1)] wird. Man erhält einen farblosen Schaum.

IR-Spektrum (Methylenchlorid):

| | |
|---|---|
| OH | 3600 $cm^{-1}$ |
| $NH_2$ | 3440 $cm^{-1}$ |
| Ester-CO | 1745 $cm^{-1}$ |
| Amid-CO | 1675 $cm^{-1}$ |

UV-Spektrum (Äthanol): $\lambda_{max.}$230 nm (Schulter), 290 nm

*Vorschrift H*

*1-[4-Amino-3-brom-5-(N-äthylcyclohexylaminomethyl)benzoyl]-D-mannit*

8,25 g (8,9 mmol) 1,6-Bis-[4-Amino-3-brom-5-(N-äthylcyclohexylaminomethyl)benzoyl]-D-mannitdihydrochlorid werden in 250 ml Tetrahydrofuran und 100 ml Wasser gelöst. Bei Raumtemperatur wird unter Rühren eine Lösung von 13,5 ml 2N-Natronlauge in 100 ml Tetrahydrofuran hinzugetropft. Die Reaktionslösung wird 2½ h bei Raumtemperatur gerührt, nach dünnschichtchromatographischer Auswertung mit 300 ml Äther versetzt und nach kurzem Schütteln die entstehenden Phasen getrennt. Anschliessend wird die organische Phase mit 2N-Salzsäure extrahiert, die wässerig salzsauren Extrakte mit konzentriertem Ammoniak alkalisch gemacht und mit Äther extrahiert. Die Ätherextrakte werden mit Wasser gewaschen, über Magnesiumsulfat getrocknet und im Vakuum eingeengt. Nach anschliessender Chromatographie des Eindampfrückstandes über Kieselgel [Chloroform/Methanol/konz. Ammoniak (9/1/0,1)] werden die erhaltenen Fraktionen im Vakuum zur Trockene eingeengt, der Eindampfrückstand in 150 ml Essigsäureäthylester gelöst und der Lösung der berechnete Anteil einer ätherischen Salzsäure hinzugegeben. Das erhaltene Hydrochlorid wird nach dem Absaugen aus Methanol/Essigsäureäthylester umkristallisiert.

Schmelzpunkt des Hydrochlorids: 153-160°C (Zers.)

*Vorschrift I*

*1-[4-Amino-3-brom-5-(N-äthylcyclohexylaminomethyl)benzoyloxy]-2,3-dihydroxy-n-propan*

9,8 g (0,025 mol) 4-Amino-3-brom-5-(N-äthylcyclohexylaminomethyl)benzoesäurehydrochlorid werden in 10 g (0,135 mol) 2,3-Epoxi-n-propanol suspendiert und nach Zusatz von 250 mg Eisen(III)chlorid 3 h auf 60°C erwärmt. Das Reaktionsgemisch wird zwischen Wasser und Chloroform verteilt, die Chloroformphase wird abgetrennt, getrocknet und zur Trockene eingeengt. Der Rückstand wird durch Chromatographie an Kieselgel [Methylenchlorid/Methanol/konz. Ammoniak (90/10/1)] gereinigt. Das Eluat wird eingeengt, der Rückstand in Isopropanol gelöst, und das Hydrochlorid mit äthanolischer Salzsäure/Essigsäureäthylester zur Kristallisation gebracht.

Schmelzpunkt des Hydrochlorids: 167-173°C

Analog den vorstehenden Vorschriften wurden folgende Verbindungen hergestellt:

1-[4-Amino-3-brom-5-(N-äthylcyclohexylaminomethyl)benzoyloxy]-2-chloräthan

Schmelzpunkt des Hydrochlorids: 162-164°C

1-[4-Amino-3-brom-5-(N-cyclohexyl-n-propylaminomethyl)benzoyloxy]-5-hydroxy-n-pentan

Schmelzpunkt des Hydrochlorids: 140-142°C

1-[4-Amino-3-brom-5-(N-äthylcyclohexylaminomethyl)benzoyloxy]-3-chlor-n-propan

Schmelzpunkt des Hydrochlorids: 185-187°C

1-[4-Amino-3-brom-5-(N-äthylcyclohexylaminomethyl)benzoyloxy]-4-chlor-n-butan

Schmelzpunkt des Hydrochlorids: 158-160°C

1-[4-Amino-3-brom-5-(N-äthylcyclohexylaminomethyl)benzoyloxy]-5-chlor-n-pentan

Schmelzpunkt des Hydrochlorids: 141-142,5°C

1-[4-Amino-3-brom-5-(N-äthylcyclohexylaminomethyl)benzoyloxy]-7-chlor-n-heptan

Schmelzpunkt des Hydrochlorids: 129-131°C

1-[4-Amino-3-brom-5-(N-äthylcyclohexylaminomethyl)benzoyloxy]-8-chlor-n-octan

Schmelzpunkt des Hydrochlorids: 114-117°C

1-[4-Amino-3-brom-5-(N-äthylcyclohexylaminomethyl)benzoyloxy]-3-hydroxy-n-propan

Schmelzpunkt des Hydrochlorids: 184-185°C

1-[4-Amino-3-brom-5-(N-äthylcyclohexylaminomethyl)benzoyloxy]-4-hydroxy-n-butan

Schmelzpunkt des Hydrochlorids: 148-150°C

1-[4-Amino-3-brom-5-(N-äthylcyclohexylaminomethyl)benzoyloxy]-5-hydroxy-n-pentan

Schmelzpunkt des Hydrochlorids: 158-160°C

1-[4-Amino-3-brom-5-(N-äthylcyclohexylaminomethyl)benzoyloxy]-6-hydroxy-n-hexan

Schmelzpunkt des Hydrochlorids: 98-101°C

1-[4-Amino-3-brom-5-(N-äthylcyclohexylaminomethyl)benzoyloxy]-7-hydroxy-n-heptan

Schmelzpunkt des Hydrochlorids: 102-104°C (Zers.)

1-[4-Amino-3-brom-5-(N-äthylcyclohexylaminomethyl)benzoyloxy]-8-hydroxy-n-octan

Schmelzpunkt des Hydrochlorids: 99-103°C

1-[4-Amino-3-brom-5-(N-äthylcyclohexylaminomethyl)benzoyloxy]-4-hydroxycyclohexan

IR-Spektrum (Methylenchlorid):

Ester-CO 1700 $cm^{-1}$

UV-Spektrum (Äthanol): $\lambda_{max.}$ 230 nm, 298-300 nm

1-[4-Amino-3-brom-5-(N-äthylcyclohexylaminomethyl)benzoyloxy]-2-hydroxy-n-propan

IR-Spektrum (Methylenchlorid):

Ester-CO 1710 $cm^{-1}$

UV-Spektrum (Äthanol): $\lambda_{max.}$ 230 nm (Schulter), 297 nm.

2-[4-Amino-3-brom-5-(N-äthylcyclohexylaminomethyl)benzoyloxy]-3-hydroxy-n-butan

IR-Spektrum (Methylenchlorid):

Ester-CO 1705 $cm^{-1}$

UV-Spektrum (Äthanol): $\lambda_{max.}$ 230 nm (Schulter), 299 nm

1-[4-Amino-3-brom-5-(N-äthylcycloheptylaminomethyl)benzoyloxy]-5-hydroxy-n-pentan

Schmelzpunkt des Hydrochlorids: 162-163°C

2-[4-Amino-3-brom-5-(N-äthylcyclohexylaminomethyl)benzoyl]-(+)-weinsäurediäthylester

$C_{24}H_{35}BrN_2O_7$ (543,5)

| | | | | |
|---|---|---|---|---|
| berechnet: | C 53,04 | H 6,49 | Br 14,70 | N 5,15 |
| gefunden: | C 53,30 | H 6,59 | Br 14,55 | N 5,12 |

1-[4-Amino-3-brom-5-(N-äthylcyclohexylaminomethyl)benzoyloxy]-2,3-epoxi-n-propan

IR-Spektrum (Methylenchlorid):

Ester-CO 1705 $cm^{-1}$

UV-Spektrum (Äthanol): $\lambda_{max.}$ 300 nm

2-[4-Amino-3-brom-5-(N-äthylcyclohexylaminomethyl)benzoyloxy]-2′-hydroxydiäthyloxid

$C_{20}H_{31}BrN_2O_4$ (443,4)

| | | | | |
|---|---|---|---|---|
| berechnet: | C 54,18 | H 7,05 | Br 18,02 | N 6,32 |
| gefunden: | C 53,90 | H 7,19 | Br 18,25 | N 6,26 |

N-{2-[4-Amino-3-brom-5-N-(äthylcyclohexylaminomethyl)benzoyloxy]äthyl}-N-(2-hydroxyäthyl)methylamin

$C_{21}H_{34}BrN_3O_3$ (456,4)

| | | | | |
|---|---|---|---|---|
| berechnet: | C 55,26 | H 7,51 | Br 17,51 | N 9,21 |
| gefunden: | C 55,30 | H 7,62 | Br 17,75 | N 9,06 |

1-{2-[4-Amino-3-brom-5-(N-äthylcyclohexylaminomethyl)benzoyloxy]äthyl}-4-(2-hydroxyäthyl)piperazin

IR-Spektrum (Methylenchlorid):

Ester-CO 1710 $cm^{-1}$

N-Alkyl 2830 $cm^{-1}$

UV-Spektrum (Äthanol): $\lambda_{max.}$ 300 nm

1-{3-[4-Amino-brom-5-(N-äthylcyclohexylaminomethyl)benzoyloxy]propyl}-4-(2-hydroxyäthyl)piperazin

Schmelzpunkt des Trihydrogenmaleinats: 144-146°C (Zers.)

1,6-Bis-[4-amino-3-brom-5-(N-äthylcyclohexylaminomethyl)benzoyl]-D-mannitdihydrochlorid

IR-Spektrum (KBr):

Ester-CO 1710 $cm^{-1}$

N-Alkyl 2840 $cm^{-1}$

UV-Spektrum (Äthanol): $\lambda_{max.}$ 230 nm, 295 nm

1-[4-Amino-3-brom-5-(N-äthylcyclohexylaminomethyl)benzoyloxy]-2-hydroxy-3-chlor-n-propan

IR-Spektrum (Methylenchlorid):

Ester-CO 1710 $cm^{-1}$

UV-Spektrum (Äthanol): $\lambda_{max.}$ 300 nm

1-[4-Amino-3-brom-5-(N-äthylcyclohexylaminomethyl)benzamido]-2-hydroxyäthan

Schmelzpunkt des Hydrochlorids: ab 65°C (Zers.)

1-[4-Amino-3-brom-5-(N-äthylcyclohexylaminomethyl)benzamido]-5-hydroxy-n-pentan

IR-Spektrum (Methylenchlorid):

Amid-CO 1650 $cm^{-1}$

UV-Spektrum (Äthanol): $\lambda_{max.}$ 285 nm

1-[4-Amino-3-brom-5-(N-äthylcyclohexylaminomethyl)benzamido]-6-hydroxy-n-hexan

IR-Spektrum (Methylenchlorid):

Amid-CO 1650 $cm^{-1}$

UV-Spektrum (Äthanol): $\lambda_{max.}$ 285 nm

trans-1-[4-Amino-3-brom-5-(N-äthylcyclohexylaminomethyl)benzamido]-4-hydroxycyclohexan

Schmelzpunkt des Dihydrochlorids: 176°C (Zers.)

N-[4-Amino-3-brom-5-(N-äthylcyclohexylaminomethyl)benzoyl]-D-glucosamin
Schmelzpunkt: 100-190°C (langsame Zersetzung unter Schäumen)

$C_{22}H_{24}BrN_3O_6$ (516,5)

| | | | | |
|---|---|---|---|---|
| berechnet: | C 51,16 | H 6,64 | Br 15,47 | N 8,14 |
| gefunden: | C 51,00 | H 6,87 | Br 15,30 | N 8,00 |

1-[4-Amino-3-brom-5-(N-äthylcyclohexylaminomethyl)benzamido]-2-aminoäthan
Schmelzpunkt: ab 85°C (Zers.)

1-(4-Amino-3-brom-5-diäthylaminomethylbenzoyloxy)-2-chloräthan
Schmelzpunkt des Hydrochlorids: 142-145°C

1-(4-Amino-3-brom-5-diäthylaminomethylbenzoyloxy)-3-chlor-n-propan
Schmelzpunkt des Hydrochlorids: 167-168°C

1-(4-Amino-3-brom-5-diäthylaminomethylbenzoyloxy)-4-chlor-n-butan
Schmelzpunkt des Hydrochlorids: 159-162°C

1-(4-Amino-3-brom-5-diäthylaminomethylbenzoyloxy)-5-chlor-n-pentan
Schmelzpunkt des Hydrochlorids: 117-119°C

1-(4-Amino-3-brom-5-diäthylaminomethylbenzoyloxy)-6-chlor-n-hexan
Schmelzpunkt des Hydrochlorids: 121-122°C

1-(4-Amino-3-brom-5-diäthylaminomethylbenzoyloxy)-2-hydroxyäthan
Schmelzpunkt des Hydrochlorids: 151-152°C

1-(4-Amino-3-brom-5-diäthylaminomethylbenzoyloxy)-3-hydroxy-n-propan
Schmelzpunkt des Hydrochlorids: 139-141°C

1-(4-Amino-3-brom-5-diäthylaminomethylbenzoyloxy)-4-hydroxy-n-butan
Schmelzpunkt des Hydrochlorids: 163-165°C

1-(4-Amino-3-brom-5-diäthylaminomethylbenzoyloxy)-5-hydroxy-n-pentan
Schmelzpunkt des Hydrochlorids: 137,5-138,5°C

1-(4-Amino-3-brom-5-diäthylaminomethylbenzoyloxy)-2,3-dihydroxy-n-propan
Massenspektrum: $M^+$ 374/6 m/e (Monobrom)

1-(4-Amino-3-brom-5-diäthylaminomethylbenzoyloxy)-2-hydroxy-3-chlor-n-propan
IR-Spektrum (Methylenchlorid):
Ester-CO 1715 $cm^{-1}$
UV-Spektrum (Äthanol): $\lambda_{max.}$ 230 nm, 298-300 nm

1-(4-Amino-3-brom-5-hexamethyleniminomethylbenzoyloxy)-5-chlor-n-pentan
Schmelzpunkt des Hydrochlorids: 160-162°C

1-[4-Amino-3-brom-5-(N-äthylcyclopentylaminomethyl)benzoyloxy]-5-chlor-n-pentan
Schmelzpunkt des Hydrochlorids: 131-133°C

1-[4-Amino-3-brom-5-(N-äthylcycloheptylaminomethyl)benzoyloxy]-5-chlor-n-pentan
Schmelzpunkt des Hydrochlorids: 156-158°C

1-[4-Amino-3-brom-5-(N-cyclohexyl-n-propylaminomethyl)benzoyloxy]-5-chlor-n-pentan
Schmelzpunkt des Hydrochlorids: 145-147°C

1-(4-Amino-3-brom-5-dimethylaminomethylbenzoyloxy)-2-hydroxyäthan
Schmelzpunkt des Hydrochlorids: 80-81°C

1-(4-Amino-3-brom-5-isopropylaminomethylbenzoyloxy)-2-hydroxyäthan
Schmelzpunkt des Hydrochlorids: 90-93°C

1-[4-Amino-3-brom-5-(N-methyl-n-propylaminomethyl)benzoyloxy]-2-hydroxyäthan
Schmelzpunkt des Hydrochlorids: 124-128°C

1-(4-Amino-3-brom-5-tert.-butylaminomethylbenzoyloxy)-2-hydroxyäthan
Schmelzpunkt des Hydrochlorids: 192-194°C

1-(4-Amino-3-brom-5-cyclohexylaminomethylbenzoyloxy)-2-hydroxyäthan
Schmelzpunkt des Dihydrochlorids: 149°C (Zers.)

1-[4-Amino-3-brom-5-(N-cyclohexylmethylaminomethyl)benzoyloxy]-2-hydroxyäthan
Schmelzpunkt des Hydrochlorids: 169-171°C

1-[4-Amino-3-brom-5-(N-äthylcyclopentylaminomethyl)benzoyloxy]-2-hydroxyäthan
Schmelzpunkt des Hydrochlorids: 186-187°C

1-[4-Amino-3-brom-5-(N-äthylcycloheptylaminomethyl)benzoyloxy]-2-hydroxyäthan
Schmelzpunkt des Hydrochlorids: 166-168°C

1-[4-Amino-3-brom-5-(N-cyclohexyl-n-propylaminomethyl)benzoyloxy]-2-hydroxyäthan
Schmelzpunkt des Hydrochlorids: 194-196°C

1-[4-Amino-3-brom-5-(4-pyridylaminomethyl)benzoyloxy]-2-hydroxyäthan
Schmelzpunkt des Hydrochlorids: 217-218°C

1-(4-Amino-3-brom-5-pyrrolidinomethylbenzoyloxy)-2-hydroxyäthan
Schmelzpunkt des Hydrochlorids: 213-214°C

1-(4-Amino-3-brom-5-piperidinomethylbenzoyloxy)-2-hydroxyäthan
Schmelzpunkt des Hydrochlorids: 214-216°C

1-(4-Amino-3-brom-5-morpholinomethylbenzoyloxy)-2-hydroxyäthan
Schmelzpunkt des Hydrochlorids: 179-181°C

1-[4-Amino-3-brom-5-(4-methylpiperazinomethyl)benzoyloxy]-2-hydroxyäthan
Schmelzpunkt des Dihydrochlorids: 163-165°C

1-(4-Amino-3-brom-5-hexamethyleniminomethylbenzoyloxy)-2-hydroxyäthan
Schmelzpunkt des Hydrochlorids: 209°C (Zers.)

1-[4-Amino-3-brom-5-(2-diäthylaminoäthylaminomethyl)benzoyloxy]-2-hydroxyäthan
Schmelzpunkt des Dihydrochlorids: 87-90°C (Zers.)

1-[4-Amino-3-brom-5-(N-äthylcyclohexylaminomethyl)benzoyloxy]-1-phenyl-2-hydroxyäthan
IR-Spektrum (Methylenchlorid):

| | |
|---|---|
| OH | 3600 $cm^{-1}$ |
| $NH_2$ | 3440 $cm^{-1}$ |
| N-Alkyl | 2930 $cm^{-1}$ |
| Ester-CO | 1710 $cm^{-1}$ |

UV-Spektrum (Äthanol): $\lambda_{max.}$ 230 nm, 297 nm

1-[4-Amino-3-brom-5-(N-äthylcyclohexylaminomethyl)benzoyloxy]-2-phenyl-2-hydroxyäthan
IR-Spektrum (Methylenchlorid):

| | |
|---|---|
| OH | 3600 $cm^{-1}$ |
| $NH_2$ | 3450 $cm^{-1}$ |
| N-Alkyl | 2950 $cm^{-1}$ |

Ester-CO 1710 $cm^{-1}$

UV-Spektrum (Äthanol): $\lambda_{max.}$ 293 nm

1-[4-Amino-3-brom-5-(N-äthylcyclohexylaminomethyl)benzoyloxy]-1,2-diphenyl-2-hydroxyäthan

IR-Spektrum (Methylenchlorid):

OH 3600 $cm^{-1}$
$NH_2$ 3450 $cm^{-1}$
N-Alkyl 2940 $cm^{-1}$
Ester-CO 1725 $cm^{-1}$

UV-Spektrum (Äthanol): $\lambda_{max.}$ 295 nm

1-[4-Amino-3-brom-5-(N-äthylcyclohexylaminomethyl)benzoyloxy]-2-hydroxymethyl-2-methyl-n-pentan

IR-Spektrum (Methylenchlorid):

OH 3620 $cm^{-1}$
$NH_2$ 3460 $cm^{-1}$
N-Alkyl 2960 $cm^{-1}$
Ester-CO 1705 $cm^{-1}$

UV-Spektrum (Äthanol): $\lambda_{max.}$ 288 nm

2-[4-Amino-3-brom-5-(N-äthylcyclohexylaminomethyl)benzoyloxymethyl]-6-hydroxymethylpyridin

IR-Spektrum (Methylenchlorid):

OH 3610 $cm^{-1}$
$NH_2$ 3440 $cm^{-1}$
N-Alkyl 2920 $cm^{-1}$
Ester-CO 1710 $cm^{-1}$

UV-Spektrum (Äthanol): $\lambda_{max.}$ 230 nm, 298 nm

1-[4-Amino-3-brom-5-(N-äthylcyclohexylaminomethyl)benzoyloxymethyl]-4-hydroxymethylbenzol

IR-Spektrum (Methylenchlorid):

OH 3600 $cm^{-1}$
$NH_2$ 3450 $cm^{-1}$
N-Alkyl 2940 $cm^{-1}$
Ester-CO 1705 $cm^{-1}$

UV-Spektrum (Äthanol): $\lambda_{max.}$ 295 nm

1-[4-Amino-3-brom-5-(N-äthylcyclohexylmethyl)benzoyloxymethyl]-4-hydroxymethylcyclohexan

IR-Spektrum (Methylenchlorid):

OH 3600 $cm^{-1}$
$NH_2$ 3440 $cm^{-1}$
N-Alkyl 2910 $cm^{-1}$
Ester-CO 1695 $cm^{-1}$

UV-Spektrum (Äthanol): $\lambda_{max.}$ 294 nm

1-(4-Amino-3-brom-5-dimethylaminomethylbenzoyloxy)-5-hydroxy-n-pentan

Schmelzpunkt des Hydrochlorids: 155-157°C

1-(4-Amino-3-brom-5-morpholinomethylbenzoyloxy)-5-hydroxy-n-pentan

Schmelzpunkt des Hydrochlorids: 205-207°C

1-(4-Amino-3-brom-5-piperidinomethylbenzoyloxy)-5-hydroxy-n-pentan

Schmelzpunkt des Hydrochlorids: 186-188°C

1-[4-Amino-3-brom-5-(4-methylpiperazinomethyl)benzoyloxy]-5-hydroxy-n-pentan

Schmelzpunkt des Dihydrochlorids: 182-185°C

1-[4-Amino-3-brom-5-(N-cyclohexylmethylaminomethyl)benzoyloxy]-5-hydroxy-n-pentan

Schmelzpunkt des Hydrochlorids: 123-126°C

1-[4-Amino-3-brom-5-(4-methylpiperazinomethyl)benzoyloxy]-4-hydroxy-n-butan

Schmelzpunkt des Dihydrochlorids: 200-202°C

1-(4-Amino-3-brom-5-hexamethyleniminomethylbenzoyloxy)-4-hydroxy-n-butan

Schmelzpunkt des Hydrochlorids: 159-161°C

1-[4-Amino-3-brom-5-(N-cyclohexylmethylaminomethyl)benzoyloxy]-4-hydroxy-n-butan

Schmelzpunkt des Hydrochlorids: 163-165°C

1-(4-Amino-3-brom-5-pyrrolidinomethylbenzoyloxy)-4-hydroxy-n-butan

Schmelzpunkt des Hydrochlorids: 140-144°C

1-(4-Amino-3-brom-5-morpholinomethylbenzoyloxy)-4-hydroxy-n-butan

Schmelzpunkt des Hydrochlorids: 182-184°C

1-(4-Amino-3-brom-5-piperidinomethylbenzoyloxy)-4-hydroxy-n-butan

Schmelzpunkt des Hydrochlorids: 170-172°C

1-[4-Amino-3-brom-5-(N-äthylcyclohexylaminomethyl)benzoyloxy]-9-hydroxy-n-nonan

Schmelzpunkt des Hydrochlorids: 73-76°C

*Beispiel 1:*

*1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthan*

25,0 g (0,0626 mol) 1-[4-Amino-3-brom-5-(N-äthylcyclohexylaminomethyl)benzoyloxy]-2-hydroxyäthan und 25,6 g (0,0626 mol) 1-(4-Chlorbenzoyl)-5-methoxy-2-methyl-[1H]-indol-3-essigsäureimidazolid werden in 300 ml Tetrahydrofuran gelöst. Unter Rühren fügt man bei Zimmertemperatur eine katalitische Menge Natriumhydriddispersion in Öl hinzu. Nach Stehen über Nacht wird im Wasserstrahlvakuum das Tetrahydrofuran abdestilliert, das verbleibende Öl in Chloroform aufgenommen und zweimal mit Wasser gewaschen. Die Chloroformlösung wird über Natriumsulfat getrocknet und eingeengt. Zur weiteren Reinigung wird das Rohprodukt über eine Kieselgelsäule mit Chloroform/Methanol (9,5:0,5) chromatographiert. Die erhaltene ölige Base wird als Hydrochlorid aus einem Gemisch aus Isopropanol und äthanolischem Chlorwasserstoff (1:1), welches 15% Chloroform enthält, kristallisiert.

Schmelzpunkt des Hydrochlorids: ab 123°C (Zers.)

*Beispiel 2:*

*1-[4-Amino-3-brom-5-(N-äthylcyclohexylaminomethyl)benzoyloxy]-3-(3-benzoyl-α-methylphenylacetoxy)-2-hydroxy-n-propan*

14,9 g (0,035 mol) 1-[4-Amino-3-brom-5-(N-äthylcyclohexylaminomethyl)benzoyloxy]-2,3-dihydroxy-n-propan und 10,6 g (0,035 mol) 3-Benzoyl-α-methylphenylessigsäureimidazolid werden in 250 ml absolutem Tetrahydrofuran gelöst. Unter Rühren gibt man bei Zimmertemperatur eine katalytische Menge Natriumhydriddispersion in Öl dazu. Man rührt 4 h und lässt über Nacht stehen. Danach filtriert man und engt das Filtrat im Vakuum ein. Der Rückstand wird in Chloroform gelöst und dreimal mit Wasser gewaschen. Die Chloroformlösung wird über Natriumsulfat getrocknet und im Vakuum zur Trockene eingeengt.

Das erhaltene Rohprodukt wird durch Säulenchromatographie mit Kieselgel und Toluol/Aceton (7:1) gereinigt. Man erhält das Hydrochlorid, indem man die erhaltene Base in absolutem Äthanol löst, mit absoluter äthanolischer Salzsäure bis zur sauren Reaktion versetzt und im Wasserstrahlvakuum zur Trockene einengt.

Schmelzpunkt des Hydrochlorids: 82-94°C

*Beispiel 3:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}methylamin*

Zu einer Lösung von 6,5 g (0,02 mol) 1-(4-Chlorbenzoyl)-5-methoxy-2-methyl-[1H]-indol-3-essigsäure in 150 ml wasserfreiem Tetrahydrofuran werden unter Rühren bei Raumtemperatur 3,6 g (0,022 mol) Carbonyldiimidazol hinzugegeben. Nach einstündigem Rühren bei Raumtemperatur wird bei −10°C eine Lösung aus 9,1 g (0,02 mol) N-{2-[4-Amino-3-brom-5-(N-äthylcyclohexylaminomethyl)benzoyloxy]äthyl}-N-(2-hydroxyäthyl)methylamin in 100 ml wasserfreiem Tetrahydrofuran hinzugetropft. Dem Reaktionsgemisch fügt man nach beendeter Zugabe eine Spatelspitze einer 50%igen öligen Natriumhydridsuspension hinzu und rührt unter langsamer Temperaturerhöhung bis zur Raumtemperatur weitere 12 h. Das Lösungsmittel wird im Vakuum bei 35°C abgedampft, den Eindampfrückstand löst man in 150 ml Äther und wäscht die ätherische Lösung mit Wasser, 2N-Ammoniak und nochmals mit Wasser. Nach dem Eindampfen der mit Magnesiumsulfat getrockneten ätherischen Lösung im Vakuum bei 30°C wird der erhaltene Eindampfrückstand über eine Kieselgelsäule mit Methylenchlorid/Methanol (30:1) gereinigt. Die gewünschten Fraktionen werden im Vakuum bei 35°C zur Trockene eingeengt. Nach Lösen des Eindampfrückstandes in 250 ml Äther und Versetzen mit ätherischer Salzsäure wird das Dihydrochlorid als amorphe Fällung isoliert.

Schmelzpunkt des Dihydrichlorids: 179-184°C (Zers.)

*Beispiel 4:*

*N-{2-[4-Amino-3-brom-5-(N-äthylcyclohexylaminomethyl)benzoyloxy]äthyl}-N-{2-{2-[(2,6-dichlorphenyl)amino]phenylacetoxy}äthyl}tert.-butylamin*

10 g (0,02 mol) N-{2-[4-Amino-3-brom-5-(N-äthylcyclohexylaminomethyl)benzoyloxy]-äthyl}-N-(2-hydroxyäthyl)-tert.-butylamin werden in 200 ml wasserfreiem Tetrahydrofuran gelöst und bei Raumtemperatur mit einer Lösung aus 6,5 g (0,022 mol) 2-[(2,6-Dichlorphenyl)amino]-phenylessigsäure sowie 0,3 g 4-Dimethylaminopyridin in 100 ml wasserfreiem Tetrahydrofuran versetzt. Bei −10°C wird zur Reaktionslösung eine aus 4,5 g (0,022 mol) Dicyclohexylcarbodiimid in 50 ml wasserfreiem Tetrahydrofuran bestehende Lösung hinzugetropft. Nach 60 min bei −10°C und weiteren 3 h bis zu einer Temperatur von 0°C wird nach einer erneuten Zugabe von 1,2 g (0,004 mol) 2-[(2,6-Dichlorphenyl)amino]-phenylessigsäure sowie 0,85 g (0,004 mol) Dicyclohexylcarbodiimid weitere 18 h bei Raumtemperatur gerührt. Das Reaktionsgemisch wird vom ausgefallenen Dicyclohexylharnstoff abgesaugt und der nach dem Einengen des Filtrats im Vakuum bei 35°C erhaltene Eindampfrückstand über eine Kieselgelsäule mit Äther/Petroläther (1:2) chromatographiert. Der nach gewünschter Fraktionierung erhaltene Eindampfrückstand wird nach Lösen in 500 ml Äther und Hinzufügen eines berechneten Anteils an ätherischer Salzsäure als amorphes Dihydrochlorid gewonnen.

Schmelzpunkt des Dihydrochlorids: 150-180°C

*Beispiel 5:*

*1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthan*

Zu einer Lösung von 168 g (0,387 mol) 1-[4-Amino-3-brom-5-(N-äthylcyclohexylaminomethyl)benzoyloxy]-2-hydroxyäthanhydrochlorid und 30,5 g (0,387 mol) Pyridin in 1500 ml Chloroform gibt man unter Rühren bei Zimmertemperatur eine Lösung von 150,5 g (0,4 mol) 1-(4-Chlorbenzoyl)-5-methoxy-2-methyl-[1H]-indol-3-acetylchlorid in 1000 ml Chloroform. Nach Rühren über Nacht wäscht man die Reaktionslösung fünfmal mit je 2 l Wasser, filtriert über Aktivkohle, trocknet über Natriumsulfat und engt im Wasserstrahlvakuum ein. Das erhaltene Öl löst man in einem Gemisch aus 150 ml Chloroform und 750 ml Isopropanol und stellt mit äthanolischer Salzsäure auf pH 3 bis 4 ein. Die Lösung wird mit 500 ml Äther bis zur beginnenden Trübung versetzt und das Produkt so zur Kristallisation gebracht. Das Kirstallisat wird in 600 ml Methanol gelöst, mit 1200 ml Isopropanol versetzt, wonach die Substanz wieder kristallisiert. Es wird bei 80°C im Vakuum getrocknet.

Schmelzpunkt des Hydrochlorids: ab 123°C (Zers.)

*Beispiel 6:*

*1-[4-Amino-3-brom-5-(N-äthylcyclohexylaminomethyl)benzoyloxy]-2-hydroxy-3-[1-(4-chlorbenzoyl)-5-methoxy-2-methyl-[1H]-indol-3-acetoxy]-n-propan*

18,5 g (0,045 mol) 1-[4-Amino-3-brom-5-(N-äthylcyclohexylaminomethyl)benzoyloxy]-2,3-epoxi-n-propan werden in 250 ml trockenem Dioxan gelöst. Zu dieser Lösung werden 15,5 g (0,045 mol) 1-(4-Chlorbenzoyl)-5-methoxy-2-methyl-[1H]-indol-3-essigsäure und 750 mg Eisen(III)chlorid gegeben. Das Gemisch wird 24 h unter Rückfluss und Feuchtigkeitsausschluss gekocht und dann im Vakuum eingeengt.

Der Rückstand wird durch Chromatographie an Kieselgel mit Chloroform/Methanol (40:1) gereinigt. Man erhält eine fast farblose, amorphe Substanz.

UV-Spektrum (Äthanol): $\lambda_{max.}$ 230 nm, 260 nm

IR-Spektrum (Methylenchlorid):
Amid-CO 1675 $cm^{-1}$
Ester-CO 1705 und 1740 $cm^{-1}$

*Beispiel 7:*

*1-[4-Amino-3-brom-5-(N-äthylcyclohexylaminomethyl)benzoyloxy]-2-methoxycarbonyl-2-[(+)-6-methoxy-α-methyl-2-naphthalinacetamino]äthan*

12 g (0,036 mol) 2-Methoxycarbonyl-2-[(+)-6-methoxy-α-methyl-2-naphthalinacetamino]-äthanol, 17,3 g (0,041 mol) 4-Amino-3-brom-5-(N-äthylcyclohexylaminomethyl)benzoylchlorid-hydrochlorid, 12,5 ml Triäthylamin und 2,2 g 4-Dimethylaminopyridin werden zu 200 ml Methylenchlorid gegeben. Die Mischung wird 1 h lang unter Rückfluss erhitzt. Nach Abkühlen auf Raumtemperatur wird zweimal mit je 200 ml Wasser gewaschen. Die Methylenchloridlösung wird abgetrennt, über Natriumsulfat getrocknet und im Vakuum zur Trockene eingedampft. Der ölige Rückstand wird über eine Chromatographiesäule gereinigt [Kieselgel: Benzol/Aceton (19:1)] und das Eluat eingeengt. Man erhält einen farblosen Schaum.

IR-Spektrum (Methylenchlorid):
$NH_2$ 3510 $cm^{-1}$
N-Alkyl 2930 $cm^{-1}$
O-$CH_3$ 2850 $cm^{-1}$
Ester-CO 1750 und 1720 $cm^{-1}$
Amid-CO 1680 $cm^{-1}$
UV-Spektrum (Äthanol): $\lambda_{max.}$ 234 nm, 295 nm

*Beispiel 8:*

*1-[4-Amino-3-brom-5-(N-äthylcyclohexylaminomethyl)benzoyloxy]-5-{2-[(2,6-dichlorphenyl)amino]phenylacetoxy}-n-pentan*

26,2 g (0,057 mol) 5-[4-Amino-3-brom-5-(N-äthylcyclohexylaminomethyl)benzoyloxy]-n-pentylchlorid, 20 g (0,063 mol) 2-[(2,6-Dichlorphenyl)amino]phenylessigsäurenatriumsalz und 4,2 g Natriumjodid werden in 280 ml absolutem Dimethylformamid gelöst und 4 h unter gelegentlichem Schütteln bei 110°C gehalten. Im Wasserstrahlvakuum wird das Dimethylformamid abdestilliert, der Rückstand in Chloroform gelöst, mit Wasser gewaschen, die organische Phase über Natriumsulfat getrocknet und im Vakuum eingeengt. Das erhaltene Rohprodukt chromatographiert man mit einer Kieselgelsäule und Cyclohexan/Aceton (9:1) und kristallisiert die erhaltene Base aus Isopropanol/Äther durch Ansäuern mit äthanolischer Salzsäure als Hydrochlorid.

Schmelzpunkt des Hydrochlorids: 129-132°C

*Beispiel 9:*

*1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan*

4,6 g (0,02 mol) (+)-6-Methoxy-α-methyl-2-naphthalinessigsäure werden in 30 ml Dimethylformamid gelöst und durch Zugabe von 875 mg (0,02 mol) Natriumhydrid in das Natriumsalz übergeführt. Hierzu werden 9 g (0,02 mol) 3-[4-Amino-3-brom-5-(N-äthylcyclohexylaminomethyl)benzoyloxy]-2-hydroxy-n-propylchlorid in 60 ml Dimethylformamid gegeben. Nach Zusatz von 3,5 g (0,021 mol) Kaliumjodid und 1 ml 15-Krone-5 wird das Gemisch 10 h lang auf ca. 80°C erhitzt, dann im Vakuum der grösste Teil des Dimethylformamids abdestilliert und der Rückstand zwischen einer Lösung von 35 g Ammoniumacetat in 400 ml Eiswasser und Chloroform verteilt. Die organische Phase wird über Natriumsulfat getrocknet und im Vakuum eingeengt. Der Rückstand wird durch Chromatographie an Kieselgel mit Toluol/Aceton/Ammoniak (80:20:0,25) gereinigt. Man erhält ein farbloses, dickflüssiges Öl.

IR-Spektrum (Methylenchlorid):
Ester-CO 1710 und 1740 $cm^{-1}$
UV-Spektrum (Äthanol): $\lambda_{max.}$ 234 nm, 298 nm
Schmelzpunkt des amorphen Hydrochlorids: ab 80°C

*Beispiel 10:*

*1-[1-(4-Chlorbenzoyl)-5-methoxy-2-methyl-[1H]-indol-3-acetyl]-6-[4-amino-3-brom-5-(N-äthylcyclohexylaminomethyl)benzoyl]-D-mannit*

12,3 g (0,03 mol) 4-Amino-3-brom-5-(N-äthylcyclohexylaminomethyl)benzoylchlorid-hydrochlorid, gelöst in 100 ml getrocknetem Chloroform, werden unter Eiswasserkühlung und Rühren zu einer Lösung aus 15,6 g (0,03 mol) 1-[1-(4-Chlorbenzoyl)-5-methoxy-2-methyl-[1H]-indol-3-acetyl]-D-mannit, gelöst in 200 ml absolutem Pyridin, zugetropft. Die Reaktionslösung wird 15 h bei Raumtemperatur gerührt, anschliessend im Vakuum bei 50°C das Pyridin abdestilliert und der Eindampfrückstand zwischen 300 ml Methylenchlorid und 300 ml Wasser verteilt. Nach Trennung der Phasen und weiterem zweimaligem Waschen der Methylenchloridphase mit je 200 ml 2N-Ammoniak sowie Wasser wird die organische Lösung über Magnesiumsulfat getrocknet und im Vakuum bei 35°C bis zur Trockne eingeengt. Zur weiteren Reinigung wird über eine Kieselgelsäule mit Chloroform/Methanol (30:1) chromatographiert und aus Methylenchlorid/Äther kristallisiert.

Schmelzpunkt: 97-112°C (Zers.)

*Beispiel 11:*

*1-Acetoxy-2-[4-amino-3-brom-5-(N-äthylcyclohexylaminomethyl)benzoyloxy]-3-[(+)-6-methoxy-α-methyl-2-naphthalinacetoxy]-n-propan*

9 g (0,025 mol) 4-Amino-3-brom-5-(N-äthylcyclohexylaminomethyl)benzoesäure werden in 60 ml Tetrahydrofuran gelöst und mit 4,2 g (0,026 mol) Carbonyldiimidazol versetzt. Nach ½ h gibt man eine Lösung von 7 g (0,021 mol) 1-Acetoxy-2-hydroxy-3-[(+)-6-methoxy-α-methyl-2-naphthalinacetoxy]-n-propan in 60 ml Tetrahydrofuran hinzu. Das Gemisch wird 2½ h unter

Rückfluss gekocht und nach Stehenlassen über Nacht bei Raumtemperatur im Vakuum eingeengt. Der Rückstand wird zwischen Wasser und Chloroform verteilt, die organische Phase abgetrennt, getrocknet und eingeengt. Der erhaltene Rückstand wird durch Chromatographie an Kieselgel mit Chloroform/Essigsäureäthylester (20:1) gereinigt. Man erhält eine farblose, amorphe Substanz.

UV-Spektrum (Äthanol): $\lambda_{max.}$ 243 nm, 300 nm
IR-Spektrum (Methylenchlorid):
Ester-CO 1710 und 1740 $cm^{-1}$

*Beispiel 12:*

*1-[4-Amino-3-brom-5-(N-äthylcyclohexylaminomethyl)benzoyloxy]-2-acetoxy-3-[(+)-6-methoxy-α-methyl-2-naphthalinacetoxy]-n-propan*

10 g (0,0156 mol) 1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan und 0,97 g (0,0156 mol) Pyridin werden in 150 ml absolutem Tetrahydrofuran gelöst. Zu dieser Lösung wird bei Zimmertemperatur eine Mischung aus 1,25 g (0,0156 mol) Acetylchlorid und 20 ml absolutem Tetrahydrofuran unter Rühren zugetropft. Man rührt 3 h und lässt über Nacht ausreagieren. Das Tetrahydrofuran wird im Wasserstrahlvakuum abdestilliert, der verbleibende ölige Rückstand in Chloroform gelöst und mit verdünnter Ammoniaklösung gewaschen. Die Chloroformphase trocknet man mit Natriumsulfat und engt im Vakuum ein. Das Rohprodukt wird über eine Kieselgelsäule mit Toluol/Aceton (4:1) gereinigt. Das erhaltene Öl löst man in absolutem Äthanol/Äther und stellt mit äthanolischer Salzsäure sauer. Das Hydrochlorid erhält man durch Eindampfen dieser Lösung zur Trockene.

Schmelzpunkt des Hydrochlorids: 87-93°C

*Beispiel 13:*

*1-[4-Amino-3-brom-5-(N-äthylcyclohexylaminomethyl)benzamido]-2-[1-(4-chlorbenzoyl)-5-methoxy-2-methyl-[1H]-indol-3-acetamido]äthan*

4,8 g (0,012 mol) 1-[4-Amino-3-brom-5-(N-äthylcyclohexylaminomethyl)benzamido]-2-aminoäthan und 4,9 g (0,012 mol) 1-(4-Chlorbenzoyl)-5-methoxy-2-methyl-[1H]-indol-3-essigsäureimidazolid werden in 75 ml Tetrahydrofuran gelöst, eine katalytische Menge Natriumhydriddispersion in Öl zugesetzt und bei Zimmertemperatur 1 h gerührt. Man destilliert das Tetrahydrofuran im Vakuum ab, löst den Rückstand in Chloroform und wäscht dreimal mit Wasser. Die organische Phase trocknet man mit Natriumsulfat und filtriert über Aktivkohle. Im Vakuum wird eingeengt, in Isopropanol gelöst, mit ätherischer Salzsäure angesäuert und das Hydrochlorid durch Zusatz von Äther gefällt.

Schmelzpunkt des Hydrochlorids: ab 145°C (Zers.)

*Beispiel 14:*

*2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]diäthylsulfoxid*

Zu einer Lösung aus 10 g (0,0125 mol) 2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]-diäthylsulfid in 100 ml Eisessig wird unter Rühren bei Raumtemperatur 1,5 g (0,0125 mol) einer 30%igen wässerigen Wasserstoffperoxidlösung hinzugegeben. Die Reaktionslösung wird 2 h bei Raumtemperatur gerührt und anschliessend im Vakuum bei 40°C eingedampft. Den erhaltenen Rückstand löst man unter Zugabe von 100 ml Tetrahydrofuran in 300 ml Äther und wäscht die Lösung mit 2N-Ammoniak und Wasser. Anschliessend wird die organische Phase mit Magnesiumsulfat getrocknet, im Vakuum bei 35°C zur Trockene eingeengt und der erhaltene Eindampfrückstand über eine Kieselgelsäule mit Methylenchlorid/Methanol (75:1) gereinigt. Die gewünschten Fraktionen werden vereinigt und im Vakuum bei 35°C zur Trockene eingeengt. Man erhält das Reaktionsprodukt als öligen Eindampfrückstand, der über Schwefelsäure-Trockenmittel im Vakuum bei Raumtemperatur von anhaftenden Lösungsmittelresten befreit wird.

$C_{39}H_{45}BrClN_3O_7S$ (815,2)

| | | | |
|---|---|---|---|
| berechnet: | C 57,46 | H 5,56 | Br 9,80 |
| | Cl 4,35 | N 5,15 | |
| gefunden: | C 57,64 | H 5,75 | Br 9,64 |
| | Cl 4,28 | N 5,00 | |

*Beispiel 15:*

*2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]diäthylsulfon*

Zu einer Lösung von 10 g (0,0125 mol) 2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]diäthylsulfid in 150 ml Eisessig werden 3 g (0,025 mol) einer 30%igen Wasserstoffperoxidlösung hinzugegeben. Nach 130 h Rühren bei Raumtemperatur wird der Eisessig im Vakuum bei 40°C abgedampft. Anschliessend wird der Eindampfrückstand in 300 ml Äther sowie 150 ml Tetrahydrofuran gelöst und die Lösung mit 2N-Ammoniak und Wasser gewaschen. Vor dem Eindampfen im Vakuum trocknet man die organische Phase über Magnesiumsulfat. Der Eindampfrückstand wird zweimal über eine Kieselgelsäule mit Äther/Tetrahydrofuran (10:1) bzw. Methylenchlorid/Methanol (75:1) chromatographiert. Nach Fraktionierung und Eindampfen der vereinigten Fraktionen im Vakuum bei 35°C erhält man einen schaumigen Eindampfrückstand.

$C_{39}H_{45}BrClN_3O_8S$ (831,2)

| | | | |
|---|---|---|---|
| berechnet: | C 56,35 | H 5,46 | Br 9,61 |
| | Cl 4,27 | N 5,06 | S 3,86 |
| gefunden: | C 56,11 | H 5,41 | Br 9,91 |
| | Cl 4,41 | N 4,95 | S 4,10 |

*Beispiel 16:*

*1-[4-Amino-3-brom-5-(N-äthylcyclohexylaminomethyl)benzoyloxy]-3-[1-(4-chlorbenzoyl)-5-methoxy-2-methyl-[1H]-indol-3-acetoxy]-n-propan*

Hergestellt aus 1-(4-Chlorbenzoyl)-5-methoxy-2-methyl-[1H]-indol-3-essigsäureimidazolid und 1-[4-Amino-3-brom-5-(N-äthylcyclohexylaminomethyl)benzoyloxy]-3-hydroxy-n-propan analog Beispiel 1. Öl.

$C_{38}H_{43}BrClN_3O_6$ (743,1)

| | | | |
|---|---|---|---|
| berechnet: | C 60,60 | H 5,76 | Br 10,61 |
| | Cl 4,71 | N 5,58 | |
| gefunden: | C 60,80 | H 5,93 | Br 10,73 |
| | Cl 4,76 | N 5,35 | |

*Beispiel 17:*

*1-[4-Amino-3-brom-5-(N-äthylcyclohexyaminomethyl)benzoyloxy]-4-[1-(4-chlorbenzoyl)-5-methoxy-2-methyl-[1H]-indol-3-acetoxy]-n-butan*

Hergestellt aus 1-(4-Chlorbenzoyl)-5-methoxy-2-methyl-[1H]-indol-3-essigsäureimidazolid und 1-[4-Amino-3-brom-5-(N-äthylcyclohexylaminomethyl)benzoyloxy]-4-hydroxy-n-butan analog Beispiel 1. Öl.

$C_{39}H_{45}BrClN_3O_6$ (767,2)

| | | | |
|---|---|---|---|
| berechnet: | C 61,06 | H 5,91 | Br 10,42 |
| | Cl 4,62 | N 5,48 | |
| gefunden: | C 61,30 | H 6,08 | Br 10,05 |
| | Cl 4,46 | N 5,46 | |

*Beispiel 18:*

*1-[4-Amino-3-brom-5-(N-äthylcyclohexylaminomethyl)benzoyloxy]-5-[1-(4-chlorbenzoyl)-5-methoxy-2-methyl-[1H]-indol-3-acetoxy]-n-pentan*

Hergestellt aus 1-(4-Chlorbenzoyl)-5-methoxy-2-methyl-[1H]-indol-3-essigsäureimidazolid und 1-[4-Amino-3-brom-5-(N-äthylcyclohexylaminomethyl)benzoyloxy]-5-hydroxy-n-pentan analog Beispiel 1. Öl.

$C_{40}H_{47}BrClN_3O_6$ (781,2)

| | | | |
|---|---|---|---|
| berechnet: | C 61,50 | H 6,06 | Br 10,23 |
| | Cl 4,54 | N 5,38 | |
| gefunden: | C 61,70 | H 6,11 | Br 10,10 |
| | Cl 4,48 | N 5,18 | |

*Beispiel 19:*

*1-[4-Amino-3-brom-5-(N-äthylcyclohexylaminomethyl)benzoyloxy]-6-[1-(4-chlorbenzoyl)-5-methoxy-2-methyl-[1H]-indol-3-acetoxy]-n-hexan*

Hergestellt aus 1-(4-Chlorbenzoyl)-5-methoxy-2-methyl-[1H]-indol-3-essigsäureimidazolid und 1-[4-Amino-3-brom-5-(N-äthylcyclohexylaminomethyl)benzoyloxy]-6-hydroxy-n-hexan analog Beispiel 1. Öl.

$C_{41}H_{49}BrClN_3O_6$ (795,2)

| | | | |
|---|---|---|---|
| berechnet: | C 61,93 | H 6,21 | Br 10,05 |
| | Cl 4,46 | N 5,28 | |
| gefunden: | C 62,20 | H 6,15 | Br 10,30 |
| | Cl 4,57 | N 5,32 | |

*Beispiel 20:*

*1-[4-Amino-3-brom-5-(N-äthylcyclohexylaminomethyl)benzoyloxy]-2-{2-[(2,6-dichlorphenyl)amino]phenylacetoxy}äthan*

Hergestellt aus 2-[4-Amino-3-brom-5-(N-äthylcyclohexylaminomethyl)benzoyloxy]äthylchlorid und 2-[(2,6-Dichlorphenyl)amino]-phenylessigsäurenatriumsalz analog Beispiel 8.

Schmelzpunkt des Hydrochlorids: 94-98°C

*Beispiel 21:*

*1-[4-Amino-3-brom-5-(N-äthylcyclohexylaminomethyl)benzoyloxy]-3-{2-[(2,6-dichlorphenyl)amino]phenylacetoxy}-n-propan*

Hergestellt aus 3-[4-Amino-3-brom-5-(N-äthylcyclohexylaminomethyl)benzoyloxy]-n-propylchlorid und 2-[(2,6-Dichlorphenyl)-amino]phenylessigsäurenatriumsalz analog Beispiel 8.

Schmelzpunkt des Hydrochlorids: 76-92°C

*Beispiel 22:*

*1-[4-Amino-3-brom-5-(N-äthylcyclohexylaminomethyl)benzoyloxy]-4-{2-[(2,6-dichlorphenyl)amino]phenylacetoxy}-n-butan*

Hergestellt aus 4-[4-Amino-3-brom-5-(N-äthylcyclohexylaminomethyl)benzoyloxy]-n-butylchlorid und 2-[(2,6-Dichlorphenyl)-amino]phenylessigsäurenatriumsalz analog Beispiel 8.

Schmelzpunkt des Hydrochlorids: 75-83°C

*Beispiel 23:*

*1-[4-Amino-3-brom-5-(N-äthylcyclohexylaminomethyl)benzoyloxy]-6-{2-[(2,6-dichlorphenyl)amino]phenylacetoxy}-n-hexan*

Hergestellt aus 6-[4-Amino-3-brom-5-(N-äthylcyclohexylaminomethyl)benzoyloxy]-n-hexylchlorid und 2-[(2,6-Dichlorphenyl)amino]-phenylessigsäurenatriumsalz analog Beispiel 8.

Schmelzpunkt des Hydrochlorids: 105-108°C

*Beispiel 24:*

*1-[4-Amino-3-brom-5-(N-äthylcyclohexylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-äthylcyclohexylaminomethyl)benzoyloxy]-2-hydroxyäthan analog Beispiel 1.

Schmelzpunkt des Hydrochlorids: 60-64°C

*Beispiel 25:*

*1-[4-Amino-3-brom-5-(N-äthylcyclohexylaminomethyl)benzoyloxy]-3-(3-benzoyl-α-methylphenylacetoxy)-n-propan*

Hergestellt aus 3-Benzoyl-α-methylphenylessigsäureimidazolid und 1-[4-Amino-3-brom-5-(N-äthylcyclohexylaminomethyl)benzoyloxy]-3-hydroxy-n-propan analog Beispiel 1.

Schmelzpunkt des Hydrochlorids: ab 56°C (sintern)

*Beispiel 26:*

*1-[4-Amino-3-brom-5-(N-äthylcyclohexylaminomethyl)benzoyloxy]-4-(3-benzoyl-α-methylphenylacetoxy)-n-butan*

Hergestellt aus 3-Benzoyl-α-methylphenylessigsäureimidazolid und 1-[4-Amino-3-brom-5-(N-äthylcyclohexylaminomethyl)benzoyloxy]-4-hydroxy-n-butan analog Beispiel 1.

Schmelzpunkt des Hydrochlorids: 59-64°C

*Beispiel 27:*

*1-[4-Amino-3-brom-5-(N-äthylcyclohexylaminomethyl)benzoyloxy]-5-(3-benzoyl-α-methylphenylacetoxy)-n-pentan*

Hergestellt aus 3-Benzoyl-α-methylphenylessigsäureimidazolid und 1-[4-Amino-3-brom-5-(N-äthylcyclohexylaminomethyl)benzoyloxy]-5-hydroxy-n-pentan analog Beispiel 1. Öl.

IR-Spektrum (Methylenchlorid):
Ester-CO 1700 und 1730 $cm^{-1}$
UV-Spektrum (Äthanol): $\lambda_{max.}$ 258 nm, 295 nm

*Beispiel 28:*

*1-[4-Amino-3-brom-5-(N-äthylcyclohexylaminomethyl)benzoyloxy]-6-(3-benzoyl-α-methylphenylacetoxy)-n-hexan*

Hergestellt aus 3-Benzoyl-α-methylphenylessigsäureimidazolid und 1-[4-Amino-3-brom-5-(N-äthylcyclohexylaminomethyl)benzoyloxy]-6-hydroxy-n-hexan analog Beispiel 1. Öl.

IR-Spektrum (Methylenchlorid):
Ester-CO 1710 und 1730 $cm^{-1}$
UV-Spektrum (Äthanol): $\lambda_{max.}$ 258 nm, 295 nm

*Beispiel 29:*

*1-[4-Amino-3-brom-5-(N-äthylcyclohexylaminomethyl)benzoyloxy]-2-[(+)-6-methoxy-α-methyl-2-naphthalinacetoxy]äthan*

Hergestellt aus (+)-6-Methoxy-α-methyl-2-naphthalinessigsäureimidazolid und 1-[4-Amino-3-brom-5-(N-äthylcyclohexylaminomethyl)-benzoyloxy]-2-hydroxyäthan analog Beispiel 1. Öl.

IR-Spektrum (Methylenchlorid):
$NH_2$ 3440 $cm^{-1}$
N-Alkyl 2920 $cm^{-1}$
$OCH_3$ 2850 $cm^{-1}$
Ester-CO 1730 und 1710 $cm^{-1}$
UV-Spektrum (Äthanol): $\lambda_{max.}$ 234 nm, 295 nm

*Beispiel 30:*

*1-[4-Amino-3-brom-5-(N-äthylcyclohexylaminomethyl)benzoyloxy]-3-[(+)-6-methoxy-α-methyl-2-naphthalinacetoxy]-n-propan*

Hergestellt aus (+)-6-Methoxy-α-methyl-2-naphthalinessigsäureimidazolid und 1-[4-Amino-3-brom-5-(N-äthylcyclohexylaminomethyl)-benzoyloxy]-3-hydroxy-n-propan analog Beispiel 1. Das Hydrochlorid ist ein Schaum.

$C_{33}H_{41}BrN_2O_5 \cdot HCl$ (662,1)

| | | | |
|---|---|---|---|
| berechnet: | C 59,87 | H 6,39 | Br 12,07 |
| | Cl 5,36 | N 4,23 | |
| gefunden: | C 59,50 | H 6,29 | Br 12,30 |
| | Cl 5,44 | N 4,02 | |

*Beispiel 31:*

*1-[4-Amino-3-brom-5-(N-äthylcyclohexylaminomethyl)benzoyloxy]-4-[(+)-6-methoxy-α-methyl-2-naphthalinacetoxy]-n-butan*

Hergestellt aus (+)-6-Methoxy-α-methyl-2-naphthalinessigsäureimidazolid und 1-[4-Amino-3-brom-5-(N-äthylcyclohexylaminomethyl)-benzoyloxy]-4-hydroxy-n-butan analog Beispiel 1. Öl.

IR-Spektrum (Methylenchlorid):
$NH_2$ 3440 $cm^{-1}$
N-Alkyl 2920 $cm^{-1}$
$OCH_3$ 2850 $cm^{-1}$
Ester-CO 1735 und 1710 $cm^{-1}$
UV-Spektrum (Äthanol): $\lambda_{max.}$ 234 nm, 295 nm

*Beispiel 32:*

*1-[4-Amino-3-brom-5-(N-äthylcyclohexylaminomethyl)benzoyloxy]-5-[(+)-6-methoxy-α-methyl-2-naphthalinacetoxy]-n-pentan*

Hergestellt aus (+)-6-Methoxy-α-methyl-2-naphthalinessigsäureimidazolid und 1-[4-Amino-3-brom-5-(N-äthylcyclohexylaminomethyl)-benzoyloxy]-5-hydroxy-n-pentan analog Beispiel 1. Öl.

IR-Spektrum (Methylenchlorid):
$NH_2$ 3440 $cm^{-1}$
N-Alkyl 2920 $cm^{-1}$
$OCH_3$ 2850 $cm^{-1}$
Ester-CO 1730 und 1710 $cm^{-1}$
UV-Spektrum (Äthanol): $\lambda_{max.}$ 234 nm, 295 nm

*Beispiel 33:*

*1-[4-Amino-3-brom-5-(N-äthylcyclohexylaminomethyl)benzoyloxy]-6-[(+)-6-methoxy-α-methyl-2-naphthalinacetoxy]-n-hexan*

Hergestellt aus (+)-6-Methoxy-α-methyl-2-naphthalinessigsäureimidazolid und 1-[4-Amino-3-brom-5-(N-äthylcyclohexylaminomethyl)-benzoyloxy]-6-hydroxy-n-hexan analog Beispiel 1. Öl.

IR-Spektrum (Methylenchlorid):
$NH_2$ 3430 $cm^{-1}$
N-Alkyl 2920 $cm^{-1}$
$OCH_3$ 2850 $cm^{-1}$
Ester-CO 1730 und 1710 $cm^{-1}$
UV-Spektrum (Äthanol): $\lambda_{max.}$ 234 nm, 295 nm

*Beispiel 34:*

*1-[4-Amino-3-brom-5-(N-äthylcyclohexyl-*

*aminomethyl)benzoyloxy]-2-[α-methyl-4-(2-methylpropyl)phenylacetoxy]äthan*

Hergestellt aus α-Methyl-4-(2-methylpropyl)-phenylessigsäureimidazolid und 1-[4-Amino-3-brom-5-(N-äthylcyclohexylaminomethyl)-benzoyloxy]-2-hydroxyäthan analog Beispiel 1. Das Hydrochlorid ist ein Öl.

$C_{31}H_{43}BrN_2O_4 \cdot HCl$ (624,1)

| | | | |
|---|---|---|---|
| berechnet: | C 59,66 | H 7,11 | Br 12,81 |
| | Cl 5,68 | N 4,49 | |
| gefunden: | C 59,50 | H 7,32 | Br 12,64 |
| | Cl 5,61 | N 4,43 | |

*Beispiel 35:*

*1-[4-Amino-3-brom-5-(N-äthylcyclohexylaminomethyl)benzoyloxy]-3-[α-methyl-4-(2-methylpropyl)phenylacetoxy]-n-propan*

Hergestellt aus α-Methyl-4-(2-methylpropyl)-phenylessigsäureimidazolid und 1-[4-Amino-3-brom-5-(N-äthylcyclohexylaminomethyl)-benzoyloxy]-3-hydroxy-n-propan analog Beispiel 1. Das Hydrochlorid ist ein Öl.

$C_{32}H_{45}BrN_2O_4 \cdot HCl$ (638,1)

| | | | |
|---|---|---|---|
| berechnet: | C 60,24 | H 7,27 | Br 12,52 |
| | Cl 5,56 | N 4,39 | |
| gefunden: | C 60,30 | H 7,53 | Br 12,30 |
| | Cl 5,45 | N 4,36 | |

*Beispiel 36:*

*1-[4-Amino-3-brom-5-(N-äthylcyclohexylaminomethyl)benzoyloxy]-4-[α-methyl-4-(2-methylpropyl)phenylacetoxy]-n-butan*

Hergestellt aus α-Methyl-4-(2-methylpropyl)-phenylessigsäureimidazolid und 1-[4-Amino-3-brom-5-(N-äthylcyclohexylaminomethyl)benzoyloxy]-4-hydroxy-n-butan analog Beispiel 1.

Schmelzpunkt des Hydrochlorids: 77-79°C

*Beispiel 37:*

*1-[4-Amino-3-brom-5-(N-äthylcyclohexylaminomethyl)benzoyloxy]-5-[α-methyl-4-(2-methylpropyl)phenylacetoxy]-n-pentan*

Hergestellt aus α-Methyl-4-(2-methylpropyl)-phenylessigsäureimidazolid und 1-[4-Amino-3-brom-5-(N-äthylcyclohexylaminomethyl)-benzoyloxy]-5-hydroxy-n-pentan analog Beispiel 1.

Schmelzpunkt des Hydrochlorids: 119-121°C

*Beispiel 38:*

*1-[4-Amino-3-brom-5-(N-äthylcyclohexylaminomethyl)benzoyloxy]-2-(2-acetoxybenzoyloxy)äthan*

Hergestellt aus 2-Acetoxybenzoylchlorid und 1-[4-Amino-3-brom-5-(N-äthylcyclohexylaminomethyl)benzoyloxy]-2-hydroxyäthan analog Beispiel 5. Öl.

IR-Spektrum (Methylenchlorid):

Ester-CO 1710 und 1770 $cm^{-1}$

UV-Spektrum (Äthanol): $\lambda_{max.}$ 231 nm, 300 nm

*Beispiel 39:*

*1-[4-Amino-3-brom-5-(N-äthylcyclohexylaminomethyl)benzoyloxy]-2-[2-fluor-α-methyl-(1,1'-biphenyl)-4-acetoxy]äthan*

Hergestellt aus 2-Fluor-α-methyl(1,1'-biphenyl)-4-essigsäureimidazolid und 1-[4-Amino-3-brom-5-(N-äthylcyclohexylaminomethyl)-benzoyloxy]-2-hydroxyäthan analog Beispiel 1. Das Hydrochlorid est ein Öl.

$C_{33}H_{38}BrFN_2O_4 \cdot HCl$ (662,1)

| | | | |
|---|---|---|---|
| berechnet: | C 59,87 | H 5,94 | Br 12,07 |
| | Cl 5,36 | N 4,23 | |
| gefunden: | C 60,16 | H 6,17 | Br 11,95 |
| | Cl 5,29 | N 4,84 | |

*Beispiel 40:*

*1-(4-Amino-3-brom-5-diäthylaminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-diäthylaminomethylbenzoyloxy]-2-hydroxyäthan analog Beispiel 1.

Schmelzpunkt des Hydrochlorids: 186-187°C

*Beispiel 41:*

*1-(4-Amino-3-brom-5-diäthylaminomethylbenzoyloxy)-3-[1-(4-chlorbenzoyl)-5-methoxy-2-methyl-[1H]-indol-3-acetoxy]-n-propan*

Hergestellt aus 1-(4-Chlorbenzoyl)-5-methoxy-2-methyl-[1H]-indol-3-essigsäureimiazolid und 1-(4-Amino-3-brom-5-diäthylaminomethylbenzoyloxy)-3-hydroxy-n-propan analog Beispiel 1.

Schmelzpunkt des Hydrochlorids: 123-125°C

*Beispiel 42:*

*1-(4-Amino-3-brom-5-diäthylaminomethylbenzoyloxy)-4-[1-(4-chlorbenzoyl)-5-methoxy-2-methyl-[1H]-indol-3-acetoxy]-n-butan*

Hergestellt aus 1-(4-Chlorbenzoyl)-5-methoxy-2-methyl-[1H]-indol-3-essigsäureimidazolid und 1-(4-Amino-3-brom-5-diäthylaminomethylbenzoyloxy)-4-hydroxy-n-butan analog Beispiel 1.

Schmelzpunkt des Hydrochlorids: 74-85°C

*Beispiel 43:*

*1-(4-Amino-3-brom-5-diäthylaminomethylbenzoyloxy)-5-[1-(4-chlorbenzoyl)-5-methoxy-2-methyl-[1H]-indol-3-acetoxy]-n-pentan*

Hergestellt aus 1-(4-Chlorbenzoyl)-5-methoxy-2-methyl-[1H]-indol-3-essigsäureimidazolid und 1-(4-Amino-3-brom-5-diätylaminomethylbenzoyloxy)-5-hydroxy-n-pentan analog Beispiel 1.

Schmelzpunkt des Hydrochlorids: 70-74°C

*Beispiel 44:*

*1-(4-Amino-3-brom-5-diäthylaminomethyl-*

*benzoyloxy)-6-[1-(4-chlorbenzoyl)-5-methoxy-2-methyl-[1H]-indol-3-acetoxy]-n-hexan*

Hergestellt aus 1-(4-Chlorbenzoyl)-5-methoxy-2-methyl-[1H]-indol-3-essigsäureimidazolid und 1-(4-Amino-3-brom-5-diäthylaminomethylbenzoyloxy)-6-hydroxy-n-hexan analog Beispiel 1.

Schmelzpunkt des Hydrochlorids: 131-134°C

*Beispiel 45:*

*1-(4-Amino-3-brom-5-diäthylaminomethylbenzoyloxy)-2-{-2-[(2,6-dichlorphenyl)amino]-phenylacetoxy}äthan*

Hergestellt aus 2-[(2,6-Dichlorphenyl)amino]-phenylessigsäurenatriumsalz und 2-(4-Amino-3-brom-5-diäthylaminomethylbenzoyloxy)äthylchlorid analog Beispiel 8.

Schmelzpunkt des Hydrochlorids: 76-82°C

*Beispiel 46:*

*1-(4-Amino-3-brom-5-diäthylaminomethylbenzoyloxy)-3-{2-[(2,6-dichlorphenyl)amino]-phenylacetoxy}-n-propan*

Hergestellt aus 2-[(2,6-Dichlorphenyl)amino]-phenylessigsäurenatriumsalz und 3-(4-Amino-3-brom-5-diäthylaminomethylbenzoyloxy)-n-propylchlorid analog Beispiel 8.

Schmelzpunkt des Hydrochlorids: 64-78°C

*Beispiel 47:*

*1-(4-Amino-3-brom-5-diäthylaminomethylbenzoyloxy)-4-{2-[(2,6-dichlorphenyl)amino]-phenylacetoxy}-n-butan*

Hergestellt aus 2-[(2,6-Dichlorphenyl)amino]-phenylessigsäurenatriumsalz und 4-(4-Amino-3-brom-5-diäthylaminomethylbenzoyloxy)-n-butylchlorid analog Beispiel 8.

Schmelzpunkt des Hydrochlorids: 58-69°C

*Beispiel 48:*

*1-(4-Amino-3-brom-5-diäthylaminomethylbenzoyloxy)-5-{2-[(2,6-dichlorphenyl)amino]-phenylacetoxy}-n-pentan*

Hergestellt aus 2-[(2,6-Dichlorphenyl)amino]-phenylessigsäurenatriumsalz und 5-(4-Amino-3-brom-5-diäthylaminomethylbenzoyloxy)-n-pentylchlorid analog Beispiel 8.

Schmelzpunkt des Hydrochlorids: 64-72°C

*Beispiel 49:*

*1-(4-Amino-3-brom-5-diäthylaminomethylbenzoyloxy)-2-[(+)-6-methoxy-α-methyl-2-naphthalinacetoxy]äthan*

Hergestellt aus (+)-6-Methoxy-α-methyl-2-naphthalinessigsäureimidazolid und 1-(4-Amino-3-brom-5-diäthylaminomethylbenzoyloxy)-2-hydroxyäthan analog Beispiel 1.

Schmelzpunkt des Hydrochlorids: 123-124°C

*Beispiel 50:*

*1-(4-Amino-3-brom-5-diäthylaminomethylbenzoyloxy)-3-[(+)-6-methoxy-α-methyl-2-naphthalinacetoxy]-n-propan*

Hergestellt aus (+)-6-Methoxy-α-methyl-2-naphthalinessigsäureimidazolid und 1-(4-Amino-3-brom-5-diäthylaminomethylbenzoyloxy)-3-hydroxy-n-propan analog Beispiel 1.

Schmelzpunkt des Hydrochlorids: 63-69°C

*Beispiel 51:*

*1-(4-Amino-3-brom-5-diäthylaminomethylbenzoyloxy)-4-[(+)-6-methoxy-α-methyl-2-naphthalinacetoxy]-n-butan*

Hergestellt aus (+)-6-Methoxy-α-methyl-2-naphthalinessigsäureimidazolid und 1-(4-Amino-3-brom-5-diäthylaminomethylbenzoyloxy)-4-hydroxy-n-butan analog Beispiel 1.

Schmelzpunkt des Hydrochlorids: 47-71°C

*Beispiel 52:*

*1-(4-Amino-3-brom-5-diäthylaminomethylbenzoyloxy)-5-[(+)-6-methoxy-α-methyl-2-naphthalinacetoxy]-n-pentan*

Hergestellt aus (+)-6-Methoxy-α-methyl-2-naphthalinessigsäureimidazolid und 1-(4-Amino-3-brom-5-diäthylaminomethylbenzoyloxy)-5-hydroxy-n-pentan analog Beispiel 1. Öl.

IR-Spektrum (Methylenchlorid):
Ester-CO 1700 und 1725 $cm^{-1}$
UV-Spektrum (Äthanol): $\lambda_{max.}$ 230 nm, 290 nm

*Beispiel 53:*

*1-[4-Amino-3-brom-5-diäthylaminomethylbenzoyloxy]-2-(2-acetoxybenzoyloxy)äthan*

Hergestellt aus 2-Acetoxybenzoylchlorid und 1-[4-Amino-3-brom-5-diäthylaminomethylbenzoyloxy]-2-hydroxyäthan analog Beispiel 5. Öl.

$C_{23}H_{27}BrN_2O_6$ (507,4)

| | | | | |
|---|---|---|---|---|
| berechnet: | C 54,45 | H 5,36 | Br 15,75 | N 5,52 |
| gefunden: | C 54,30 | H 5,46 | Br 16,20 | N 5,47 |

*Beispiel 54:*

*1-(4-Amino-3-brom-5-hexamethyleniminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-hexamethyleniminomethylbenzoyloxy)-2-hydroxyäthan analog Beispiel 1.

Schmelzpunkt des Hydrochlorids: 207-208°C

*Beispiel 55:*

*1-[4-Amino-3-brom-5-(N-äthylcycloheptylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-äthylcycloheptylaminomethyl)benzoyloxy]-2-hydroxyäthan analog Beispiel 1

Schmelzpunkt des Hydrochlorids: 90-120°C

*Beispiel 56:*

*1-(4-Amino-3-brom-5-tert.-butylaminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-tert.-butylaminomethylbenzoyloxy)-2-hydroxyäthan analog Beispiel 1. Das Hydrochlorid ist ein Öl.

$C_{33}H_{35}BrClN_3O_6 \cdot HCl$ (721,5)

| | | | |
|---|---|---|---|
| berechnet: | C 54,89 | H 5,03 | Br 11,08 |
| | Cl 9,83 | N 5,82 | |
| gefunden: | C 55,10 | H 5,26 | Br 10,90 |
| | Cl 9,67 | N 5,62 | |

*Beispiel 57:*

*1-[4-Amino-3-brom-5-(N-äthylcyclopentylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-äthylcyclopentylaminomethyl)benzoyloxy]-2-hydroxyäthan analog Beispiel 1.

Schmelzpunkt des Hydrochlorids: 206-207°C

*Beispiel 58:*

*1-(4-Amino-3-brom-5-dimethylaminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-dimethylaminomethylbenzoyloxy)-2-hydroxyäthan analog Beispiel 1.

Schmelzpunkt des Hydrochlorids: 174-176°C

*Beispiel 59:*

*1-[4-Amino-3-brom-5-(N-cyclohexylmethylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-cyclohexylmethylaminomethyl)benzoyloxy]-2-hydroxyäthan analog Beispiel 1.

Schmelzpunkt des Hydrochlorids: 151-153°C

*Beispiel 60:*

*1-[4-Amino-3-brom-5-(N-cyclohexyl-n-propylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-cyclohexyl-n-propylaminomethyl)benzoyloxy]-2-hydroxyäthan analog Beispiel 1.

Schmelzpunkt des Hydrochlorids: 129-132°C

*Beispiel 61:*

*1-(4-Amino-3-brom-5-pyrrolidinomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-pyrrolidinomethylbenzoyloxy)-2-hydroxyäthan analog Beispiel 1.

Schmelzpunkt des Hydrochlorids: 218-220°C

*Beispiel 62:*

*1-(4-Amino-3-brom-5-piperidinomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-piperidinomethylbenzoyloxy)-2-hydroxyäthan analog Beispiel 1.

Schmelzpunkt des Hydrochlorids: 208-209°C

*Beispiel 63:*

*1-(4-Amino-3-brom-5-morpholinomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-morpholinomethylbenzoyloxy)-2-hydroxyäthan analog Beispiel 1.

Schmelzpunkt des Hydrochlorids: 197-199°C

*Beispiel 64:*

*1-[4-Amino-3-brom-5-(4-methylpiperazinomethyl)benzoyloxy]-2-[1-(4-chlorbenzoyl)-5-methoxy-2-methyl-[1H]-indol-3-acetoxy]äthan*

Hergestellt aus 1-(Chlorbenzoyl)-5-methoxy-2-methyl-[1H]-indol-3-essigsäureimidazolid und 1-[4-Amino-3-brom-5-(4-methylpiperazinomethyl)benzoyloxy]-2-hydroxyäthan analog Beispiel 1.

Schmelzpunkt des Dihydrochlorids: 175-177°C

*Beispiel 65:*

*1-(4-Amino-3-brom-5-isopropylaminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-isopropylaminomethylbenzoyloxy)-2-hydroxyäthan analog Beispiel 1.

Schmelzpunkt des Dihydrochlorids: 115°C (Zers.)

*Beispiel 66:*

*1-[4-Amino-3-brom-5-(N-methyl-n-propylaminomethyl)benzoyloxy]-2-[1-(4-chlor-*

*benzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-methyl-n-propylaminomethyl)benzoyloxy]-2-hydroxyäthan analog Beispiel 1.

Schmelzpunkt des Hydrochlorids: 193-195°C (Zers.)

*Beispiel 67:*

*1-(4-Amino-3-brom-5-cyclohexylaminomethylbenzoyloxy)-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-(4-Amino-3-brom-5-cyclohexylaminomethylbenzoyloxy)-2-hydroxyäthan analog Beispiel 1.

Schmelzpunkt des Hydrochlorids: 177-179°C

*Beispiel 68:*

*1-[4-Amino-3-brom-5-(N-benzylmethylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(N-benzylmethylaminomethyl)benzoyloxy]-2-hydroxyäthan analog Beispiel 1. Das Hydrochlorid ist ein Öl.

$C_{37}H_{34}BrN_3O_6 \cdot HCl$ (733,1)

| | | | |
|---|---|---|---|
| berechnet: | C 60,62 | H 4,81 | Br 10,90 |
| | Cl 4,84 | N 5,73 | |
| gefunden: | C 60,35 | H 5,04 | Br 11,24 |
| | Cl 4,97 | N 5,51 | |

*Beispiel 69:*

*1-[4-Amino-3-brom-5-(2-diäthylaminoäthylaminomethyl)benzoyloxy]-2-[1-(4-chlorbenzoyl)-5-methoxy-2-methyl-[1H]-indol-3-acetoxy]äthan*

Hergestellt aus 1-(4-Chlorbenzoyl)-5-methoxy-2-methyl-[1H]-indol-3-essigsäureimidazolid und 1-[4-Amino-3-brom-5-(2-diäthylaminoäthylaminomethyl)benzoyloxy]-2-hydroxyäthan analog Beispiel 1.

Schmelzpunkt des Dihydrochlorids: 131-135°C

*Beispiel 70:*

*1-[4-Amino-3-brom-5-(N-äthylcyclopentylaminomethyl)benzoyloxy]-5-{2-[(2,6-dichlorphenyl)amino]phenylacetoxy}-n-pentan*

Hergestellt aus 2-[(2,6-Dichlorphenyl)amino]-phenylessigsäurenatriumsalz und 5-[4-Amino-3-brom-5-(N-äthylcyclopentylaminomethyl)-benzoyloxy]-n-pentylchlorid analog Beispiel 8. Das Hydrochlorid ist ein Öl.

$C_{34}H_{40}BrCl_2N_3O_4 \cdot HCl$ (742,0)

| | | | |
|---|---|---|---|
| berechnet: | C 55,04 | H 5,57 | Br 10,77 |
| | Cl 14,33 | N 5,66 | |
| gefunden: | C 55,08 | H 5,62 | Br 10,57 |
| | Cl 14,07 | N 5,47 | |

*Beispiel 71:*

*1-[4-Amino-3-brom-5-(N-äthylcycloheptylaminomethyl)benzoyloxy]-5-{2-[(2,6-dichlorphenyl)amino]phenylacetoxy}-n-pentan*

Hergestellt aus 2-[(2,6-Dichlorphenyl)amino]-phenylessigsäurenatriumsalz und 5-[4-Amino-3-brom-5-(N-äthylcycloheptylaminomethyl)-benzoyloxy]-n-pentylchlorid analog Beispiel 8.

Schmelzpunkt des Hydrochlorids: 107-110°C

*Beispiel 72:*

*1-(4-Amino-3-brom-5-tert.-butylaminomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-tert.-butylaminomethylbenzoyloxy)-2-hydroxyäthan analog Beispiel 1.

Schmelzpunkt des Hydrochlorids: 77-98°C

*Beispiel 73:*

*1-(4-Amino-3-brom-5-hexamethyleniminomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-hexamethyleniminomethylbenzoyloxy)-2-hydroxyäthan analog Beispiel 1.

Schmelzpunkt des Hydrochlorids: 66-80°C

*Beispiel 74:*

*1-[4-Amino-3-brom-5-(N-äthylcycloheptylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-äthylcycloheptylaminomethyl)benzoyloxy]-2-hydroxyäthan analog Beispiel 1.

Schmelzpunkt des Hydrochlorids: 68-79°C

*Beispiel 75:*

*1-[4-Amino-3-brom-5-(N-äthylcyclopentylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-äthylcyclopentylaminomethyl)benzoyloxy]-2-hydroxyäthan analog Beispiel 1.

Schmelzpunkt des Hydrochlorids: 63-71°C

*Beispiel 76:*

*1-[4-Amino-3-brom-5-(4-methylpiperazinomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(4-methylpiperazinomethyl)benzoyloxy]-2-hydroxyäthan analog Beispiel 1.

Schmelzpunkt des Dihydrochlorids: 182-184°C

*Beispiel 77:*

*1-(4-Amino-3-brom-5-morpholinomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-morpholinomethylbenzoyloxy)-2-hydroxyäthan analog Beispiel 1.

Schmelzpunkt des Hydrochlorids: 75°C (Zers.)

*Beispiel 78:*

*1-[4-Amino-3-brom-5-(N-methyl-n-propylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-methyl-n-propylaminomethyl)benzoyloxy]-2-hydroxyäthan analog Beispiel 1.

Schmelzpunkt des Hydrochlorids: ab 50°C (Zers.)

*Beispiel 79:*

*1-(4-Amino-3-brom-5-isopropylaminomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-isopropylaminomethylbenzoyloxy)-2-hydroxyäthan analog Beispiel 1.

Schmelzpunkt des Hydrochlorids: ab 70°C (Zers.)

*Beispiel 80:*

*1-(4-Amino-3-brom-5-cyclohexylaminomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-cyclohexylaminomethylbenzoyloxy)-2-hydroxyäthan analog Beispiel 1.

Schmelzpunkt des Hydrochlorids: ab 80°C (Zers.)

*Beispiel 81:*

*1-[4-Amino-3-brom-5-(N-benzylmethylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-benzylmethylaminomethyl)benzoyloxy]-2-hydroxyäthan analog Beispiel 1.

Schmelzpunkt des Hydrochlorids: ab 75°C (Zers.)

*Beispiel 82:*

*1-[4-Amino-3-brom-5-(N-cyclohexyl-n-propylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-cyclohexyl-n-propylaminomethyl)benzoyloxy]-2-hydroxyäthan analog Beispiel 1. Das Hydrochlorid ist ein Öl.

$C_{35}H_{41}BrN_2O_5 \cdot HCl$ (686,1)

| | | | |
|---|---|---|---|
| berechnet: | C 61,27 | H 6,17 | Br 11,65 |
| | Cl 5,17 | N 4,08 | |
| gefunden: | C 61,15 | H 6,36 | Br 11,60 |
| | Cl 5,13 | N 4,07 | |

*Beispiel 83:*

*1-[4-Amino-3-brom-5-(N-cyclohexylmethylaminomethyl)benzoyloxy]-2-(3-benzoyl-α-methylphenylacetoxy)äthan*

Hergestellt aus 3-Benzoyl-α-methylphenylessigsäureimidazolid und 1-[4-Amino-3-brom-5-(N-cyclohexylmethylaminomethyl)benzoyloxy]-2-hydroxyäthan analog Beispiel 1.

Schmelzpunkt des Hydrochlorids: 50-65°C (Zers.)

*Beispiel 84:*

*1-(4-Amino-3-brom-5-dimethylaminomethylbenzoyloxy)-2-(3-benzoyl-α-methylphenylacetoxy)äthan*

Hergestellt aus 3-Benzoyl-α-methylphenylessigsäureimidazolid und 1-(4-Amino-3-brom-5-dimethylaminomethylbenzoyloxy)-2-hydroxyäthan analog Beispiel 1.

Schmelzpunkt des Hydrochlorids: 65-75°C (Zers.)

*Beispiel 85:*

*1-(4-Amino-3-brom-5-pyrrolidinomethylbenzoyloxy)-2-(3-benzoyl-α-methylphenylacetoxy)-äthan*

Hergestellt aus 3-Benzoyl-α-methylphenylessigsäureimidazolid und 1-(4-Amino-3-brom-5-pyrrolidinomethylbenzoyloxy)-2-hydroxyäthan analog Beispiel 1.

Schmelzpunkt des Hydrochlorids: ab 75°C (sintern)

*Beispiel 86:*

*1-(4-Amino-3-brom-5-piperidinomethylbenzoyloxy)-2-(3-benzoyl-α-methylphenylacetoxy)-äthan*

Hergestellt aus 3-Benzoyl-α-methylphenylessigsäureimidazolid und 1-(4-Amino-3-brom-5-piperidinomethylbenzoyloxy)-2-hydroxyäthan analog Beispiel 1.

Schmelzpunkt des Hydrochlorids: ab 120°C (Zers.)

*Beispiel 87:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}amin*

Hergestellt aus 1-(4-Chlorbenzoyl)-5-methoxy-2-methyl-[1H]-indol-3-essigsäurechlorid und N-{2-[4-Amino-3-brom-5-(N-äthylcyclohexylaminomethyl)benzoyloxy]äthyl}-N-(2-hydroxyäthyl)aminhydrochlorid analog Beispiel 5. Das Hydrochlorid ist ein amorphes Pulver.

IR-Spektrum (Methylenchlorid):
Amid-CO 1680 $cm^{-1}$

Ester-CO 1720 und 1740 $cm^{-1}$
O-Methyl 2870 $cm^{-1}$
N-Alkyl 2950 $cm^{-1}$
UV-Spektrum (Äthanol): $\lambda_{max.}$ 230 nm (Schulter), 285 nm

*Beispiel 88:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}äthylamin*

Hergestellt aus 1-(4-Chlorbenzoyl)-5-methoxy-2-methyl-[1H]-indol-3-essigsäureimidazolid und N-{2-[4-Amino-3-brom-5-(N-äthylcyclohexylaminomethyl)benzoyloxy]äthyl}-N-(2-hydroxyäthyl)äthylamin analog Beispiel 3. Das Dihydrochlorid ist ein amorphes Pulver.

$C_{41}H_{50}BrClN_4O_6 \cdot 2\,HCl$ (883,2)

| | | | |
|---|---|---|---|
| berechnet: | C 55,76 | H 5,93 | Br 9,05 |
| | Cl 12,04 | N 6,34 | |
| gefunden: | C 55,48 | H 6,12 | Br 8,97 |
| | Cl 11,91 | N 6,24 | |

*Beispiel 89:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}-n-propylamin*

Hergestellt aus 1-(4-Chlorbenzoyl)-5-methoxy-2-methyl-[1H]-indol-3-essigsäurechlorid und N-{2-[4-Amino-3-brom-5-(N-äthylcyclohexylaminomethyl)benzoyloxy]äthyl}-N-(2-hydroxyäthyl)-n-propylamin in Dichlormethan analog Beispiel 5. Öl.

$C_{42}H_{52}BrClN_4O_6$ (824,3)

| | | | |
|---|---|---|---|
| berechnet: | C 61,20 | H 6,36 | Br 9,70 |
| | Cl 4,30 | N 6,80 | |
| gefunden: | C 60,80 | H 6,61 | Br 9,94 |
| | Cl 4,30 | N 6,77 | |

*Beispiel 90:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}isopropylamin*

Hergestellt aus 1-(4-Chlorbenzoyl)-5-methoxy-2-methyl-[1H]-indol-3-essigsäurechlorid und N-{2-[4-Amino-3-brom-5-(N-äthylcyclohexylaminomethyl)benzoyloxy]äthyl}-N-(2-hydroxyäthyl)isopropylamin in Dichlormethan bei 0°C analog Beispiel 5. Öl.

$C_{42}H_{52}BrClN_4O_6$ (824,3)

| | | | |
|---|---|---|---|
| berechnet: | C 61,20 | H 6,36 | Br 9,70 |
| | Cl 4,30 | N 6,80 | |
| gefunden: | C 61,00 | H 6,51 | Br 10,00 |
| | Cl 4,43 | N 6,61 | |

*Beispiel 91:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}-tert.-butylamin*

Hergestellt aus 4-Amino-3-brom-5-(N-äthylcyclohexylaminomethyl)benzoylchloridhydrochlorid und N-{2-[1-(4-Chlorbenzoyl)-5-methoxy-2-methyl-[1H]-indol-3-acetoxy]äthyl}-N-(2-hydroxyäthyl)-tert.-butylamin analog Beispiel 10. Schaum.

$C_{43}H_{54}BrClN_4O_6$ (838,3)

| | | | |
|---|---|---|---|
| berechnet: | C 61,61 | H 6,49 | Br 9,53 |
| | Cl 4,23 | N 6,68 | |
| gefunden: | C 61,50 | H 6,63 | Br 9,69 |
| | Cl 4,30 | N 6,53 | |

*Beispiel 92:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}benzylamin*

Hergestellt aus 1-(4-Chlorbenzoyl)-5-methoxy-2-methyl-[1H]-indol-3-essigsäure und N-{2-[4-Amino-3-brom-5-(N-äthylcyclohexylaminomethyl)benzoyloxy]äthyl}-N-(2-hydroxyäthyl)benzylamin analog Beispiel 4. Öl.

IR-Spektrum (Methylenchlorid):
Amid-CO 1675 $cm^{-1}$
Ester-CO 1700 und 1730 $cm^{-1}$
O-Methyl 2840 $cm^{-1}$
N-Alkyl 2940 $cm^{-1}$
UV-Spektrum (Äthanol): $\lambda_{max.}$ 235 nm (Schulter), 290 nm

*Beispiel 93:*

*N-{2-[4-Amino-3-brom-5-(N-äthylcyclohexylaminomethyl)benzoyloxy]äthyl}-N-{2-[1-(4-chlorbenzoyl)-5-methoxy-2-methyl-[1H]-indol-3-acetoxy]äthyl}anilin*

Hergestellt aus 1-(4-Chlorbenzoyl)-5-methoxy-2-methyl-[1H]-indol-3-essigsäure und N-{2-[4-Amino-3-brom-5-(N-äthylcyclohexylaminomethyl)benzoyloxy]äthyl}-N-(2-hydroxyäthyl)anilin analog Beispiel 4. Öl.

$C_{45}H_{50}BrClN_4O_6$ (858,3)

| | | | |
|---|---|---|---|
| berechnet: | C 62,97 | H 5,87 | Br 9,31 |
| | Cl 4,13 | N 6,53 | |
| gefunden: | C 62,90 | H 6,11 | Br 9,54 |
| | Cl 4,23 | N 6,59 | |

*Beispiel 94:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]methylamin*

Hergestellt aus 2-[(2,6-Dichlorphenyl)amino]-phenylessigsäurenatriumsalz und N-{2-[4-Amino-3-brom-5-(N-äthylcyclohexylaminomethyl)benzoyloxy]äthyl}-N-(2-chloräthyl)-methylamin analog Beispiel 8. Öl.

$C_{35}H_{43}BrCl_2N_4O_4$ (734,6)

| | | | |
|---|---|---|---|
| berechnet: | C 57,23 | H 5,90 | Br 10,87 |
| | Cl 9,65 | N 7,62 | |

| | | | |
|---|---|---|---|
| gefunden: | C 57,60 | H 6,12 | Br 10,58 |
| | Cl 9,39 | N 7,54 | |

*Beispiel 95:*

*N-{2-[4-Amino-3-brom-5-(N-äthylcyclohexylaminomethyl)benzoyloxy]-äthyl}-N-[2-{2-[(2,6-dichlorphenyl)amino]phenylacetoxy}äthyl]äthylamin*

Hergestellt aus 2-[(2,6-Dichlorphenyl)amino]-phenylessigsäurenatriumsalz und N-{2-[4-Amino-3-brom-5-(N-äthylcyclohexylaminomethyl)benzoyloxy]äthyl}-N-(2-chloräthyl)-äthylamin analog Beispiel 8. Das Dihydrochlorid ist ein amorphes Pulver.

IR-Spektrum (Methylenchlorid):
Ester-CO 1720 und 1740 $cm^{-1}$
N-Alkyl 2940 $cm^{-1}$
UV-Spektrum (Äthanol): $\lambda_{max.}$ 285 nm

*Beispiel 96:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]-n-propylamin*

Hergestellt aus 2-[(2,6-Dichlorphenyl)amino]-phenylessigsäurenatriumsalz und N-{2-[4-Amino-3-brom-5-(N-äthylcyclohexylaminomethyl)benzoyloxy]äthyl}-N-(2-chloräthyl)-n-propylamin analog Beispiel 8. Öl.

$C_{37}H_{47}BrCl_2N_4O_4$ (762,6)

| | | | |
|---|---|---|---|
| berechnet: | C 58,27 | H 6,21 | Br 10,48 |
| | Cl 9,30 | N 7,35 | |
| gefunden: | C 58,50 | H 6,45 | Br 10,58 |
| | Cl 9,31 | N 7,06 | |

*Beispiel 97:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]isopropylamin*

Hergestellt aus 2-[(2,6-Dichlorphenyl)amino]-phenylessigsäurenatriumsalz und N-{2-[4-Amino-3-brom-5-(N-äthylcyclohexylaminomethyl)benzoyloxy]äthyl}-N-(2-chloräthyl)isopropylamin analog Beispiel 8. Öl.

$C_{37}H_{47}BrCl_2N_4O_4$ (762,6)

| | | | |
|---|---|---|---|
| berechnet: | C 58,27 | H 6,21 | Br 10,48 |
| | Cl 9,30 | N 7,35 | |
| gefunden: | C 58,40 | H 6,37 | Br 10,22 |
| | Cl 9,05 | N 7,08 | |

*Beispiel 98:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]-n-butylamin*

Hergestellt aus 2-[(2,6-Dichlorphenyl)amino]-phenylessigsäure und N-{2-[4-Amino-3-brom-5-(N-äthylcyclohexylaminomethyl)benzoyloxy]-äthyl}-N-(2-hydroxyäthyl)-n-butylamin analog Beispiel 4. Öl.

IR-Spektrum (Methylenchlorid):
Ester-CO 1710 $cm^{-1}$
N-Alkyl 2935 $cm^{-1}$
UV-Spektrum (Äthanol): $\lambda_{max.}$ 230 nm (Schulter), 284 nm

*Beispiel 99:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]benzylamin*

Hergestellt aus 2-[(2,6-Dichlorphenyl)amino]-phenylessigsäure und N-{2-[4-Amino-3-brom-5-(N-äthylcyclohexylaminomethyl)benzoyloxy]-äthyl}-N-(2-hydroxyäthyl)benzylamin analog Beispiel 4. Öl.

IR-Spektrum (Methylenchlorid):
Ester-CO 1710 $cm^{-1}$
N-Alkyl 2935 $cm^{-1}$
UV-Spektrum (Äthanol): $\lambda_{max.}$ 235 nm (Schulter), 280 nm

*Beispiel 100:*

*N-{2-[4-Amino-3-brom-5-(N-äthylcyclohexylaminomethyl)benzoyloxy]äthyl}-N-[2-{2-[(2,6-dichlorphenyl)amino]phenylacetoxy}äthyl]anilin*

Hergestellt aus 2-[(2,6-Dichlorphenyl)amino]-phenylessigsäure und N-{2-[4-Amino-3-brom-5-(N-äthylcyclohexylaminomethyl)benzoyloxy]-äthyl}-N-(2-hydroxyäthyl)anilin analog Beispiel 4. Schaum.

IR-Spektrum (Methylenchlorid):
Ester-CO 1710 $cm^{-1}$
N-Alkyl 2935 $cm^{-1}$
UV-Spektrum (Äthanol): $\lambda_{max.}$ 250 nm, 280 nm

*Beispiel 101:*

*N-{2-[4-Amino-3-brom-5-(N-äthylcyclohexylaminomethyl)benzoyloxy]äthyl}-N-[2-(3-benzoyl-α-methylphenylacetoxy)äthyl]amin*

Hergestellt aus 3-Benzoyl-α-methylphenylessigsäurechlorid und N-{2-[4-Amino-3-brom-5-(N-äthylcyclohexylaminomethyl)benzoyloxy]-äthyl}-N-(2-hydroxyäthyl)aminhydrochlorid analog Beispiel 5. Das Dihydrochlorid ist ein amorphes Pulver.

$C_{36}H_{44}BrN_2O_5 \cdot 2\ HCl$ (751,6)

| | | | |
|---|---|---|---|
| berechnet: | C 57,53 | H 6,17 | Br 10,63 |
| | Cl 9,43 | N 5,59 | |
| gefunden: | C 57,25 | H 6,43 | Br 10,38 |
| | Cl 9,18 | N 5,46 | |

*Beispiel 102:*

*N-{2-[4-Amino-3-brom-5-(N-äthylcyclohexylaminomethyl)benzoyloxy]äthyl}-N-[2-(3-benzoyl-α-methylphenylacetoxy)äthyl]methylamin*

Hergestellt aus 3-Benzoyl-α-methylphenylessigsäureimidazolid und N-{2-[4-Amino-3-brom-5-(N-äthylcyclohexylaminomethyl)-benzoyloxy]äthyl}-N-(2-hydroxyäthyl)methylamin analog Beispiel 3. Das Dihydrochlorid ist ein amorphes Pulver.

$C_{37}H_{46}BrN_3O_5 \cdot 2\ HCl$ (756,6)

| | | | |
|---|---|---|---|
| berechnet: | C 58,04 | H 6,32 | Br 10,44 |
| | Cl 9,26 | N 5,48 | |
| gefunden: | C 57,90 | H 6,26 | Br 11,11 |
| | Cl 8,96 | N 5,35 | |

*Beispiel 103:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]äthylamin*

Hergestellt aus 3-Benzoyl-α-methylphenyl-essigsäureimidazolid und N-{2-[4-Amino-3-brom-5-(N-äthylcyclohexylaminomethyl)-benzoyloxy]äthyl}-N-(2-hydroxyäthyl)äthylamin analog Beispiel 3. Das Dihydrochlorid ist ein amorphes Pulver.

$C_{38}H_{48}BrN_3O_5 \cdot 2\ HCl$ (779,6)

| | | | |
|---|---|---|---|
| berechnet: | C 58,54 | H 6,46 | Br 10,25 |
| | Cl 9,10 | N 5,39 | |
| gefunden: | C 58,20 | H 6,54 | Br 10,18 |
| | Cl 9,06 | N 5,38 | |

*Beispiel 104:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]-n-propylamin*

Hergestellt aus 3-Benzoyl-α-methylphenyl-essigsäureimidazolid und N-{2-[4-Amino-3-brom-5-(N-äthylcyclohexylaminomethyl)benzoyloxy]äthyl}-N-(2-hydroxyäthyl)-n-propylamin in siedendem Tetrahydrofuran analog Beispiel 3. Öl.

IR-Spektrum (Methylenchlorid):
Keton-CO 1650 $cm^{-1}$
Ester-CO 1690 und 1720 $cm^{-1}$
N-Alkyl 2850 $cm^{-1}$
UV-Spektrum (Äthanol): $\lambda_{max.}$ 253 nm, 290 nm

*Beispiel 105:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]isopropyl-amin*

Hergestellt aus 3-Benzoyl-α-methylphenyl-essigsäureimidazolid und N-{2-[4-Amino-3-brom-5-(N-äthylcyclohexylaminomethyl)-benzoyloxy]äthyl}-N-(2-hydroxyäthyl)isopropylamin analog Beispiel 3. Öl.

$C_{39}H_{50}BrN_3O_5$ (720,8)

| | | | | |
|---|---|---|---|---|
| berechnet: | C 64,99 | H 6,99 | Br 11,09 | N 5,83 |
| gefunden: | C 64,80 | H 7,28 | Br 10,95 | N 5,59 |

*Beispiel 106:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]-n-butylamin*

Hergestellt aus 3-Benzoyl-α-methylphenylessigsäure und N-{2-[4-Amino-3-brom-5-(N-äthylcyclohexylaminomethyl)benzoyloxy]äthyl}-N-(2-hydroxyäthyl)-n-butylamin analog Beispiel 4. Öl.

IR-Spektrum (Methylenchlorid):
Keton-CO 1660 $cm^{-1}$
Ester-CO 1705 und 1730 $cm^{-1}$
N-Alkyl 2935 $cm^{-1}$
UV-Spektrum (Äthanol): $\lambda_{max.}$ 255 nm, 290 nm

*Beispiel 107:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]-tert.-butylamin*

Hergestellt aus 3-Benzoyl-α-methylphenylessigsäure und N-{2-[4-Amino-3-brom-5-(N-äthylcyclohexylaminomethyl)benzoyloxy]äthyl}-N-(2-hydroxyäthyl)-tert.-butylamin analog Beispiel 4. Das Dihydrochlorid ist ein amorphes Pulver.

IR-Spektrum (Methylenchlorid):
Keton-CO 1640 $cm^{-1}$
Ester-CO 1710 und 1730 $cm^{-1}$
N-Alkyl 2940 $cm^{-1}$
UV-Spektrum (Äthanol): $\lambda_{max.}$ 256 nm, 280 nm

*Beispiel 108:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]benzylamin*

Hergestellt aus 3-Benzoyl-α-methylphenylessigsäure und N-{2-[4-Amino-3-brom-5-(N-äthylcyclohexylaminomethyl)benzoyloxy]äthyl}-N-(2-hydroxyäthyl)benzylamin analog Beispiel 4. Öl.

IR-Spektrum (Methylenchlorid):
Keton-CO 1660 $cm^{-1}$
Ester-CO 1710 und 1730 $cm^{-1}$
N-Alkyl 2940 $cm^{-1}$
UV-Spektrum (Äthanol): $\lambda_{max.}$ 252 nm, 288 nm

*Beispiel 109:*

*N-{2-[4-Amino-3-brom-5-(N-äthylcyclohexyl-aminomethyl)benzoyloxy]äthyl}-N-[2-(3-benzoyl-α-methylphenylacetoxy)äthyl]anilin*

Hergestellt aus 3-Benzoyl-α-methylphenylessigsäure und N-{2-[4-Amino-3-brom-5-(N-äthylcyclohexylaminomethyl)benzoyloxy]äthyl}-N-(2-hydroxyäthyl)anilin analog Beispiel 4. Öl.

IR-Spektrum (Methylenchlorid):
Keton-CO 1660 $cm^{-1}$
Ester-CO 1710 und 1735 $cm^{-1}$
N-Alkyl 2940 $cm^{-1}$
UV-Spektrum (Äthanol): $\lambda_{max.}$ 250 nm, 290 nm

*Beispiel 110:*

*N-{2-[4-Amino-3-brom-5-(N-äthylcyclohexyl-aminomethyl)benzoyloxy]äthyl}-N-{2-[(+)-6-methoxy-2-α-methyl-2-naphthalinacetoxy]-äthyl}amin*

Hergestellt aus (+)-6-Methoxy-α-methyl-2-naphthalinessigsäurechlorid und N-äthylcyclo-hexylaminomethyl)benzoyloxy]äthyl}-N-(2-hydroxyäthyl)aminhydrochlorid analog Beispiel 5. Das Dihydrochlorid ist ein amorphes Pulver.

IR-Spektrum (Methylenchlorid):
Ester-CO 1720 und 1740 $cm^{-1}$
O-Methyl 2850 $cm^{-1}$
N-Alkyl 2930 $cm^{-1}$
UV-Spektrum (Äthanol): $\lambda_{max.}$ 221 nm, 282 nm

*Beispiel 111:*

*N-{2-[4-Amino-3-brom-5-(N-äthylcyclohexylaminomethyl)benzoyloxy]äthyl}-N-{2-[(+)-6-methoxy-2-α-methyl-2-naphthalinacetoxy]-äthyl}methylamin*

Hergestellt aus (+)-6-Methoxy-α-methyl-2-naphthalinessigsäureimidazolid und N-{2-[4-Amino-3-brom-5-(N-äthylcyclohexylaminomethyl)benzoyloxy]äthyl}-N-(2-hydroxyäthyl)methylamin analog Beispiel 3. Das Dihydrochlorid ist ein amorphes Pulver.
IR-Spektrum (Kaliumbromid):
Ester-CO 1720 und 1730 $cm^{-1}$
O-Methyl 2860 $cm^{-1}$
N-Alkyl 2940 $cm^{-1}$
UV-Spektrum (Äthanol): $\lambda_{max.}$ 233 nm, 285 nm

*Beispiel 112:*

*N-{2-[4-Amino-3-brom-5-(N-äthylcyclohexylaminomethyl)benzoyloxy]äthyl}-N-{2-[(+)-6-methoxy-2-α-methyl-2-naphthalinacetoxy]-äthyl}äthylamin*

Hergestellt aus (+)-6-Methoxy-α-methyl-2-naphthalinessigsäureimidazolid und N-{2-[4-Amino-3-brom-5-(N-äthylcyclohexylaminomethyl)benzoyloxy]äthyl}-N-(2-hydroxyäthyl)äthylamin analog Beispiel 3. Das Dihydrochlorid ist ein amorphes Pulver.

$C_{36}H_{48}BrN_3O_5 \cdot 2\,HCl$ (755,7)

| | | | |
|---|---|---|---|
| berechnet: | C 57,22 | H 6,67 | Br 10,58 |
| | Cl 9,38 | N 5,56 | |
| gefunden: | C 57,00 | H 6,78 | Br 10,62 |
| | Cl 9,47 | N 5,37 | |

*Beispiel 113:*

*N-{2-[4-Amino-3-brom-5-(N-äthylcyclohexylaminomethyl)benzoyloxy]äthyl}-N-{2-[(+)-6-methoxy-α-methyl-2-naphthalinacetoxy]äthyl}-n-propylamin*

Hergestellt aus (+)-6-Methoxy-α-methyl-2-naphthalinessigsäureimidazolid und N-{2-[4-Amino-3-brom-5-(N-äthylcyclohexylaminomethyl)benzoyloxy]äthyl}-N-(2-hydroxyäthyl-n-propylamin in siedendem Tetrahydrofuran analog Beispiel 3. Öl.

$C_{37}H_{50}BrN_3O_5$ (696,7)

| | | | | |
|---|---|---|---|---|
| berechnet: | C 63,78 | H 7,23 | Br 11,47 | N 6,03 |
| gefunden: | C 63,90 | H 7,51 | Br 11,54 | N 5,97 |

*Beispiel 114:*

*N-{-2-[4-Amino-3-brom-5-(N-äthylcyclohexylaminomethyl)benzoyloxy]äthyl}-N-{2-[(+)-6-methoxy-α-methyl-2-naphthalinacetoxy]äthyl}-isopropylamin*

Hergestellt aus (+)-6-Methoxy-α-methyl-2-naphthalinessigsäureimidazolid und N-{2-[4-Amino-3-brom-5-(N-äthylcyclohexylaminomethyl)benzoyloxy]äthyl}-N-(2-hydroxyäthyl)-isopropylamin analog Beispiel 3. Öl.

$C_{37}H_{50}BrN_3O_5$ (696,7)

| | | | | |
|---|---|---|---|---|
| berechnet: | C 63,78 | H 7,23 | Br 11,47 | N 6,03 |
| gefunden: | C 63,80 | H 7,40 | Br 11,40 | N 6,00 |

*Beispiel 115:*

*N-{2-[4-Amino-3-brom-5-(N-äthylcyclohexylaminomethyl)benzoyloxy]äthyl}-N-{2-[α-methyl-4-(2-methylpropyl)phenylacetoxy]äthyl}amin*

Hergestellt aus α-Methyl-4-(2-methylpropyl)-phenylessigsäurechlorid und N-{2-[4-Amino-3-brom-5-(N-äthylcyclohexylaminomethyl)benzoyloxy]äthyl}-N-(2-hydroxyäthyl)amin-hydrochlorid analog Beispiel 5. Das Dihydrochlorid ist ein amorphes Pulver.
IR-Spektrum (Methylenchlorid):
Ester-CO 1720 und 1730 $cm^{-1}$
N-Alkyl 2950 $cm^{-1}$
UV-Spektrum (Äthanol): $\lambda_{max.}$ 218 nm, 284 nm

*Beispiel 116:*

*N-{2-[4-Amino-3-brom-5-(N-äthylcyclohexylaminomethyl)benzoyloxy]äthyl}-N-{2-[α-methyl-4-(2-methylpropyl)phenylacetoxy]äthyl}methylamin*

Hergestellt aus α-Methyl-4-(2-methylpropyl)-phenylessigsäureimidazolid und N-{2-[4-Amino-3-brom-5-(N-äthylcyclohexylaminomethyl)benzoyloxy]äthyl}-N-(2-hydroxyäthyl)methylamin analog Beispiel 3. Öl.

$C_{34}H_{50}BrN_3O_4$ (644,7)

| | | | | |
|---|---|---|---|---|
| berechnet: | C 63,34 | H 7,82 | Br 12,40 | N 6,52 |
| gefunden: | C 63,10 | H 7,84 | Br 12,10 | N 6,59 |

*Beispiel 117:*

*N-{2-[4-Amino-3-brom-5-(N-äthylcyclohexylaminomethyl)benzoyloxy]äthyl}-N-{2-[α-methyl-4-(2-methylpropyl)phenylacetoxy]äthyl}-n-propylamin*

Hergestellt aus α-Methyl-4-(2-methylpropyl)-phenylessigsäureimidazolid und N-{2-[4-Amino-3-brom-5-(N-äthylcyclohexylaminomethyl)-benzoyloxy]äthyl}-N-(2-hydroxyäthyl)-n-propylamin analog Beispiel 3. Öl.
IR-Spektrum (Methylenchlorid):
Ester-CO 1700 und 1725 $cm^{-1}$
N-Alkyl 2820 $cm^{-1}$
UV-Spektrum (Äthanol): $\lambda_{max.}$ 230 nm (Schulter), 294 nm

*Beispiel 118:*

*N-{2-[4-Amino-3-brom-5-(N-äthylcyclohexylaminomethyl)benzoyloxy]äthyl}-N-{2-[α-methyl-4-(2-methylpropyl)phenylacetoxy]äthyl} isopropylamin*

Hergestellt aus α-Methyl-4-(2-methylpropyl)-phenylessigsäureimidazolid und N-{2-[4-Amino-3-brom-5-(N-äthylcyclohexylaminomethyl)-

benzoyloxy]äthyl}-N-(2-hydroxyäthyl)isopropylamin in siedendem Tetrahydrofuran analog Beispiel 3. Öl.

$C_{36}H_{54}BrN_3O_4$ (672,8)

| | | | | |
|---|---|---|---|---|
| berechnet: | C 64,27 | H 8,09 | Br 11,88 | N 6,25 |
| gefunden: | C 64,50 | H 8,28 | Br 12,05 | N 6,32 |

*Beispiel 119:*

*N-[2-(4-Amino-3-brom-5-diäthylaminomethylbenzoyloxy)äthyl]-N-{2-[1-(4-chlorbenzoyl)-5-methoxy-2-methyl-[1H]-indol-3-acetoxy]-äthyl}methylamin*

Hergestellt aus 1-(4-Chlorbenzoyl)-5-methoxy-2-methyl-[1H]-indol-3-essigsäurechlorid und N-[2-(4-Amino-3-brom-5-diäthylaminomethylbenzoyloxy)äthyl]-N-(2-hydroxyäthyl)-methylamin analog Beispiel 5. Öl.

IR-Spektrum (Methylenchlorid):
Amid-CO 1690 $cm^{-1}$
Ester-CO 1705 und 1735 $cm^{-1}$
UV-Spektrum (Äthanol): $\lambda_{max.}$ 230 nm (Schulter), 290 nm

*Beispiel 120:*

*2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]diäthyloxid*

Hergestellt aus 4-Amino-3-brom-5-(N-äthylcyclohexylaminomethyl)benzoylchloridhydrochlorid und 2-[1-(4-Chlorbenzoyl)-5-methoxy-2-methyl-[1H]-indol-3-acetoxy]-2'-hydroxydiäthyloxid analog Beispiel 10. Öl.

IR-Spektrum (Methylenchlorid):
Amid-CO 1690 $cm^{-1}$
Ester-CO 1710 und 1735 $cm^{-1}$
O-Methyl 1850 $cm^{-1}$
N-Alkyl 2920 $cm^{-1}$
UV-Spektrum (Äthanol): $\lambda_{max.}$ 230 nm (Schulter), 295 nm

*Beispiel 121:*

*2-[Amino-3-brom-5-(N-äthylcyclohexylaminomethyl)benzoloxy]-2'-[(+)-6-methoxy-α-methyl-2-naphthalinacetoxy]diäthyloxid*

Hergestellt aus 2-[4-Amino-3-brom-5-(N-äthylcyclohexylaminomethyl)benzoyloxy]-2'-hydroxydiäthyloxid und (+)-6-Methoxy-α-methyl-2-naphthalinessigsäureimidazolid analog Beispiel 3. Öl.

$C_{34}H_{43}BrN_2O_6$ (655,6)

| | | | | |
|---|---|---|---|---|
| berechnet: | C 62,28 | H 6,61 | Br 12,19 | N 4,27 |
| gefunden: | C 62,20 | H 6,78 | Br 12,10 | N 4,09 |

*Beispiel 122:*

*2-[4-Amino-3-brom-5-(N-äthylcyclohexylaminomethyl)benzoyloxy]-2'-{2-[(2,6-dichlorphenyl)amino]phenylacetoxy}diäthyloxid*

Hergestellt aus 2-[4-Amino-3-brom-5-(N-äthylcyclohexylaminomethyl)benzoyloxy]-2'-chlordiäthyloxid und 2-[(2,6-Dichlorphenyl)-amino]phenylessigsäurenatriumsalz analog Beispiel 8. Das Hydrochlorid ist ein amorphes Pulver.

IR-Spektrum (Methylenchlorid):
Ester-CO 1710 und 1720 $cm^{-1}$
N-Alkyl 2950 $cm^{-1}$
UV-Spektrum (Äthanol): $\lambda_{max.}$ 283 nm

*Beispiel 123:*

*2-[4-Amino-3-brom-5-(N-äthylcyclohexylaminomethyl)benzoyloxy]-2'-(3-benzoyl-α-methylphenylacetoxy)diäthyloxid*

Hergestellt aus 2-[4-Amino-3-brom-5-(N-äthylcyclohexylaminomethyl)benzoyloxy]-2'-hydroxydiäthyloxid und 3-Benzoyl-α-methylphenylessigsäureimidazolid analog Beispiel 3. Öl.

$C_{36}H_{43}BrN_2O_6$ (679,7)

| | | | | |
|---|---|---|---|---|
| berechnet: | C 63,62 | H 6,38 | Br 11,76 | N 4,12 |
| gefunden: | C 63,40 | H 6,58 | Br 11,35 | N 4,03 |

*Beispiel 124:*

*2-[4-Amino-3-brom-5-(N-äthylcyclohexylaminomethyl)benzoyloxy]-2'-[α-methyl-4-(2-methylpropyl)phenylacetoxy]diäthyloxid*

Hergestellt aus 2-[4-Amino-3-brom-5-(N-äthylcyclohexylaminomethyl)benzoyloxy]-2'-hydroxydiäthyloxid und α-Methyl-4-(2-methylpropyl)phenylessigsäureimidazolid analog Beispiel 3. Öl.

$C_{33}H_{47}BrN_2O_5$ (631,7)

| | | | | |
|---|---|---|---|---|
| berechnet: | C 62,75 | H 7,50 | Br 12,65 | N 4,44 |
| gefunden: | C 62,60 | H 7,58 | Br 12,90 | N 4,54 |

*Beispiel 125:*

*2-[4-Amino-3-brom-5-(N-äthylcyclohexylaminomethyl)benzoyloxy]-2'-[1-(4-chlorbenzoyl)-5-methoxy-2-methyl-[1H]-indol-3-acetoxy]diäthylsulfid*

Hergestellt aus 2-[4-Amino-3-brom-5-(N-äthylcyclohexylaminomethyl)benzoyloxy]-2'-hydroxy-2'-hydroxydiäthylsulfid und 1-(4-Chlorbenzoyl)-5-methoxy-2-methyl-[1H]-indol-3-essigsäurechlorid analog Beispiel 5. Das Hydrochlorid ist ein amorphes Pulver.

$C_{39}H_{45}BrClN_3O_6S \cdot HCl$ (835,7)

| | | | |
|---|---|---|---|
| berechnet: | C 56,05 | H 5,54 | Br 9,56 |
| | Cl 8,49 | N 5,02 | |
| gefunden: | C 56,00 | H 5,80 | Br 9,74 |
| | Cl 8,69 | N 4,84 | |

*Beispiel 126:*

*2-[4-Amino-3-brom-5-(N-äthylcyclohexylaminomethyl)benzoyloxy]-2'-[(+)-6-methoxy-α-methyl-2-naphthalinacetoxy]diäthylsulfid*

Hergestellt aus 2-[4-Amino-3-brom-5-(N-äthylcyclohexylaminomethyl)benzoyloxy]-2'-hydroxydiäthylsulfid und (+)-6-Methoxy-α-methyl-2-naphthalinessigsäureimidazolid analog Beispiel 3. Öl.

IR-Spektrum (Methylenchlorid):
Ester-CO 1705 und 1730 $cm^{-1}$
O-Methyl 2860 $cm^{-1}$

N-Alkyl 2930 $cm^{-1}$
UV-Spektrum (Äthanol): $\lambda_{max.}$ 233 nm, 298 nm

*Beispiel 127:*

*2-[4-Amino-3-brom-5-(N-äthylcyclohexylaminomethyl)benzoyloxy]-2'-{2-[(2,6-dichlorphenyl)amino]phenylacetoxy}diäthylsulfid*

Hergestellt aus 2-[4-Amino-3-brom-5-(N-äthylcyclohexylaminomethyl)benzoyloxy]-2'-chlordiäthylsulfid und 2-[(2,6-Dichlorphenyl)-amino]phenylessigsäurenatriumsalz analog Beispiel 8. Öl.

$C_{34}H_{40}BrCl_2N_3O_4S$ (737,6)

| | | | |
|---|---|---|---|
| berechnet: | C 55,36 | H 5,47 | Br 10,83 |
| | Cl 9,61 | N 5,69 | S 4,35 |
| gefunden: | C 55,20 | H 5,90 | Br 10,65 |
| | Cl 9,47 | N 5,59 | S 4,31 |

*Beispiel 128:*

*2-[4-Amino-3-brom-5-(N-äthylcyclohexylaminomethyl)benzoyloxy]-2'-(3-benzoyl-α-methylphenylacetoxy)diäthylsulfid*

Hergestellt aus 2-[4-Amino-3-brom-5-(N-äthylcyclohexylaminomethyl)benzoyloxy]-2'-hydroxydiäthylsulfid und 3-Benzoyl-α-methylphenylessigsäureimidazolid analog Beispiel 3. Öl.

$C_{36}H_{43}BrN_2O_5S$ (695,7)

| | | | |
|---|---|---|---|
| berechnet: | C 62,15 | H 6,23 | Br 11,49 |
| | N 4,02 | S 4,60 | |
| gefunden: | C 62,20 | H 6,50 | Br 11,25 |
| | N 3,87 | S 4,72 | |

*Beispiel 129:*

*2-[4-Amino-3-brom-5-(N-äthylcyclohexylaminomethyl)benzoyloxy]-2'-[2-fluor-α-methyl-(1,1'-biphenyl)-4-acetoxy]diäthylsulfid*

Hergestellt aus 2-[4-Amino-3-brom-5-(N-äthylcyclohexylaminomethyl)benzoyloxy]-2'-hydroxydiäthylsulfid und 2-Fluor-α-methyl-(1,1'-biphenyl)-4-essigsäure analog Beispiel 4. Öl.

IR-Spektrum (Methylenchlorid):
Ester-CO 1710 und 1735 $cm^{-1}$
N-Alkyl 2935 $cm^{-1}$
UV-Spektrum (Äthanol): $\lambda_{max.}$ 232 nm, 288 nm

*Beispiel 130:*

*2-[Amino-3-brom-5-(N-äthylcyclohexylaminomethyl)benzoyloxy]-2'-[2-fluor-α-methyl-(1,1'-biphenyl)-4-acetoxy]diäthyloxid*

Hergestellt aus 2-[4-Amino-3-brom-5-(N-äthylcyclohexylaminomethyl)benzoyloxy]-2'-hydroxydiäthyloxid und 2-Fluor-α-methyl-(1,1'-biphenyl)-4-essigsäure analog Beispiel 4. Öl.

$C_{35}H_{42}BrFN_2O_5$ (669,7)

| | | | | |
|---|---|---|---|---|
| berechnet: | C 62,78 | H 6,32 | Br 11,93 | N 4,18 |
| gefunden: | C 63,04 | H 6,48 | Br 11,90 | N 4,07 |

*Beispiel 131:*

*2-[4-Amino-3-brom-5-(N-äthylcyclohexylaminomethyl)benzoyloxy]-2'-{2-[(2,6-dichlorphenyl)amino]phenylacetoxy}diäthylsulfoxid*

Hergestellt aus 2-[3-Amino-3-brom-5-(N-äthylcyclohexylaminomethyl)benzoyloxy]-2'-{2-[(2,6-dichlorphenyl)amino]phenylacetoxy}diäthylsulfid mit Wasserstoffperoxid analog Beispiel 14. Schaum.

IR-Spektrum (Methylenchlorid):
Sulfoxid 1040 $cm^{-1}$
Ester-CO 1710 $cm^{-1}$ (breit)
N-Alkyl 2930 $cm^{-1}$
UV-Spektrum (Äthanol): $\lambda_{max.}$ 300 nm

*Beispiel 132*

*2-[4-Amino-3-brom-5-(N-äthylcyclohexylaminomethyl)benzoyloxy]-2'-{2-[(2,6-dichlorphenyl)amino]phenylacetoxy}diäthylsulfon*

Hergestellt aus 2-[4-Amino-3-brom-5-(N-äthylcyclohexylaminomethyl)benzoyloxy]-2'-{2-[(2,6-dichlorphenyl)amino]phenylacetoxy}diäthylsulfid mit Wasserstoffperoxid analog Beispiel 15. Schaum.

$C_{34}H_{40}BrCl_2N_3O_6S$ (769,6)

| | | | |
|---|---|---|---|
| berechnet: | C 53,06 | H 5,24 | Br 10,38 |
| | Cl 9,21 | N 5,46 | |
| gefunden: | C 53,01 | H 5,35 | Br 10,56 |
| | Cl 9,33 | N 5,34 | |

*Beispiel 133:*

*2-(4-Amino-3-brom-5-diäthylaminomethylbenzoyloxy)-2'-{2-[(2,6-dichlorphenyl)-amino]phenylacetoxy}diäthyloxid*

Hergestellt aus 2'-{2-[(2,6-Dichlorphenyl)-amino]phenylacetoxy}-2-hydroxydiäthyloxid und 4-Amino-3-brom-5-diäthylaminomethylbenzoylchloridhydrochlorid analog Beispiel 7. Öl.

IR-Spektrum (Methylenchlorid):
Ester-CO 1710 $cm^{-1}$
N-Alkyl 2960 $cm^{-1}$
UV-Spektrum (Äthanol): $\lambda_{max.}$ 230 nm (Schulter), 282 nm

*Beispiel 134:*

*N-{2-[4-Amino-3-brom-5-(N-äthylcyclohexylaminomethyl)benzoyloxy]äthyl}-N'-{2-[1-(4-chlorbenzoyl)-5-methoxy-2-methyl-[1H]-indol-3-acetoxy]äthyl}piperazin*

Hergestellt aus N-{2-[4-Amino-3-brom-5-(N-äthylcyclohexylaminomethyl)benzoyloxy]äthyl}-N'-(2-hydroxyäthyl)piperazin und 1-(4-Chlorbenzoyl)-5-methoxy-2-methyl-[1H]-indol-3-essigsäureimidazolid analog Beispiel 3. Öl.

$C_{43}H_{53}BrClN_5O_6$ (851,3)

| | | | |
|---|---|---|---|
| berechnet: | C 60,67 | H 6,28 | Br 9,39 |
| | Cl 4,16 | N 8,23 | |
| gefunden: | C 60,50 | H 6,65 | Br 9,22 |
| | Cl 4,09 | N 8,10 | |

*Beispiel 135:*

*N-{3-[4-Amino-3-brom-5-(N-äthylcyclohexylaminomethyl)benzoyloxy]propyl}-N'-{2-[1-(4-*

*chlorbenzoyl)-5-methoxy-2-methyl-[1H]-indol-3-acetoxy]äthyl}piperazin*

Hergestellt aus N-{3-[4-Amino-3-brom-5-(N-äthylcyclohexylaminomethyl)benzoyloxy]propyl}-N'-(2-hydroxyäthyl)piperazin und 1-(4-Chlorbenzoyl)-5-methoxy-2-methyl-[1H]-indol-3-essigsäure mit Dicyclohexylcarbodiimid analog Beispiel 4.

Schmelzpunkt des Trihydrogenmaleinats: 120°C

*Beispiel 136:*

*N-{3-[4-Amino-3-brom-5-(N-äthylcyclohexylaminomethyl)benzoyloxy]propyl}-N'-[2-{2-[(2,6-dichlorphenyl)amino]phenylacetoxy}äthyl] piperazin*

Hergestellt aus N-{3-[4-Amino-3-brom-5-(N-äthylcyclohexylaminomethyl)benzoyloxy]propyl}-N'-(2-hydroxyäthyl)piperazin und 2-[(2,6-Dichlorphenyl)amino]phenylessigsäure mit Dicyclohexylcarbodiimid analog Beispiel 4.

Schmelzpunkt des Trihydrogenmaleinats: ab 122°C (Zers.)

*Beispiel 137:*

*N-[3-(4-Amino-3-brom-5-diäthylaminomethylbenzoyloxy)propyl]-N'-{2-[1-(4-chlorbenzoyl)-5-methoxy-2-methyl-[1H]-indol-3-acetoxy]-äthyl}piperazin*

Hergestellt aus N-[3-(4-Amino-3-brom-5-diäthylaminomethylbenzoyloxy)propyl]-N'-(2-hydroxyäthyl)piperazin und 1-(4-Chlorbenzoyl)-5-methoxy-2-methyl-[1H]-indol-3-essigsäure mit Dicyclohexylcarbodiimid analog Beispiel 4.

Schmelzpunkt des Trihydrogenmaleinats: ab 132°C (Zers.)

*Beispiel 138:*

*2-[4-Amino-3-brom-5-(N-äthylcyclohexylaminomethyl)benzoyloxy]-1-[1-(4-chlorbenzoyl)-5-methoxy-2-methyl-[1H]-indol-3-acetoxy]-n-propan*

Hergestellt aus 4-Amino-3-brom-5-(N-äthylcyclohexylaminomethyl)benzoylchlorid und 1-[1-(4-Chlorbenzoyl)-5-methoxy-2-methyl-[1H]-indol-3-acetoxy]-2-hydroxy-n-propan analog Beispiel 7, anstatt Pyridin wurde 4-Dimethylaminopyridin und anstatt Toluol wurde Methylenchlorid verwendet. Öl.

IR-Spektrum (Methylenchlorid):

| | |
|---|---|
| $NH_2$ | 3420-3450 $cm^{-1}$ |
| N-Alkyl | 2925 $cm^{-1}$ |
| $OCH_3$ | 2850 $cm^{-1}$ |
| Ester-CO | 1730 und 1720 $cm^{-1}$ |
| Amid-CO | 1680 $cm^{-1}$ |

UV-Spektrum (Äthanol): $\lambda_{max.}$ 230 nm, 290 nm

*Beispiel 139:*

*1-[4-Amino-3-brom-5-(N-äthylcyclohexylaminomethyl)benzoyloxy]-2-[(+)-6-methoxy-α-methyl-2-naphthalinacetoxy]-n-propan*

Hergestellt aus 1-[4-Amino-3-brom-5-(N-äthylcyclohexylaminomethyl)benzoyloxy]-2-hydroxy-n-propan und (+)-6-Methoxy-α-methyl-2-naphthalinessigsäureimidazolid analog Beispiel 1. Öl.

IR-Spektrum (Methylenchlorid):

| | |
|---|---|
| $NH_2$ | 3430 $cm^{-1}$ |
| N-Alkyl | 2920 $cm^{-1}$ |
| $OCH_3$ | 2850 $cm^{-1}$ |
| Ester-CO | 1730 und 1710 $cm^{-1}$ |

UV-Spektrum (Äthanol): $\lambda_{max.}$ 234 nm, 295 nm

*Beispiel 140:*

*2-[4-Amino-3-brom-5-(N-äthylcyclohexylaminomethyl)benzoyloxy]-3-[(+)-6-methoxy-α-methyl-2-naphthalinacetoxy]-n-butan*

Hergestellt aus 2-[4-Amino-3-brom-5-(N-äthylcyclohexylaminomethyl)benzoyloxy]-3-hydroxy-n-butan und (+)-6-Methoxy-α-methyl-2-naphthalinessigsäureimidazolid analog Beispiel 1. Öl.

IR-Spektrum (Methylenchlorid):

| | |
|---|---|
| $NH_2$ | 3430 $cm^{-1}$ |
| N-Alkyl | 2920 $cm^{-1}$ |
| $OCH_3$ | 2850 $cm^{-1}$ |
| Ester-CO | 1730 und 1710 $cm^{-1}$ |

UV-Spektrum (Äthanol): $\lambda_{max.}$ 234 nm, 295 nm

*Beispiel 141:*

*3-(4-Amino-3-brom-5-diäthylaminomethylbenzoyloxy)-2-[1-(4-chlorbenzoyl)-5-methoxy-2-methyl-[1H]-indol-3-acetoxy]-n-propylchlorid*

Hergestellt aus 3-(4-Amino-3-brom-5-diäthylaminomethylbenzoyloxy)-2-hydroxy-n-propylchlorid und 1-(4-Chlorbenzoyl)-5-methoxy-2-methyl-[1H]-indol-3-essigsäureimidazolid analog Beispiel 1. Öl.

IR-Spektrum (Methylenchlorid):

| | |
|---|---|
| $NH_2$ | 3440 $cm^{-1}$ |
| Ester-CO | 1730 und 1705 $cm^{-1}$ |

UV-Spektrum (Äthanol): $\lambda_{max.}$ 230 nm, 290-300 nm

*Beispiel 142:*

*2-[4-Amino-3-brom-5-(N-äthylcyclohexylaminomethyl)benzoyloxy]-3-[1-(4-chlorbenzoyl)-5-methoxy-2-methyl-[1H]-indol-3-acetoxy]-n-propylchlorid*

Hergestellt aus 4-Amino-3-brom-5-(N-äthylcyclohexylaminomethyl)benzoesäureimidazolid und 3-[1-(4-Chlorbenzoyl)-5-methoxy-2-methyl-[1H]-indol-3-acetoxy]-2-hydroxy-n-propylchlorid analog Beispiel 11. Öl.

IR-Spektrum (Methylenchlorid):

| | |
|---|---|
| Ester-CO | 1710 und 1740 $cm^{-1}$ |
| Amid-CO | 1670 $cm^{-1}$ |

UV-Spektrum (Äthanol): $\lambda_{max.}$ 230 nm, 295 nm

*Beispiel 143:*

*2-[4-Amino-3-brom-5-(N-äthylcyclohexylaminomethyl)benzoyl]-3-[1-(4-chlorbenzoyl)-5-methoxy-2-methyl-[1H]-indol-3-acetyl]-(+)-weinsäurediäthylester*

Hergestellt aus 4-Amino-3-brom-5-(N-äthyl-

cyclohexylaminomethyl)benzoylchloridhydrochlorid und 2-[1-(4-Chlorbenzoyl)-5-methoxy-2-methyl-[1H]-indol-3-acetyl]-3-hydroxy-(+)-weinsäurediäthylester in Tetrahydrofuran/Pyridin analog Beispiel 10. Schaum.

IR-Spektrum (Methylenchlorid):
Amid-CO 1680 $cm^{-1}$
Ester-CO 1720 und 1750-1760 $cm^{-1}$
O-Methyl 2850 $cm^{-1}$
N-Alkyl 2930 $cm^{-1}$
UV-Spektrum (Äthanol): $\lambda_{max.}$ 237 nm (Schulter), 305 nm

*Beispiel 144:*

*2-[4-Amino-3-brom-5-(N-äthylcyclohexylaminomethyl)benzoyloxy]-1-acetoxy-3-[1-]4-chlorbenzoyl)-5-methoxy-2-methyl-[1H]-indol-3-acetoxy]-n-propan*

Hergestellt aus 4-Amino-3-brom-5-(N-äthylcyclohexylaminomethyl)benzoylchlorid und 3-[1-(4-Chlorbenzoyl)-5-methoxy-2-methyl-[1H]-indol-3-acetoxy]-1-acetoxy-2-hydroxy-n-propan analog Beispiel 10. Öl.

IR-Spektrum (Methylenchlorid):
Ester-CO 1710 und 1745 $cm^{-1}$
Amid-CO 1675 $cm^{-1}$
UV-Spektrum (Äthanol): $\lambda_{max.}$ 230 nm, 295 nm

*Beispiel 145:*

*2-[4-Amino-3-brom-5-(N-äthylcyclohexylaminomethyl)benzoyloxy]-1-octadecanoyloxy-3-[1-(4-chlorbenzoyl)-5-methoxy-2-methyl-[1H]-indol-3-acetoxy]-n-propan*

Hergestellt aus 4-Amino-3-brom-5-(N-äthylcyclohexylaminomethyl)benzoylchlorid und 3-[1-(4-Chlorbenzoyl)-5-methoxy-2-methyl-[1H]-indol-3-acetoxy]-1-octadecanoyloxy-2-hydroxy-n-propan analog Beispiel 10. Öl.

$C_{56}H_{77}BrClN_3O_8$ (1035,6)

| | | | |
|---|---|---|---|
| berechnet: | C 64,95 | H 7,49 | Br 7,72 |
| | Cl 3,42 | N 4,06 | |
| gefunden: | C 64,70 | H 7,43 | Br 7,70 |
| | Cl 3,40 | N 3,70 | |

*Beispiel 146:*

*2-[4-Amino-3-brom-5-(N-äthylcyclohexylaminomethyl)benzoyloxy]-1-octadecanoyloxy-3-[(+)-6-methoxy-α-methyl-2-naphthalinacetoxy]-n-propan*

Hergestellt aus 4-Amino-3-brom-5-(N-äthylcyclohexylaminomethyl)benzoesäureimidazolid und 3-[(+)-6-Methoxy-α-methyl-2-naphthalinacetoxy]-1-octadecanoyloxy-2-hydroxy-n-propan analog Beispiel 11. Öl.

$C_{51}H_{75}BrN_2O_7$ (908,1)

| | | | | |
|---|---|---|---|---|
| berechnet: | C 67,46 | H 8,32 | Br 8,80 | N 3,08 |
| gefunden: | C 67,80 | H 8,36 | Br 8,63 | N 3,16 |

*Beispiel 147:*

*1,2-Bis-[4-amino-3-brom-5-(N-äthylcyclohexylaminomethyl)benzoyloxy]-3-[(+)-6-methoxy-α-methyl-2-naphthalinacetoxy]-n-propan*

Hergestellt aus 4-Amino-3-brom-5-(N-äthylcyclohexylaminomethyl)benzoesäureimidazolid und 3-[(+)-6-Methoxy-α-methyl-2-naphthalinacetoxy]-1,2-dihydroxy-n-propan analog Beispiel 11. Öl.

IR-Spektrum (Methylenchlorid):
Ester-CO 1710 und 1740 $cm^{-1}$
UV-Spektrum (Äthanol): $\lambda_{max.}$ 243 nm, 302 nm

*Beispiel 148:*

*3-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]-1-hydroxy-n-propan*

Hergestellt aus 3-[4-Amino-3-brom-5-(N-äthylcyclohexylaminomethyl)benzoyloxy]-1,2-epoxi-n-propan und 1-(4-Chlorbenzoyl)-5-methoxy-2-methyl-[1H]-indol-3-essigsäure analog Beispiel 6. Feste, amorphe Substanz.

IR-Spektrum (Methylenchlorid):
Ester-CO 1710 und 1735 $cm^{-1}$
Amid-CO 1675 $cm^{-1}$
UV-Spektrum (Äthanol): $\lambda_{max.}$ 230 nm, 297 nm

*Beispiel 149:*

*1-[4-Amino-3-brom-5-(N-äthylcyclohexylaminomethyl)benzoyloxy]-2-hydroxy-3-{2-[(2,6-dichlorphenyl)amino]phenylacetoxy}-n-propan*

Hergestellt aus 4-Amino-3-brom-5-(N-äthylcyclohexylaminomethyl)benzoesäureimidazolid und 1-{2-[(2,6-Dichlorphenyl)amino]phenylacetoxy}-2,3-dihydroxy-n-propan analog Beispiel 2.

Schmelzpunkt des Hydrochlorids: 87-96°C

*Beispiel 150:*

*1-[4-Amino-3-brom-5-(N-äthylcyclohexylaminomethyl)benzoyloxy]-2-pentanoyloxy-3-[(+)-6-methoxy-α-methyl-2-naphthalinacetoxy]-n-propan*

Hergestellt aus 1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan und Valeriansäurechlorid analog Beispiel 12. Das Hydrochlorid ist ein Öl.

$C_{38}H_{49}BrN_2O_7 \cdot HCl$ (762,2)

| | | | |
|---|---|---|---|
| berechnet: | C 59,88 | H 6,61 | Br 10,48 |
| | Cl 4,65 | N 3,68 | |
| gefunden: | C 59,60 | H 6,74 | Br 10,78 |
| | Cl 4,78 | N 3,97 | |

*Beispiel 151:*

*1-[4-Amino-3-brom-5-(N-äthylcyclohexylaminomethyl)benzoyloxy]-2-tetradecanoyloxy-3-[(+)-6-methoxy-α-methyl-2-naphthalinacetoxy]-n-propan*

Hergestellt aus 1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan und Myristinsäurechlorid analog Beispiel 12. Das Hydrochlorid ist ein Öl.

$C_{47}H_{65}BrN_2O_7$ · HCl (886,4)

| | | | |
|---|---|---|---|
| berechnet: | C 63,69 | H 7,51 | Br 9,02 |
| | Cl 4,00 | N 3,16 | |
| gefunden: | C 63,40 | H 7,78 | Br 9,13 |
| | Cl 4,04 | N 3,05 | |

*Beispiel 152:*

*1-[4-Amino-3-brom-5-(N-äthylcyclohexylaminomethyl)benzoyloxy]-2-octadecanoyloxy-3-[(+)-6-methoxy-α-methyl-2-naphthalinacetoxy]-n-propan*

Hergestellt aus 1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan und Stearinsäurechlorid analog Beispiel 12. Das Hydrochlorid ist ein Öl.

$C_{51}H_{75}BrN_2O_7$ · HCl (944,6)

| | | | |
|---|---|---|---|
| berechnet: | C 64,85 | H 8,11 | Br 8,46 |
| | Cl 3,75 | N 2,97 | |
| gefunden: | C 64,58 | H 8,04 | Br 8,45 |
| | Cl 3,74 | N 3,09 | |

*Beispiel 153:*

*1-[4-Amino-3-brom-5-(N-äthylcyclohexylaminomethyl)benzoyloxy]-2-decanoyloxy-3-[(+)-6-methoxy-α-methyl-2-naphthalinacetoxy]-n-propan*

Hergestellt aus 1-[4-Amino-3-brom-5-(N-äthylcyclohexylaminomethyl)benzoyloxy]-2-hydroxy-3-[(+)-6-methoxy-α-methyl-2-naphthalinacetoxy]-n-propan und Caprinsäurechlorid analog Beispiel 12. Das Hydrochlorid ist ein Öl.

$C_{43}H_{59}BrN_2O_7$ · HCl (832,3)

| | | | |
|---|---|---|---|
| berechnet: | C 62,05 | H 7,27 | Br 9,60 |
| | Cl 4,26 | N 3,37 | |
| gefunden: | C 61,62 | H 7,25 | Br 10,15 |
| | Cl 4,46 | N 3,74 | |

*Beispiel 154:*

*1-[1-(4-Chlorbenzoyl)-5-methoxy-2-methyl-[1H]-indol-3-acetyl]-6-(4-amino-3-brom-5-diäthylaminomethylbenzoyl)-D-mannit*

Hergestellt aus 1-[1-(4-Chlorbenzoyl)-5-methoxy-2-methyl-[1H]-indol-3-acetyl]-D-mannit und 4-Amino-3-brom-5-diäthylaminomethylbenzoylchlorid in Diochlormethan/Pyridin analog Beispiel 10. Amorphes Pulver.

IR-Spektrum (Kaliumbromid):
Ester-CO 1710 $cm^{-1}$
Amid-CO 1680 $cm^{-1}$
UV-Spektrum (Äthanol): $\lambda_{max.}$ 230 nm (Schulter), 285 nm

*Beispiel 155:*

*1-{2-[(2,6-Dichlorphenyl)amino]phenylacetyl}-6-[4-amino-3-brom-5-(N-äthylcyclohexylaminomethyl)-benzoyl]-D-mannit*

Hergestellt aus 1-[4-Amino-3-brom-5-(N-äthylcyclohexylaminomethyl)benzoyl]-D-mannit und 2-[(2,6-Dichlorphenyl)amino]phenylessigsäure analog Beispiel 4. Amorphes Pulver.

Schmelzpunkt des Hydrochlorids: 170-185°C (Aufschäumen)

*Beispiel 156:*

*1-[4-Amino-3-brom-5-(N-äthylcyclohexylaminomethyl)benzamido]-2-[1-(4-chlorbenzoyl)-5-methoxy-2-methyl-[1H]-indol-3-acetoxy]äthan*

Hergestellt aus 1-[4-Amino-3-brom-5-(N-äthylcyclohexylaminomethyl)benzamido]-2-hydroxyäthan und 1-(4-Chlorbenzoyl)-5-methoxy-2-methyl-[1H]-indol-3-essigsäureimidazolid analog Beispiel 1.

Schmelzpunkt des Hydrochlorids: ab 125°C (Zers.)

*Beispiel 157:*

*1-[4-Amino-3-brom-5-(N-äthylcyclohexylaminomethyl)benzamido]-3-[1-(4-chlorbenzoyl)-5-methoxy-2-methyl-[1H]-indol-3-acetoxy]-n-propan*

Hergestellt aus 1-[4-Amino-3-brom-5-(N-äthylcyclohexylaminomethyl)benzamido]-3-hydroxy-n-propan und 1-(4-Chlorbenzoyl)-5-methoxy-2-methyl-[1H]-indol-3-essigsäureimidazolid analog Beispiel 13. Öl.

IR-Spektrum (Methylenchlorid):
Ester-CO 1730 $cm^{-1}$
Amid-CO 1650 und 1675 $cm^{-1}$
UV-Spektrum (Äthanol): $\lambda_{max.}$ 275 nm (Schulter), ~325 nm

*Beispiel 158:*

*1-[4-Amino-3-brom-5-(N-äthylcyclohexylaminomethyl)benzamido]-4-[1-(4-chlorbenzoyl)-5-methoxy-2-methyl-[1H]-indol-3-acetoxy]-n-butan*

Hergestellt aus 1-[4-Amino-3-brom-5-(N-äthylcyclohexylaminomethyl)benzamido]-4-hydroxy-n-butan und 1-(4-Chlorbenzoyl)-5-methoxy-2-methyl-[1H]-indol-3-essigsäureimidazolid analog Beispiel 13. Öl. Das Hydrochlorid ist eine feste, amorphe Substanz.

$C_{39}H_{46}BrClN_4O_5$ · HCl (802,6)

| | | | |
|---|---|---|---|
| berechnet: | C 58,36 | H 5,90 | Br 9,96 |
| | Cl 8,83 | N 6,98 | |
| gefunden: | C 58,10 | H 6,02 | Br 9,92 |
| | Cl 8,81 | N 6,84 | |

*Beispiel 159:*

*1-[4-Amino-3-brom-5-(N-äthylcyclohexylaminomethyl)benzamido]-5-[1-(4-chlorbenzoyl)-5-methoxy-2-methyl-[1H]-indol-3-acetoxy]-n-pentan*

Hergestellt aus 1-[4-Amino-3-brom-5-(N-äthylcyclohexylaminomethyl)benzamido]-5-hydroxy-n-pentan und 1-(4-Chlorbenzoyl)-5-methoxy-2-methyl-[1H]-indol-3-essigsäure-imidazolid analog Beispiel 13. Öl.

Das Hydrochlorid ist eine feste, amorphe Substanz.

$C_{40}H_{48}BrClN_4O_5 \cdot HCl$ (816,7)

| | | | |
|---|---|---|---|
| berechnet: | C 58,83 | H 6,05 | Br 9,79 |
| | Cl 8,68 | N 6,86 | |
| gefunden: | C 58,60 | H 6,18 | Br 9,62 |
| | Cl 8,53 | N 6,85 | |

*Beispiel 160:*

*1-[4-Amino-3-brom-5-(N-äthylcyclohexyl-aminomethyl)benzamido]-6-[1-(4-chlorbenzoyl)-5-methoxy-2-methyl-[1H]-indol-3-acetoxy]-n-hexan*

Hergestellt aus 1-[4-Amino-3-brom-5-(N-äthylcyclohexylaminomethyl)benzamido]-6-hydroxy-n-hexan und 1-(4-(Chlorbenzoyl)-5-methoxy-2-methyl-[1H]-indol-3-essigsäure-imidazolid analog Beispiel 13. Öl.

Das Hydrochlorid ist eine feste, amorphe Substanz.

$C_{41}H_{50}BrClN_4O_5 \cdot HCl$ (830,7)

| | | | |
|---|---|---|---|
| berechnet: | C 59,28 | H 6,19 | Br 9,62 |
| | Cl 8,53 | N 6,74 | |
| gefunden: | C 59,00 | H 6,32 | Br 9,68 |
| | Cl 8,58 | N 6,50 | |

*Beispiel 161:*

*1-[4-Amino-3-brom-5-(N-äthylcyclohexyl-aminomethyl)benzamido]-2-[(+)-6-methoxy-α-methyl-2-naphthalinacetoxy]äthan*

Hergestellt aus 1-[4-Amino-3-brom-5-(N-äthylcyclohexylaminomethyl)benzamido]-2-hydroxyäthan und (+)-6-Methoxy-α-methyl-2-naphthalinessigsäureimidazolid analog Beispiel 13. Das Hydrogenmaleinat ist ein amorphes Pulver.

$C_{32}N_{40}BrN_3O_4 \cdot C_4H_4O_4$ (726,7)

| | | | | |
|---|---|---|---|---|
| berechnet: | C 59,30 | H 6,16 | Br 10,86 | N 5,64 |
| gefunden: | C 59,50 | H 6,10 | Br 11,00 | N 5,78 |

*Beispiel 162:*

*1-[4-Amino-3-brom-5-(N-äthylcyclohexyl-aminomethyl)benzamido]-3-[(+)-6-methoxy-α-methyl-2-naphthalinacetoxy]-n-propan*

Hergestellt aus 1-[4-Amino-3-brom-5-(N-äthylcyclohexylaminomethyl)benzamido]-3-hydroxy-n-propan und (+)-6-Methoxy-α-methyl-2-naphthalinessigsäureimidazolid analog Beispiel 13.

Schmelzpunkt des Hydrochlorids: 70-90°C

*Beispiel 163:*

*1-[4-Amino-3-brom-5-(N-äthylcyclohexyl-aminomethyl)benzamido]-5-[(+)-6-methoxy-α-methyl-2-naphthalinacetoxy]-n-pentan*

Hergestellt aus 1-[4-Amino-3-brom-5-N-äthylcyclohexylaminomethyl)benzamido]-5-hydroxy-n-pentan und (+)-6-Methoxy-α-methyl-2-naphthalinessigsäureimidazolid analog Beispiel 13. Öl.

IR-Spektrum (Methylenchlorid):
Ester-CO 1725 $cm^{-1}$
Amid-CO 1650 $cm^{-1}$
UV-Spektrum (Äthanol): $\lambda_{max.}$ 235 nm, 280 nm

*Beispiel 164:*

*1-[4-Amino-3-brom-5-(N-äthylcyclohexyl-aminomethyl)benzamido]-6-[(+)-6-methoxy-α-methyl-2-naphthalinacetoxy]-n-hexan*

Hergestellt aus 1-[4-Amino-3-brom-5-äthylcyclohexylaminomethyl)benzamido]-6-hydroxy-n-hexan und (+)-6-Methoxy-α-methyl-2-naphthalinessigsäureimidazolid analog Beispiel 13. Öl.

Das Hydrochlorid ist eine feste, amorphe Substanz:

$C_{36}H_{48}BrN_3O_4 \cdot HCl$ (703,2)

| | | | |
|---|---|---|---|
| berechnet: | C 61,49 | H 7,02 | Br 11,36 |
| | Cl 5,04 | N 5,98 | |
| gefunden: | C 61,70 | H 7,10 | Br 11,22 |
| | Cl 4,98 | N 5,59 | |

*Beispiel 165:*

*1-[4-Amino-3-brom-5-(N-äthylcyclohexyl-aminomethyl)benzoyloxy]-2-[1-(4-chlorbenzoyl)-5-methoxy-2-methyl-[1H]-indol-3-acetamido]äthan*

Hergestellt aus 1-[1-(4-Chlorbenzoyl)-5-methoxy-2-methyl-[1H]-indol-3-acetamido]-2-hydroxyäthan und 4-Amino-3-brom-5-(N-äthylcyclohexylaminomethyl)benzoylchlorid analog Beispiel 5.

Schmelzpunkt des Hydrochlorids: ab 140°C (Zers.)

*Beispiel 166:*

*1-[4-Amino-3-brom-5-(N-äthylcyclohexyl-aminomethyl)benzoyloxy]-3-[1-(4-chlorbenzoyl)-5-methoxy-2-methyl-[1H]-indol-3-acetamido]-n-propan*

Hergestellt aus 1-[1-(4-Chlorbenzoyl)-5-methoxy-2-methyl-[1H]-indol-3-acetamido]-3-hydroxy-n-propan und 4-Amino-3-brom-5-(N-äthylcyclohexylaminomethyl)benzoylchlorid analog Beispiel 10. Öl.

IR-Spektrum (Methylenchlorid):
Ester-CO 1700 $cm^{-1}$
Amid-CO 1665 und 1680 $cm^{-1}$
UV-Spektrum (Äthanol): $\lambda_{max.}$ 230 nm (Schulter), 290 nm

*Beispiel 167:*

*1-[4-Amino-3-brom-5-(N-äthylcyclohexyl-aminomethyl)benzoyloxy]-4-[1-(4-chlorbenzoyl)-5-methoxy-2-methyl-[1H]-indol-3-acetamido]-n-butan*

Hergestellt aus 1-[1-(4-Chlorbenzoyl)-5-methoxy-2-methyl-[1H]-indol-3-acetamido]-4-hydroxy-n-butan und 4-Amino-3-brom-5-(N-äthylcyclohexylaminomethyl)benzoylchlorid analog Beispiel 10. Öl.

IR-Spektrum (Methylenchlorid):
Ester-CO 1710 $cm^{-1}$
Amid-CO 1660 und 1680 $cm^{-1}$
UV-Spektrum (Äthanol): $\lambda_{max.}$ 230 nm, 285 nm
Das Hydrochlorid ist eine feste, amorphe Substanz.

*Beispiel 168:*

*1-[4-Amino-3-brom-5-(N-äthylcyclohexylaminomethyl)benzoyloxy]-5-[1-(4-chlorbenzoyl)-5-methoxy-2-methyl-[1H]-indol-3-acetamido]-n-pentan*

Hergestellt aus 1-[1-(4-Chlorbenzoyl)-5-methoxy-2-methyl-[1H]-indol-3-acetamido]-5-hydroxy-n-pentan und 4-Amino-3-brom-5-(N-äthylcyclohexylaminomethyl)benzoylchlorid analog Beispiel 10. Öl.
IR-Spektrum (Methylenchlorid):
Ester-CO 1710 $cm^{-1}$
Amid-CO 1660 und 1680 $cm^{-1}$
UV-Spektrum (Äthanol): $\lambda_{max.}$ 230 nm, 290 nm

*Beispiel 169:*

*1-[4-Amino-3-brom-5-(N-äthylcyclohexylaminomethyl)benzoyloxy]-6-[1-(4-chlorbenzoyl)-5-methoxy-2-methyl-[1H]-indol-3-acetamido]-n-hexan*

Hergestellt aus 1-[1-(4-Chlorbenzoyl)-5-methoxy-2-methyl-[1H]-indol-3-acetamido]-6-hydroxy-n-hexan und 4-Amino-3-brom-5-(N-äthylcyclohexylaminomethyl)benzoylchlorid analog Beispiel 10. Öl.
IR-Spektrum (Methylenchlorid):
Ester-CO 1710 $cm^{-1}$
Amid-CO 1665 und 1680 $cm^{-1}$
UV-Spektrum (Äthanol): $\lambda_{max.}$ 232 nm, 285 nm

*Beispiel 170:*

*1-[4-Amino-3-brom-5-(N-äthylcyclohexylaminomethyl)benzoyloxy]-3-[(+)-6-methoxy-α-methylphenylacetamido]-n-propan*

Hergestellt aus 1-[(+)-6-Methoxy-α-methylphenylacetamido]-3-hydroxy-n-propan und 4-Amino-3-brom-5-(N-äthylcyclohexylaminomethyl)benzoylchlorid analog Beispiel 10. Öl.
IR-Spektrum (Methylenchlorid):
Ester-CO 1710 $cm^{-1}$
Amid-CO 1640 $cm^{-1}$
UV-Spektrum (Äthanol): $\lambda_{max.}$ 234 nm, 285 nm

*Beispiel 171:*

*1-[4-Amino-3-brom-5-(N-äthylcyclohexylaminomethyl)benzoyloxy]-5-[(+)-6-methoxy-α-methylphenylacetamido]-n-pentan*

Hergestellt aus 1-[(+)-6-Methoxy-α-methylphenylacetamido]-5-hydroxy-n-pentan und 4-Amino-3-brom-5-(N-äthylcyclohexylaminomethyl)benzoylchlorid analog Beispiel 10. Öl.
IR-Spektrum (Methylenchlorid):
Ester-CO 1700 $cm^{-1}$
Amid-CO 1670 $cm^{-1}$
UV-Spektrum (Äthanol): $\lambda_{max.}$ 235 nm, 295 nm

*Beispiel 172:*

*1-[4-Amino-3-brom-5-(N-äthylcyclohexylaminomethyl)benzoyloxy]-6-[(+)-6-methoxy-α-methylphenylacetamido]-n-hexan*

Hergestellt aus 1-[(+)-6-Methoxy-α-methylphenylacetamido]-6-hydroxy-n-hexan und 4-Amino-3-brom-5-(N-äthylcyclohexylaminomethyl)benzoylchlorid analog Beispiel 10. Öl.
IR-Spektrum (Methylenchlorid):
Ester-CO 1705 $cm^{-1}$
Amid-CO 1645 $cm^{-1}$
UV-Spektrum (Äthanol): $\lambda_{max.}$ 235 nm, 280 nm

*Beispiel 173:*

*trans-4-[4-Amino-3-brom-5-(N-äthylcyclohexylaminomethyl)benzamido]-1-[1-(4-chlorbenzoyl)-5-methoxy-2-methyl-[1H]-indol-3-acetoxy]cyclohexan*

Hergestellt aus trans-4-[4-Amino-3-brom-5-(N-äthylcyclohexylaminomethylbenzamido]-cyclohexanol und 1-(4-Chlorbenzoyl)-5-methoxy-2-methyl-[1H]-indol-3-essigsäureimidazolid analog Beispiel 1.
Schmelzpunkt: 160-163°C (Zers.)

*Beispiel 174:*

*1-[4-Amino-3-brom-5-(N-äthylcyclohexylaminomethyl)benzamido]-3-[1-(4-chlorbenzoyl)-5-methoxy-2-methyl-[1H]-indol-3-acetamido]-n-propan*

Hergestellt aus 1-[1-(4-Chlorbenzoyl)-5-methoxy-2-methyl-[1H]-indol-3-acetamido]-3-amino-n-propan und 4-Amino-3-brom-5-(N-äthylcyclohexylaminomethyl)benzoylchlorid in Gegenwart von Triäthylamin analog Beispiel 7.
Schmelzpunkt: 161-167°C

*Beispiel 175:*

*1-[4-Amino-3-brom-5-(N-äthylcyclohexylaminomethyl)benzamido]-2-[(+)-6-methoxy-α-methyl-2-naphthalinacetamido]äthan*

Hergestellt aus 1-[(+)-6-Methoxy-α-methyl-2-naphthalinacetamido]-2-aminoäthan und 4-Amino-3-brom-5-(N-äthylcyclohexylaminomethyl)benzoylchlorid in Gegenwart von Triäthylamin analog Beispiel 7.
Schmelzpunkt: 136-137°C

*Beispiel 176:*

*1-[4-Amino-3-brom-5-(N-äthylcyclohexylaminomethyl)benzamido]-3-[(+)-6-methoxy-α-methyl-2-naphthalinacetamido]-n-propan*

Hergestellt aus 1-[(+)-6-Methoxy-α-methyl-2-naphthalinacetamido]-3-amino-n-propan und 4-Amino-3-brom-5-(N-äthylcyclohexylaminomethyl)benzoylchlorid in Gegenwart von Triäthylamin analog Beispiel 7.
Schmelzpunkt des Hydrochlorids: ab 126°C (Zers.)

*Beispiel 177:*

*1-[4-Amino-3-brom-5-(N-äthylcyclohexyl-*

*aminomethyl)benzamido]-4-[(+)-6-methoxy-α-methyl-2-naphthalinacetamido]-n-butan*

Hergestellt aus 1-[(+)-6-Methoxy-α-methyl-2-naphthalinacetamido]-4-amino-n-butan und 4-Amino-3-brom-5-(N-äthylcyclohexylamino-methyl)benzoylchlorid in Gegenwart von Triäthylamin analog Beispiel 7.

Schmelzpunkt: 130-131,5°C

*Beispiel 178:*

*1-[4-Amino-3-brom-5-(N-äthylcyclohexyl-aminomethyl)benzamido]-5-[(+)-6-methoxy-α-methyl-2-naphthalinacetamido]-n-pentan*

Hergestellt aus 1-[(+)-6-Methoxy-α-methyl-2-naphthalinacetamido]-5-amino-n-pentan und 4-Amino-3-brom-5-(N-äthylcyclohexylamino-methyl)benzoylchlorid in Gegenwart von Triäthylamin analog Beispiel 7.

Schmelzpunkt: ab 72°C (sintern)

*Beispiel 179:*

*1-[4-Amino-3-brom-5-(N-äthylcyclohexyl-aminomethyl)benzamido]-6-[(+)-6-methoxy-α-methyl-2-naphthalinacetamido]-n-hexan*

Hergestellt aus 1-[(+)-6-Methoxy-α-methyl-2-naphthalinacetamido]-6-amino-n-hexan und 4-Amino-3-brom-5-(N-äthylcyclohexylamino-methyl)benzoylchlorid in Gegenwart von Triäthylamin analog Beispiel 7.

Schmelzpunkt: 149-150°C

*Beispiel 180:*

*N-[4-Amino-3-brom-5-(N-äthylcyclohexyl-aminomethyl)benzoyl]-O-[1-(4-chlorbenzoyl)-5-methoxy-2-methyl-[1H]-indol-3-acetyl]-L-serinmethylester*

Hergestellt aus N-[4-Amino-3-brom-5-(N-äthylcyclohexylaminomethyl)benzoyl]-L-serinmethylester und 1-(4-Chlorbenzoyl)-5-methoxy-2-methyl-[1H]-indol-3-essigsäureimidazolid analog Beispiel 1. Schaum.

IR-Spektrum (Methylenchlorid):

| | |
|---|---|
| $NH_2$ | 3435 $cm^{-1}$ |
| N-Alkyl | 2920 $cm^{-1}$ |
| $OCH_3$ | 2850 $cm^{-1}$ |
| Ester-CO | 1745 $cm^{-1}$ |
| Amid-CO | 1675 und 1655 $cm^{-1}$ |

UV-Spektrum (Methanol): $\lambda_{max.}$ 230 nm, 290 nm

*Beispiel 181:*

*O-[4-Amino-3-brom-5-(N-äthylcyclohexyl-aminomethyl)benzoyl]-N-[1-(4-chlorbenzoyl)-5-methoxy-2-methyl-[1H]-indol-3-acetyl]-L-serinmethylester*

Hergestellt aus N-[1-(4-Chlorbenzoyl)-5-methoxy-2-methyl-[1H]-indol-3-acetyl]-L-serinmethylester und 4-Amino-3-brom-5-(N-äthylcyclohexylaminomethyl)benzoylchlorid analog Beispiel 7, anstatt Triäthylamin und 4-Dimethylaminopyridin wurde Pyridin verwendet. Öl.

IR-Spektrum (Methylenchlorid):

| | |
|---|---|
| $NH_2$ | 3410 $cm^{-1}$ |
| N-Alkyl | 2910 $cm^{-1}$ |
| $OCH_3$ | 2840 $cm^{-1}$ |
| Ester-CO | 1750 und 1700 $cm^{-1}$ |
| Amid-CO | 1675 $cm^{-1}$ |

UV-Spektrum (Methanol): $\lambda_{max.}$ 230 nm, 295 nm

*Beispiel 182:*

*N-[4-Amino-3-brom-5-(N-äthylcyclohexyl-aminomethyl)benzoyl]-O-[(+)-6-methoxy-α-methyl-2-naphthalinacetyl]-L-serinmethylester*

Hergestellt aus N-[4-Amino-3-brom-5-(N-äthylcyclohexylaminomethyl)benzoyl]-L-serinmethylester und (+)-6-Methoxy-α-methyl-2-naphthalinessigsäureimidazolid analog Beispiel 1. Schaum.

IR-Spektrum (Methylenchlorid):

| | |
|---|---|
| $NH_2$ | 3440 $cm^{-1}$ |
| N-Alkyl | 2920 $cm^{-1}$ |
| $OCH_3$ | 2850 $cm^{-1}$ |
| Ester-CO | 1730 $cm^{-1}$ |
| Amid-CO | 1660 $cm^{-1}$ |

UV-Spektrum (Äthanol): $\lambda_{max.}$ 234 nm und 295 nm

*Beispiel 183:*

*1-[4-Amino-3-brom-5-(N-methylbenzylamino-methyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-methylbenzylamino-methyl)benzoyloxy]-5-hydroxy-n-pentan analog Beispiel 1. Öl.

IR-Spektrum (Methylenchlorid):

| | |
|---|---|
| $NH_2$ | 3450 $cm^{-1}$ |
| Ester-CO | 1705 $cm^{-1}$, 1725 $cm^{-1}$ |

UV-Spektrum (Äthanol): $\lambda_{max.}$ 232 nm und 292 nm

*Beispiel 184:*

*1-[4-Amino-3-brom-5-(N-methylcyclohexyl-aminomethyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-methylcyclohexylamino-methyl)benzoyloxy]-5-hydroxy-n-pentan analog Beispiel 1.

Schmelzpunkt des Hydrochlorids: 107-111°C

*Beispiel 185:*

*1-(4-Amino-3-brom-5-cyclohexylaminomethyl-benzoyloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-cyclohexylaminomethyl-benzoyloxy)-5-hydroxy-n-pentan analog Beispiel 1. Öl.

IR-Spektrum (Methylenchlorid):

| | |
|---|---|
| $NH_2$ | 3440 $cm^{-1}$ |
| Ester-CO | 1700 $cm^{-1}$, 1730 $cm^{-1}$ |

UV-Spektrum (Äthanol): $\lambda_{max.}$ 232 nm und 290 nm

*Beispiel 186:*

*1-(4-Amino-3-brom-5-isopropylaminomethyl-benzoyloxy)-5-[2-fluor-α-methyl(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-isopropylaminomethyl-benzoyloxy)-5-hydroxy-n-pentan analog Beispiel 1. Öl.

IR-Spektrum (Methylenchlorid):
$NH_2$ 3440 $cm^{-1}$
Ester-CO 1700 $cm^{-1}$, 1725 $cm^{-1}$
UV-Spektrum (Äthanol): $\lambda_{max.}$ 234 nm und 292 nm

*Beispiel 187:*

*1-(4-Amino-3-brom-5-tert.-butylaminomethyl-benzoyloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-tert.-butylaminomethyl-benzoyloxy)-5-hydroxy-n-pentan analog Beispiel 1. Öl.

IR-Spektrum (Methylenchlorid):
$NH_2$ 3450 $cm^{-1}$
Ester-CO 1710 $cm^{-1}$, 1730 $cm^{-1}$
UV-Spektrum (Äthanol): $\lambda_{max.}$ 235 nm und 295 nm

*Beispiel 188:*

*1-(4-Amino-3-brom-5-dimethylaminomethyl-benzoyloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-dimethylaminomethylbenzoyl-oxy)-5-hydroxy-n-pentan analog Beispiel 1.

Schmelzpunkt des Hydrochlorids: 105°C (sintern ab 75°C)

*Beispiel 189:*

*1-[4-Amino-3-brom-5-(N-methylpiperazino-methyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-methylpiperazinomethyl)-benzoyloxy]-5-hydroxy-n-pentan analog Beispiel 1. Öl.

IR-Spektrum (Methylenchlorid):
$NH_2$ 3440 $cm^{-1}$
Ester-CO 1700 $cm^{-1}$, 1730 $cm^{-1}$
UV-Spektrum (Äthanol): $\lambda_{max.}$ 232 nm und 286 nm

*Beispiel 190:*

*1-(4-Amino-3-brom-5-morpholinomethyl-benzoyloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-morpholinomethylbenzoyl-oxy)-5-hydroxy-n-pentan analog Beispiel 1. Öl.

IR-Spektrum (Methylenchlorid):
$NH_2$ 3440 $cm^{-1}$
Ester-CO 1700 $cm^{-1}$, 1725 $cm^{-1}$
UV-Spektrum (Äthanol): $\lambda_{max.}$ 232 nm und 290 nm

*Beispiel 191:*

*1-(4-Amino-3-brom-5-hexamethylenimino-methylbenzoyloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-hexamethyleniminomethyl-benzoyloxy)-5-hydroxy-n-pentan analog Beispiel 1. Öl.

IR-Spektrum (Methylenchlorid):
$NH_2$ 3440 $cm^{-1}$
Ester-CO 1700 $cm^{-1}$, 1730 $cm^{-1}$
UV-Spektrum (Äthanol): $\lambda_{max.}$ 232 nm und 290 nm

*Beispiel 192:*

*1-(4-Amino-3-brom-5-piperidinomethylbenzo-yloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-piperidinomethylbenzoyloxy)-5-hydroxy-n-pentan analog Beispiel 1. Öl.

IR-Spektrum (Methylenchlorid):
$NH_2$ 3450 $cm^{-1}$
Ester-CO 1705 $cm^{-1}$, 1730 $cm^{-1}$
UV-Spektrum (Äthanol): $\lambda_{max.}$ 235 nm und 288 nm

*Beispiel 193:*

*1-(4-Amino-3-brom-5-pyrrolidinomethylbenzo-yloxy)-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-(4-Amino-3-brom-5-pyrrolidinomethylbenzoyl-oxy)-5-hydroxy-n-pentan analog Beispiel 1. Öl.

IR-Spektrum (Methylenchlorid):
$NH_2$ 3450 $cm^{-1}$
Ester-CO 1705 $cm^{-1}$, 1730 $cm^{-1}$
UV-Spektrum (Äthanol): $\lambda_{max.}$ 232 nm und 290 nm

*Beispiel 194:*

*1-[4-Amino-3-brom-5-(N-n-propylcyclohexyl-aminomethyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-n-propylcyclohexylamino-methyl)benzoyloxy]-5-hydroxy-n-pentan analog Beispiel 1. Öl.

IR-Spektrum (Methylenchlorid):
$NH_2$ 3450 $cm^{-1}$
Ester-CO 1700 $cm^{-1}$, 1725 $cm^{-1}$

UV-Spektrum (Äthanol): $\lambda_{max.}$ 233 nm und 290 nm

*Beispiel 195:*

*1-[4-Amino-3-brom-5-(N-äthylcycloheptylaminomethyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-äthylcycloheptylaminomethyl)benzoyloxy]-5-hydroxy-n-pentan analog Beispiel 1. Öl.

IR-Spektrum (Methylenchlorid):
$NH_2$ 3450 $cm^{-1}$
Ester-CO 1705 $cm^{-1}$, 1730 $cm^{-1}$
UV-Spektrum (Äthanol): $\lambda_{max.}$ 230 nm und 285 nm

*Beispiel 196:*

*1-[4-Amino-3-brom-5-(N-äthylcyclopentylaminomethyl)benzoyloxy]-5-[2-fluor-α-methyl-(1,1'-biphenyl)-4-acetoxy]-n-pentan*

Hergestellt aus 2-Fluor-α-methyl-(1,1'-biphenyl)-4-essigsäureimidazolid und 1-[4-Amino-3-brom-5-(N-äthylcyclopentylaminomethyl)benzoyloxy]-5-hydroxy-n-pentan analog Beispiel 1. Öl.

IR-Spektrum (Methylenchlorid):
$NH_2$ 3450 $cm^{-1}$
Ester-CO 1700 $cm^{-1}$, 1730 $cm^{-1}$
UV-Spektrum (Äthanol): $\lambda_{max.}$ 232 nm und 290 nm

*Beispiel 197:*

*1-(4-Amino-3-brom-5-diäthylaminomethylbenzoyloxy)-5-[2-fluor-α-methyl-(1,1'-biphenyl)-4-acetoxy]-n-pentan*

Hergestellt aus 1-(4-Amino-3-brom-5-diäthylaminomethylbenzoyloxy)-5-hydroxy-n-pentan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2940 $cm^{-1}$
Ester-CO 1705 und 1735 $cm^{-1}$
UV-Spektrum (Äthanol): $\lambda_{max.}$ 235 nm, 280 nm

*Beispiel 198:*

*1-(4-Amino-3-brom-5-diäthylaminomethylbenzoyloxy)-2-[2-fluor-α-methyl-(1,1'-biphenyl)-4-acetoxy]äthan*

Hergestellt aus 1-(4-Amino-3-brom-5-diäthylaminomethylbenzoyloxy)-2-hydroxyäthan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2940 $cm^{-1}$
Ester-CO 1705 und 1735 $cm^{-1}$
UV-Spektrum (Äthanol): $\lambda_{max.}$ 235 nm, 280 nm

*Beispiel 199:*

*1-(4-Amino-3-brom-5-diäthylaminomethylbenzoyloxy)-3-[2-fluor-α-methyl-(1,1'-biphenyl)-4-acetoxy]-n-propan*

Hergestellt aus 1-(4-Amino-3-brom-5-diäthylaminomethylbenzoyloxy)-3-hydroxy-n-propan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2930 $cm^{-1}$
Ester-CO 1700 und 1730 $cm^{-1}$
UV-Spektrum (Äthanol): $\lambda_{max.}$ 235 nm, 280 nm

*Beispiel 200:*

*1-(4-Amino-3-brom-5-diäthylaminomethylbenzoyloxy)-4-[2-fluor-α-methyl-(1,1'-biphenyl)-4-acetoxy]-n-butan*

Hergestellt aus 1-(4-Amino-3-brom-5-diäthylaminomethylbenzoyloxy)-4-hydroxy-n-butan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2940 $cm^{-1}$
Ester-CO 1700 und 1730 $cm^{-1}$
UV-Spektrum (Äthanol): $\lambda_{max.}$ 235 nm, 280 nm

*Beispiel 201:*

*1-(4-Amino-3-brom-5-diäthylaminomethylbenzoyloxy)-6-[2-fluor-α-methyl-(1,1'-biphenyl)-4-acetoxy]-n-hexan*

Hergestellt aus 1-(4-Amino-3-brom-5-diäthylaminomethylbenzoyloxy)-6-hydroxy-n-hexan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2940 $cm^{-1}$
Ester-CO 1700 und 1730 $cm^{-1}$
UV-Spektrum (Äthanol): $\lambda_{max.}$ 235 nm, 280 nm

*Beispiel 202:*

*1-[4-Amino-3-brom-5-(N-äthylcyclohexylaminomethyl)benzoyloxy]-3-[2-fluor-α-methyl-(1,1'-biphenyl)-4-acetoxy]-n-propan*

Hergestellt aus 1-[4-Amino-3-brom-5-(N-äthylcyclohexylaminomethyl)benzoyloxy]-3-hydroxy-n-propan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2930 $cm^{-1}$
Ester-CO 1705 und 1730 $cm^{-1}$
UV-Spektrum (Äthanol): $\lambda_{max.}$ 235 nm, 280 nm

*Beispiel 203:*

*1-[4-Amino-3-brom-5-(N-äthylcyclohexylaminomethyl)benzoyloxy]-4-[2-fluor-α-methyl-(1,1'-biphenyl)-4-acetoxy]-n-butan*

Hergestellt aus 1-[4-Amino-3-brom-5-(N-äthylcyclohexylaminomethyl)benzoyloxy]-4-hydroxy-n-butan und 2-Fluor-α-methyl-(1,1'-biphenyl)-4-essigsäure analog Beispiel 4. Öl.

IR-Spektrum (Methylenchlorid):

$NH_2$ 3460 $cm^{-1}$
N-Alkyl 2930 $cm^{-1}$
Ester-CO 1705 und 1730 $cm^{-1}$
UV-Spektrum (Äthanol): $\lambda_{max.}$ 235 nm, 280 nm

*Beispiel 204:*

*1-[4-Amino-3-brom-5-(N-äthylcyclohexylaminomethyl)benzoyloxy]-5-[2-fluor-α-methyl-(1,1'-biphenyl)-4-acetoxy]-n-pentan*

Hergestellt aus 1-[4-Amino-3-brom-5-(N-äthylcyclohexylaminomethyl)benzoyloxy]-5-hydroxy-n-pentan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2930 $cm^{-1}$
Ester-CO 1700 und 1730 $cm^{-1}$
UV-Spektrum (Äthanol): $\lambda_{max.}$ 235 nm, 280 nm

*Beispiel 205:*

*1-[4-Amino-3-brom-5-(N-äthylcyclohexylaminomethyl)benzoyloxy]-6-[2-fluor-α-methyl-(1,1'-biphenyl)-4-acetoxy]-n-hexan*

Hergestellt aus 1-[4-Amino-3-brom-5-(N-äthylcyclohexylaminomethyl)benzoyloxy]-6-hydroxy-n-hexan und 2-Fluor-α-methyl-(1,1'-biphenyl)-4-essigsäure analog Beispiel 4. Öl.

IR-Spektrum (Methylenchlorid):
$NH_2$ 3440 $cm^{-1}$
N-Alkyl 2930 $cm^{-1}$
Ester-CO 1700 und 1730 $cm^{-1}$
UV-Spektrum (Äthanol): $\lambda_{max.}$ 235 nm, 280 nm

*Beispiel 206:*

*1-[4-Amino-3-brom-5-(N-äthylcyclohexylaminomethyl)benzamido]-4-[2-fluor-α-methyl-(1,1'-biphenyl)-4-acetoxy]-n-butan*

Hergestellt aus 1-[4-Amino-3-brom-5-(N-äthylcyclohexylaminomethyl)benzamido]-4-hydroxy-n-butan und 2-Fluor-α-methyl-(1,1'-biphenyl)-4-essigsäure analog einem der Beispiele 3 oder 4. Farbloser Schaum.

IR-Spektrum (Methylenchlorid):
$NH_2$ 3510 $cm^{-1}$
Ester-CO 1740 $cm^{-1}$
Amid-CO 1670 $cm^{-1}$

*Beispiel 207:*

*N-[2-(4-Amino-3-brom-5-diäthylaminomethylbenzoyloxy)äthyl]-N'-{3-[2-fluor-α-methyl-(1,1'-biphenyl)-4-acetoxy]propyl}piperazin*

Hergestellt aus N-{3-[2-Fluor-α-methyl-(1,1'-biphenyl)-4-acetoxy]propyl}-N'-(2-hydroxyäthyl)piperazin und 4-Amino-3-brom-5-diäthylaminomethylbenzoesäure analog Beispiel 4.

Schmelzpunkt des Trishydrogenmaleinats: 163-166°C

*Beispiel 208:*

*N-[3-(4-Amino-3-brom-5-diäthylaminomethylbenzoyloxy)propyl]-N'-{2-[2-fluor-α-methyl-(1,1'-biphenyl)-4-acetoxy]äthyl}piperazin*

Hergestellt aus N-[3-(4-Amino-3-brom-5-diäthylaminomethylbenzoyloxy)propyl]-N'-(2-hydroxyäthyl)piperazin und 2-Fluor-α-methyl-(1,1'-biphenyl)-4-essigsäure analog Beispiel 4.

Schmelzpunkt des Trishydrogenmaleinats: 144-147°C

*Beispiel 209:*

*N-{3-[4-Amino-3-brom-5-(N-äthylcyclohexylaminomethyl)benzoyloxy]propyl}-N'-{2-[2-fluor-α-methyl-(1,1'-biphenyl)-4-acetoxy]-äthyl}piperazin*

Hergestellt aus N-{3-[4-Amino-3-brom-5-(N-äthylcyclohexylaminomethyl)benzoyloxy]propyl}-N'-(2-hydroxyäthyl)piperazin und 2-Fluor-α-methyl-(1,1'-biphenyl)-4-essigsäure analog Beispiel 4.

Schmelzpunkt des Trishydrogenmaleinats: 132-135°C

*Beispiel 210:*

*N-{2-[4-Amino-3-brom-5-(N-äthylcyclohexylaminomethyl)benzoyloxy]äthyl}-N'-{3-[2-fluor-α-methyl-(1,1'-biphenyl)-4-acetoxy]propyl}-piperazin*

Hergestellt aus N-{3-[2-Fluor-α-methyl-(1,1'-biphenyl)-4-acetoxy]propyl}-N'-(2-hydroxyäthyl)piperazin und 4-Amino-3-brom-5-(N-äthylcyclohexylaminomethyl)benzoesäure analog Beispiel 4.

Schmelzpunkt des Trishydrogenmaleinats: 150-153°C

*Beispiel 211:*

*N-{2-[4-Amino-3-brom-5-(N-äthylcyclohexylaminomethyl)benzoyloxy]äthyl}-N-{2-[2-fluor-α-methyl-(1,1'-biphenyl)-4-acetoxy]äthyl}-methylamin*

Hergestellt aus N-{2-[4-Amino-3-brom-5-(N-äthylcyclohexylaminomethyl)benzoyloxy]äthyl}-N-(2-hydroxyäthyl)methylamin und 2-Fluor-α-methyl-(1-1'-biphenyl)-4-essigsäure analog Beispiel 4. Öl.

$C_{36}H_{45}BrFN_3O_4$ (682,7)

| | | | | |
|---|---|---|---|---|
| berechnet: | C 63,34 | H 6,64 | Br 11,71 | N 6,16 |
| gefunden: | C 63,45 | H 6,59 | Br 11,85 | N 6,05 |

*Beispiel 212:*

*N-{2-[4-Amino-3-brom-5-(N-äthylcyclohexylaminomethyl)benzoyloxy]äthyl}-N-{2-[2-fluor-α-methyl-(1,1'-biphenyl)-4-acetoxy]äthyl}äthylamin*

Hergestellt aus N-{2-[4-Amino-3-brom-5-(N-äthylcyclohexylaminomethyl)benzoyloxy]äthyl}-N-(2-hydroxyäthyl)äthylamin und 2-Fluor-α-methyl-(1-1'-biphenyl)-4-essigsäure analog Beispiel 4. Öl.

$C_{37}H_{47}BrFN_3O_4$ (696,7)

| | | | | |
|---|---|---|---|---|
| berechnet: | C 63,79 | H 6,80 | Br 11,47 | N 6,04 |
| gefunden: | C 63,80 | H 6,85 | Br 11,40 | N 5,96 |

*Beispiel 213:*

*N-[2-(4-Amino-3-brom-5-diäthylaminomethyl-*

*benzoyloxy)äthyl]-N-{2-[2-fluor-α-methyl-(1,1'-biphenyl)-4-acetoxy]äthyl}methylamin*

Hergestellt aus N-[2-(4-Amino-3-brom-5-diäthylaminomethylbenzoyloxy)äthyl]-N-(2-hydroxyäthyl)methylamin und 2-Fluor-α-methyl-(1,1'-biphenyl)-4-essigsäure analog Beispiel 4. Öl.

$C_{32}H_{39}BrFN_3O_4$ (628,6)

| | | | | |
|---|---|---|---|---|
| berechnet: | C 61,44 | H 6,25 | Br 12,71 | N 6,69 |
| gefunden: | C 61,36 | H 6,54 | Br 12,55 | N 6,71 |

*Beispiel 214:*

*N-[2-(4-Amino-3-brom-5-diäthylaminomethylbenzoyloxy)äthyl]-N-{2-[2-fluor-α-methyl-(1,1'-biphenyl)-4-acetoxy]äthyl}äthylamin*

Hergestellt aus N-[2-(4-Amino-3-brom-5-diäthylaminomethylbenzoyloxy)äthyl]-N-(2-hydroxyäthyl)äthylamin und 2-Fluor-α-methyl-(1,1'-biphenyl)-4-essigsäure analog Beispiel 4. Öl.

$C_{33}H_{41}BrFN_3O_4$ (642,6)

| | | | | |
|---|---|---|---|---|
| berechnet: | C 61,68 | H 6,43 | Br 12,44 | N 6,54 |
| gefunden: | C 61,87 | H 6,21 | Br 12,30 | N 6,50 |

*Beispiel 215:*

*2-(4-Amino-3-brom-5-diäthylaminomethylbenzoyloxy)-2'-[2-fluor-α-methyl-(1,1'-biphenyl)-4-acetoxy]diäthyloxid*

Hergestellt aus 2-(4-Amino-3-brom-5-diäthylaminomethylbenzoyloxy)-2'-hydroxydiäthyloxid und 2-Fluor-α-methyl-(1,1'-biphenyl)-4-essigsäure analog Beispiel 4. Öl.

$C_{31}H_{36}BrFN_2O_5$ (615,6)

| | | | | |
|---|---|---|---|---|
| berechnet: | C 60,49 | H 5,90 | Br 12,98 | N 4,55 |
| gefunden: | C 60,72 | H 5,78 | Br 12,75 | N 4,69 |

*Beispiel 216:*

*2-(4-Amino-3-brom-5-diäthylaminomethylbenzoyloxy)-2'-[2-fluor-α-methyl-(1,1'-biphenyl)-4-acetoxy]diäthylsulfid*

Hergestellt aus 2-(4-Amino-3-brom-5-diäthylaminomethylbenzoyloxy)-2'-hydroxyäthylsulfid und 2-Fluor-α-methyl-(1,1'-biphenyl)-4-essigsäure analog Beispiel 4. Öl.

$C_{31}H_{36}BrFN_2O_4S$ (631,6)

| | | | |
|---|---|---|---|
| berechnet: | C 58,95 | H 5,75 | Br 12,65 |
| | N 4,44 | S 5,08 | |
| gefunden: | C 59,45 | H 5,75 | Br 12,95 |
| | N 4,58 | S 5,18 | |

*Beispiel 217:*

*1-(4-Amino-3-brom-5-dimethylaminomethylbenzoyloxy)-4-(3-benzoyl-α-methylphenylacetoxy)-n-butan*

Hergestellt aus 1-(4-Amino-3-brom-5-dimethylaminomethylbenzoyloxy)-4-hydroxy-n-butan und 3-Benzoyl-α-methylphenylessigsäureimidazolid analog Beispiel 1.

Schmelzpunkt des Hydrochlorids: 117-122°C

*Beispiel 218:*

*1-(4-Amino-3-brom-5-piperidinomethylbenzoyloxy)-4-(3-benzoyl-α-methylphenylacetoxy)-n-butan*

Hergestellt aus 1-(4-Amino-3-brom-4-piperidinomethylbenzoyloxy)-4-hydroxy-n-butan und 3-Benzoyl-α-methylphenylessigsäureimidazolid analog Beispiel 1.

Schmelzpunkt des Hydrochlorids: 123-125°C

*Beispiel 219:*

*1-(4-Amino-3-brom-5-pyrrolidinomethylbenzoyloxy)-4-(3-benzoyl-α-methylphenylacetoxy)-n-butan*

Hergestellt aus 1-(4-Amino-3-brom-5-pyrrolidinomethylbenzoyloxy)-4-hydroxy-n-butan und 3-Benzoyl-α-methylphenylessigsäureimidazolid analog Beispiel 1. Amorphes Hydrochlorid.

$C_{32}H_{35}BrN_2O_5 \cdot HCl$ (644,02)

| | | | |
|---|---|---|---|
| berechnet: | C 59,68 | H 5,63 | Br 12,41 |
| | Cl 5,51 | N 4,35 | |
| gefunden: | C 59,63 | H 5,61 | Br 12,18 |
| | Cl 5,40 | N 4,34 | |

*Beispiel 220:*

*1-(4-Amino-3-brom-5-cyclohexylaminomethylbenzoyloxy)-4-(3-benzoyl-α-methylphenylacetoxy)-n-butan*

Hergestellt aus 1-(4-Amino-3-brom-5-cyclohexylaminomethylbenzoyloxy)-4-hydroxy-n-butan und 3-Benzoyl-α-methylphenylessigsäureimidazolid analog Beispiel 1. Amorphes Hydrochlorid.

$C_{34}H_{39}BrN_2O_5 \cdot HCl$ (672,1)

| | | | |
|---|---|---|---|
| berechnet: | C 60,76 | H 6,00 | Br 11,89 |
| | Cl 5,27 | N 4,17 | |
| gefunden: | C 60,52 | H 6,02 | Br 12,05 |
| | Cl 5,35 | N 4,34 | |

*Beispiel 221:*

*1-[4-Amino-3-brom-5-(N-methylpiperazinomethyl)benzoyloxy]-4-(3-benzoyl-α-methylphenylacetoxy)-n-butan*

Hergestellt aus 1-[4-Amino-3-brom-5-(N-methylpiperazinomethyl)benzoyloxy]-4-hydroxy-n-butan und 3-Benzoyl-α-methylphenylessigsäureimidazolid analog Beispiel 1.

Schmelzpunkt des Hydrochlorids: 183-186°C

*Beispiel 222:*

*1-(4-Amino-3-brom-5-isopropylaminomethylbenzoyloxy)-4-(3-benzoyl-α-methylphenylacetoxy)-n-butan*

Hergestellt aus 1-(4-Amino-3-brom-5-isopropylaminomethylbenzoyloxy)-4-hydroxy-n-butan und 3-Benzoyl-α-methylphenylessigsäureimidazolid analog Beispiel 1. Amorphes Hydrochlorid.

$C_{31}H_{35}BrN_2O_5 \cdot HCl$ (632,0)

| | | | |
|---|---|---|---|
| berechnet: | C 58,9 | H 5,70 | Br 12,60 |
| | Cl 5,60 | N 4,41 | |

gefunden: C 57,7 H 6,08 Br 12,57
Cl 5,60 N 4,70

*Beispiel 223:*

*1-(4-Amino-3-brom-5-tert.-butylaminomethylbenzoyloxy)-4-(3-benzoyl-α-methylphenylacetoxy)-n-butan*

Hergestellt aus 1-(4-Amino-3-brom-5-tert.-butylaminomethylbenzoyloxy)-4-hydroxy-n-butan und 3-Benzoyl-α-methylphenylessigsäureimidazolid analog Beispiel 1. Amorphe Substanz.

IR-Spektrum (Methylenchlorid):
CO 1660 $cm^{-1}$
Ester-CO 1710 $cm^{-1}$, 1730 $cm^{-1}$
UV-Spektrum (Äthanol): $\lambda_{max.}$ 254 nm, 280 nm

*Beispiel 224:*

*1-(4-Amino-3-brom-5-hexamethyleniminomethylbenzoyloxy)-4-(3-benzoyl-α-methylphenylacetoxy)-n-butan*

Hergestellt aus 1-(4-Amino-3-brom-5-hexamethyleniminomethylbenzoyloxy)-4-hydroxy-n-butan und 3-Benzoyl-α-methylphenylessigsäureimidazolid analog Beispiel 1. Amorphes Hydrochlorid.

$C_{34}H_{39}BrN_2O_5 \cdot HCl$ (672,1)

| | | | |
|---|---|---|---|
| berechnet: | C 60,8 | H 6,0 | Br 11,9 |
| | Cl 5,3 | N 4,2 | |
| gefunden: | C 59,5 | H 6,5 | Br 12,0 |
| | Cl 5,3 | N 4,5 | |

*Beispiel 225:*

*1-[4-Amino-3-brom-5-(N-äthylcycloheptylaminomethyl)benzoyloxy]-4-(3-benzoyl-α-methylphenylacetoxy)-n-butan*

Hergestellt aus 1-[4-Amino-3-brom-5-(N-äthylcycloheptylaminomethyl)benzoyloxy]-4-hydroxy-n-butan und 3-Benzoyl-α-methylphenylessigsäureimidazolid analog Beispiel 1. Amorphes Hydrochlorid.

$C_{37}H_{45}BrN_2O_5 \cdot HCl$ (714,2)

| | | | |
|---|---|---|---|
| berechnet: | C 62,2 | H 6,5 | Br 11,2 |
| | Cl 5,0 | N 3,9 | |
| gefunden: | C 61,2 | H 6,7 | Br 11,2 |
| | Cl 5,1 | N 4,1 | |

*Beispiel 226:*

*1-[4-Amino-3-brom-5-(N-äthylcyclopentylaminomethyl)benzoyloxy]-4-(3-benzoyl-α-methylphenylacetoxy)-n-butan*

Hergestellt aus 1-[4-Amino-3-brom-5-(N-äthylcyclopentylaminomethyl)benzoyloxy]-4-hydroxy-n-butan und 3-Benzoyl-α-methylphenylessigsäureimidazolid analog Beispiel 1. Amorphes Hydrochlorid.

$C_{35}H_{41}BrN_2O_5 \cdot HCl$ (686,1)

| | | | | |
|---|---|---|---|---|
| berechnet: | C 61,3 | H 6,2 | Br 11,6 | Cl 5,2 |
| gefunden: | C 61,7 | H 6,5 | Br 11,4 | Cl 5,1 |

*Beispiel 227:*

*1-(4-Amino-3-brom-5-morpholinomethylbenzoyloxy)-4-(3-benzoyl-α-methylphenylacetoxy)-n-butan*

Hergestellt aus 1-(4-Amino-3-brom-5-morpholinomethylbenzoyloxy)-4-hydroxy-n-butan und 3-Benzoyl-α-methylphenylessigsäureimidazolid analog Beispiel 1.

Schmelzpunkt des Hydrochlorids: 148-150°C

*Beispiel 228:*

*1-[4-Amino-3-brom-5-(2-diäthylaminoäthylaminomethyl)benzoyloxy]-2-(3-benzoyl-α-methylphenylacetoxy)äthan*

Hergestellt aus 1-[4-Amino-3-brom-5-(2-diäthylaminoäthylaminomethyl)benzoyloxy]-2-hydroxyäthan und 3-Benzoyl-α-methylphenylessigsäureimidazolid analog Beispiel 1.

Schmelzpunkt des Hydrochlorids: ab 70°C (Zers.)

*Beispiel 229:*

*1-(4-Amino-3-brom-5-diäthylaminomethylbenzoyloxy)-2-(3-benzoyl-α-methylphenylacetoxy)äthan*

Hergestellt aus 2-(4-Amino-3-brom-5-diäthylaminomethylbenzoyloxy)äthylchlorid und 3-Benzoyl-α-methylphenylessigsäurenatriumsalz analog Beispiel 8.

Schmelzpunkt des Hydrochlorids: 53-59°C

*Beispiel 230:*

*1-(4-Amino-3-brom-5-diäthylaminomethylbenzoyloxy)-3-(3-benzoyl-α-methylphenylacetoxy)-n-propan*

Hergestellt aus 3-(4-Amino-3-brom-5-diäthylaminomethylbenzoyloxy)-n-propylchlorid und 3-Benzoyl-α-methylphenylessigsäurenatriumsalz analog Beispiel 8. Öl.

IR-Spektrum (Methylenchlorid):
$NH_2$ 3450 $cm^{-1}$
CO 1660 $cm^{-1}$
Ester-CO 1710 $cm^{-1}$, 1730 $cm^{-1}$
UV-Spektrum (Äthanol): $\lambda_{max.}$ 254 nm, 288 nm

*Beispiel 231:*

*1-(4-Amino-3-brom-5-diäthylaminomethylbenzoyloxy)-4-(3-benzoyl-α-methylphenylacetoxy)-n-butan*

Hergestellt aus 4-(4-Amino-3-brom-5-diäthylaminomethylbenzoyloxy)-n-butylchlorid und 3-Benzoyl-α-methylphenylessigsäurenatriumsalz analog Beispiel 8.

$C_{32}H_{37}BrN_2O_5$ (609)

| | | | |
|---|---|---|---|
| berechnet: | C 63,05 | H 6,11 | N 4,59 |
| gefunden: | C 62,50 | H 6,51 | N 4,42 |

*Beispiel 232:*

*2-(4-Amino-3-brom-5-morpholinomethylbenzoyloxy)-2'-(3-benzoyl-α-methylphenylacetoxy)diäthylsulfid*

Hergestellt aus 2-(4-Amino-3-brom-5-morpholinomethylbenzoyloxy)-2'-hydroxydiäthylsulfid und 3-Benzoyl-α-methylphenylessigsäure analog Beispiel 8.

Schmelzpunkt des Hydrochlorids: 146-149°C

*Beispiel 233:*

*2-(4-Amino-3-brom-5-morpholinomethylbenzoyloxy)-2'-(3-benzoyl-α-methylphenylacetoxy)diäthyloxid*

Hergestellt aus 2-(4-Amino-3-brom-5-morpholinomethylbenzoyloxy)-2'-hydroxydiäthyloxid und 3-Benzoyl-α-methylphenylessigsäure analog Beispiel 4. Öl.

IR-Spektrum (Methylenchlorid):
Ester-CO 1710 und 1730 $cm^{-1}$
Keto-CO 1660 $cm^{-1}$
UV-Spektrum (Äthanol): $\lambda_{max.}$ 255 und 285 nm

*Beispiel 234:*

*1-(4-Amino-3-brom-5-piperidinomethylbenzoyloxy)-2-{2-[(2,6-dichlorphenyl)amino]phenylacetoxy}äthan*

Hergestellt aus 2-(4-Amino-3-brom-5-piperidinomethylbenzoyloxy)äthylchlorid und 2-[(2,6-Dichlorphenyl)amino]phenylessigsäurenatriumsalz analog Beispiel 8.

Schmelzpunkt des Hydrochlorids: 186-191°C

*Beispiel 235:*

*1-(4-Amino-3-brom-5-pyrrolidinomethylbenzoyloxy)-2-{2-[(2,6-dichlorphenyl)amino]phenylacetoxy}äthan*

Hergestellt aus 2-(4-Amino-3-brom-5-pyrrolidinomethylbenzoyloxy)äthylchlorid und 2-[(2,6-Dichlorphenyl)amino]phenylessigsäurenatriumsalz analog Beispiel 8.

Schmelzpunkt des Hydrochlorids: 186-187°C

*Beispiel 236:*

*1-[4-Amino-3-brom-5-(N-methylcyclohexylaminomethyl)benzoyloxy]-2-{2-[(2,6-dichlorphenyl)amino]phenylacetoxy}äthan*

Hergestellt aus 2-[4-Amino-3-brom-5-(N-methylcyclohexylaminomethyl)benzoyloxy]-äthylchlorid und 2-[(2,6-Dichlorphenyl)amino]-phenylessigsäurenatriumsalz analog Beispiel 8. Öl.

IR-Spektrum (Methylenchlorid):
$NH_2$ 3440 $cm^{-1}$
Ester-CO 1710 $cm^{-1}$
UV-Spektrum (Äthanol): $\lambda_{max.}$ 228 nm, 286 nm

*Beispiel 237:*

*1-(4-Amino-3-brom-5-morpholinomethylbenzoyloxy)-2-{2-[(2,6-dichlorphenyl)amino]-phenylacetoxy}äthan*

Hergestellt aus 2-(4-Amino-3-brom-5-morpholinomethylbenzoyloxy)äthylchlorid und 2-[(2,6-Dichlorphenyl)amino]phenylessigsäurenatriumsalz analog Beispiel 8. Öl.

IR-Spektrum (Methylenchlorid):
$NH_2$ 3440 $cm^{-1}$
Ester-CO 1710 $cm^{-1}$
UV-Spektrum (Äthanol): $\lambda_{max.}$ 282 nm

*Beispiel 238:*

*1-[4-Amino-3-brom-5-(N-cyclohexyl-n-propylaminomethyl)benzoyloxy]-2-{2-[(2,6-dichlorphenyl)amino]phenylacetoxy}äthan*

Hergestellt aus 2-[4-Amino-3-brom-5-(N-cyclohexyl-n-propylaminomethyl)benzoyloxy]-äflchlorid und 2-[(2,6-Dichlorphenyl)amino]-phenylessigsäurenatriumsalz analog Beispiel 8.

Schmelzpunkt: 110,5-112°C

*Beispiel 239:*

*1-[4-Amino-3-brom-5-(N-äthylcycloheptylaminomethyl)benzoyloxy]-2-{2-[(2,6-dichlorphenyl)amino]phenylacetoxy}äthan*

Hergestellt aus 2-[4-Amino-3-brom-5-(N-äthylcycloheptylaminomethyl)benzoyloxy]äthyl chlorid und 2-[(2,6-Dichlorphenyl)amino]-phenylessigsäurenatriumsalz analog Beispiel 8. Öl.

IR-Spektrum (Methylenchlorid):
$NH_2$ 3440 $cm^{-1}$
Ester-CO 1710 $cm^{-1}$
UV-Spektrum (Äthanol): $\lambda_{max.}$ 285 nm

*Beispiel 240:*

*1-[4-Amino-3-brom-5-(N-äthylcyclopentylaminomethyl)benzoyloxy]-2-{2-[(2,6-dichlorphenyl)amino]phenylacetoxy}äthan*

Hergestellt aus 2-[4-Amino-3-brom-5-(N-äthylcyclopentylaminomethyl)benzoyloxy]äthyl chlorid und 2-[(2,6-Dichlorphenyl)amino]-phenylessigsäurenatriumsalz analog Beispiel 8. Öl.

IR-Spektrum (Methylenchlorid):
$NH_2$ 3440 $cm^{-1}$
Ester-CO 1710 $cm^{-1}$
UV-Spektrum (Äthanol): $\lambda_{max.}$ 285 nm

*Beispiel 241:*

*1-(4-Amino-3-brom-5-tert.-butylaminomethylbenzoyloxy)-2-{2-[(2,6-dichlorphenyl)amino]-phenylacetoxy}äthan*

Hergestellt aus 2-(4-Amino-3-brom-5-tert.-butylaminomethylbenzoyloxy)äthylchlorid und 2-[(2,6-Dichlorphenyl)amino]phenylessigsäurenatriumsalz analog Beispiel 8.

Schmelzpunkt des Hydrochlorids: 224-225°C

*Beispiel 242:*

*1-(4-Amino-3-brom-5-dimethylaminomethylbenzoyloxy)-2-{2-[(2,6-dichlorphenyl)amino]-phenylacetoxy}äthan*

Hergestellt aus 2-(4-Amino-3-brom-5-dimethylaminomethylbenzoyloxy)äthylchlorid und 2-[(2,6-Dichlorphenyl)amino]phenylessigsäurenatriumsalz analog Beispiel 8.

Schmelzpunkt des Hydrochlorids: 130,5-132°C

*Beispiel 243:*

*1-(4-Amino-3-brom-5-hexamethyleniminomethylbenzoyloxy)-2-{2-[(2,6-dichlorphenyl)-amino]phenylacetoxy}äthan*

Hergestellt aus 2-(4-Amino-3-brom-5-hexa-

methyleniminomethylbenzoyloxyäthylchlorid und 2-[(2,6-Dichlorphenyl)amino]phenylessigsäurenatriumsalz analog Beispiel 8.

Schmelzpunkt des Hydrochlorids: 182-183°C

*Beispiel 244:*

*1-[4-Amino-3-brom-5-(N-äthylcyclohexylaminomethyl)benzamido]-3-{2-[(2,6-dichlorphenyl)amino]phenylacetoxy}-n-propan*

Hergestellt aus 3-[4-Amino-3-brom-5-(N-äthylcyclohexylaminomethyl)benzamido]-n-propylchlorid und 2-[(2,6-Dichlorphenyl)-amino]phenylessigsäurenatriumsalz analog Beispiel 8.

Schmelzpunkt: 148-150°C

*Beispiel 245:*

*1-[4-Amino-3-brom-5-(N-äthylcycloheptylaminomethyl)benzoyloxy]-4-{2-[(2,6-dichlorphenyl)amino]phenylacetoxy}-n-butan*

Hergestellt aus 4-[4-Amino-3-brom-5-(N-äthylcycloheptylaminomethyl)benzoyloxy]-n-butylchlorid und 2-[(2,6-Dichlorphenyl)amino]-phenylessigsäurenatriumsalz analog Beispiel 8.

$C_{35}H_{42}BrCl_2N_3O_4$ (719)

| | | | |
|---|---|---|---|
| berechnet: | C 58,42 | H 5,88 | Br 11,11 |
| | Cl 9,86 | N 5,84 | |
| gefunden: | C 58,97 | H 5,77 | Br 10,84 |
| | Cl 9,61 | N 5,71 | |

*Beispiel 246:*

*1-[4-Amino-3-brom-5-(N-cyclohexyl-n-propylaminomethyl)benzoyloxy]-4-{2-[(2,6-dichlorphenyl)amino]phenylacetoxy}-n-butan*

Hergestellt aus 4-[4-Amino-3-brom-5-(N-cyclohexyl-n-propylaminomethyl)benzoyloxy]-n-butylchlorid und 2-[(2,6-Dichlorphenyl-amino]phenylessigsäurenatriumsalz analog Beispiel 8. Öl

$C_{35}H_{42}BrCl_2N_3O_4$ (719)

| | | | |
|---|---|---|---|
| berechnet: | C 58,42 | H 5,88 | Br 11,11 |
| | Cl 9,86 | N 5,84 | |
| gefunden: | C 58,16 | H 6,03 | Br 10,95 |
| | Cl 9,73 | N 5,84 | |

*Beispiel 247:*

*1-(4-Amino-3-brom-5-hexamethyleniminomethylbenzoyloxy)-4-{2-[(2,6-dichlorphenyl)-amino]phenylacetoxy}-n-butan*

Hergestellt aus 4-(4-Amino-3-brom-5-hexamethyleniminomethylbenzoyloxy)-n-butylchlorid und 2-[(2,6-Dichlorphenylamino]phenylessigsäurenatriumsalz analog Beispiel 8.

Schmelzpunkt des Hydrochlorids: 122-125°C

*Beispiel 248:*

*1-(4-Amino-3-brom-5-tert.-butylaminomethylbenzoyloxy)-4-{2-[(2,6-dichlorphenyl)amino]-phenylacetoxy}-n-butan*

Hergestellt aus 4-(4-Amino-3-brom-5-tert.-butylaminomethylbenzoyloxy)-n-butylchlorid und 2-[(2,6-Dichlorphenyl)amino]phenylessigsäurenatriumsalz analog Beispiel 8.

Schmelzpunkt des Hydrochlorids: 122-124°C

*Beispiel 249:*

*1-[4-Amino-3-brom-5-(N-methylcyclohexylaminomethyl)benzoyloxy]-4-{2-[(2,6-dichlorphenyl)amino]phenylacetoxy}-n-butan*

Hergestellt aus 4-[4-Amino-3-brom-5-(N-methylcyclohexylaminomethyl)benzoyloxy]-n-butylchlorid und 2-[(2,6-Dichlorphenyl)amino]-phenylessigsäurenatriumsalz analog Beispiel 8.

Schmelzpunkt des Hydrochlorids: 145-147°C

*Beispiel 250:*

*1-(4-Amino-3-brom-5-dimethylaminomethylbenzoyloxy)-4-{2-[(2,6-dichlorphenyl)amino]-phenylacetoxy}-n-butan*

Hergestellt aus 4-(4-Amino-3-brom-5-dimethylaminomethylbenzoyloxy)-n-butylchlorid und 2-[(2,6-Dichlorphenyl)amino]phenylessigsäurenatriumsalz analog Beispiel 8.

Schmelzpunkt des Hydrochlorids: 87-90°C

*Beispiel 251:*

*1-(4-Amino-3-brom-5-pyrrolidinomethylbenzoyloxy)-4-{2-[(2,6-dichlorphenyl)amino]-phenylacetoxy}-n-butan*

Hergestellt aus 4-(4-Amino-3-brom-5-pyrrolidinomethylbenzoyloxy)-n-butylchlorid und 2-[(2,6-Dichlorphenyl)amino]phenylessigsäurenatriumsalz analog Beispiel 8.

Schmelzpunkt des Hydrochlorids: 143-145°C

*Beispiel 252:*

*1-(4-Amino-3-brom-5-morpholinomethylbenzoyloxy)-4-{2-[(2,6-dichlorphenyl)amino]-phenylacetoxy}-n-butan*

Hergestellt aus 4-(4-Amino-3-brom-5-morpholinomethylbenzoyloxy)-n-butylchlorid und 2-[(2,6-Dichlorphenyl)amino]phenylessigsäurenatriumsalz analog Beispiel 8.

Schmelzpunkt des Hydrochlorids: 174-176°C

*Beispiel 253:*

*1-(4-Amino-3-brom-5-piperidinomethylbenzoyloxy)-4-{2-[(2,6-dichlorphenyl)amino]phenylacetoxy}-n-butan*

Hergestellt aus 4-(4-Amino-3-brom-5-piperidinomethylbenzoyloxy)-n-butylchlorid und 2-[(2,6-Dichlorphenyl)amino]phenylessigsäurenatriumsalz analog Beispiel 8.

Schmelzpunkt des Hydrochlorids: 184-185°C

*Beispiel 254:*

*1-[4-Amino-3-brom-5-(N-methyl-n-propylaminomethyl)benzoyloxy]-4-{2-[(2,6-dichlorphenyl)amino]phenylacetoxy}-n-butan*

Hergestellt aus 4-[4-Amino-3-brom-5-(N-methyl-n-propylaminomethyl)benzoyloxy]-n-butylchlorid und 2-[(2,6-Dichlorphenyl)amino]-phenylessigsäurenatriumsalz analog Beispiel 8.

IR-Spektrum (Methylenchlorid):
$NH_2$ 3450 $cm^{-1}$
Ester-CO 1700 $cm^{-1}$
UV-Spektrum (Äthanol): $\lambda_{max.}$ 282 nm

*Beispiel 255:*

*1-[4-Amino-3-brom-5-(N-methylbenzylaminomethyl)benzoyloxy]-4-{2-[(2,6-dichlorphenyl)-amino]phenylacetoxy}-n-butan*

Hergestellt aus 4-[4-Amino-3-brom-5-(N-methylbenzylaminomethyl)benzoyloxy]-n-butylchlorid und 2-[(2,6-Dichlorphenyl)amino]-phenylessigsäurenatriumsalz analog Beispiel 8.
IR-Spektrum (Methylenchlorid):
$NH_2$ 3445 $cm^{-1}$
Ester-CO 1700 $cm^{-1}$
UV-Spektrum (Äthanol): $\lambda_{max.}$ 283 nm

*Beispiel 256:*

*1-(4-Amino-3-brom-5-cyclohexylaminomethylbenzoyloxy)-4-{2-[(2,6-dichlorphenyl)amino]-phenylacetoxy}-n-butan*

Hergestellt aus 4-(4-Amino-3-brom-5-cyclohexylaminomethylbenzoyloxy)-n-butylchlorid und 2-[(2,6-Dichlorphenyl)amino]phenylessigsäurenatriumsalz analog Beispiel 8.
Schmelzpunkt des Hydrochlorids: 166-168°C

*Beispiel 257:*

*1-(4-Amino-3-brom-5-isopropylaminomethylbenzoyloxy)-4-{2-[(2,6-dichlorphenyl)amino]-phenylacetoxy}-n-butan*

Hergestellt aus 4-(4-Amino-3-brom-5-isopropylaminomethylbenzoyloxy)-n-butylchlorid und 2-[(2,6-Dichlorphenyl)amino]phenylessigsäurenatriumsalz analog Beispiel 8.
IR-Spektrum (Methylenchlorid):
$NH_2$ 3440 $cm^{-1}$
Ester-CO 1705 $cm^{-1}$
UV-Spektrum (Äthanol): $\lambda_{max.}$ 282 nm

*Beispiel 258:*

*1-[4-Amino-3-brom-5-(N-äthylcyclohexylaminomethyl)benzamido]-4-{2-[(2,6-dichlorphenyl)amino]phenylacetoxy}-n-butan*

Hergestellt aus 4-[4-Amino-3-brom-5-(N-äthylcyclohexylaminomethyl)benzamido]-n-butylchlorid und 2-[(2,6-Dichlorphenyl)amino]-phenylessigsäurenatriumsalz analog Beispiel 8. Amorphes Hydrochlorid.
IR-Spektrum (Methylenchlorid):
Ester-CO 1720 $cm^{-1}$
Amid-CO 1640 $cm^{-1}$
UV-Spektrum (Äthanol): $\lambda_{max.}$ 275 nm

*Beispiel 259:*

*1-[4-Amino-3-brom-5-(N-methylbenzylaminomethyl)benzoyloxy]-5-{2-[(2,6-dichlorphenyl)-amino]phenylacetoxy}-n-pentan*

Hergestellt aus 5-[4-Amino-3-brom-5-(N-methylbenzylaminomethyl)benzoyloxy]-n-pentylchlorid und 2-[(2,6-Dichlorphenyl)amino]-phenylessigsäurenatriumsalz analog Beispiel 8. Öl.
IR-Spektrum (Methylenchlorid):
$NH_2$ 3450 $cm^{-1}$
Ester-CO 1700 $cm^{-1}$
UV-Spektrum (Äthanol): $\lambda_{max.}$ 283 nm

*Beispiel 260:*

*1-(4-Amino-3-brom-5-isopropylaminomethylbenzoyloxy)-5-{2-[(2,6-dichlorphenyl)amino]-phenylacetoxy}-n-pentan*

Hergestellt aus 5-(4-Amino-3-brom-5-isopropylaminomethylbenzoyloxy)-n-pentylchlorid und 2-[(2,6-Dichlorphenyl)amino]phenylessigsäurenatriumsalz analog Beispiel 8. Öl.
IR-Spektrum (Methylenchlorid):
$NH_2$ 3450 $cm^{-1}$
Ester-CO 1705 $cm^{-1}$
UV-Spektrum (Äthanol): $\lambda_{max.}$ 283 nm

*Beispiel 261:*

*1-(4-Amino-3-brom-5-cyclohexylaminomethylbenzoyloxy)-5-{2-[(2,6-dichlorphenyl)amino]-phenylacetoxy}-n-pentan*

Hergestellt aus 5-(4-Amino-3-brom-5-cyclohexylaminomethylbenzoyloxy)-n-pentylchlorid und 2-[(2,6-Dichlorphenyl)amino]phenylessigsäurenatriumsalz analog Beispiel 8.
Schmelzpunkt des Hydrochlorids: 152-153°C

*Beispiel 262:*

*1-[4-Amino-3-brom-5-(N-methyl-n-propylaminomethyl)benzoyloxy]-5-{2-[(2,6-dichlorphenyl)amino]phenylacetoxy}-n-pentan*

Hergestellt aus 5-[4-Amino-3-brom-5-(N-methyl-n-propylaminomethyl)benzoyloxy]-n-pentylchlorid und 2-[(2,6-Dichlorphenyl)-amino]phenylessigsäurenatriumsalz analog Beispiel 8.
Schmelzpunkt des Hydrochlorids: 50-60°C

*Beispiel 263:*

*1-(4-Amino-3-brom-5-piperidinomethylbenzoyloxy)-5-{2-[(2,6-dichlorphenyl)amino]-phenylacetoxy}-n-pentan*

Hergestellt aus 5-(4-Amino-3-brom-5-piperidinomethylbenzoyloxy)-n-pentylchlorid und 2-[(2,6-Dichlorphenyl)amino]phenylessigsäurenatriumsalz analog Beispiel 8.
Schmelzpunkt des Hydrochlorids: 183-185°C

*Beispiel 264:*

*1-[4-Amino-3-brom-5-(N-methylpiperazinomethyl)benzoyloxy]-5-{2-[(2,6-dichlorphenyl)-amino]phenylacetoxy}-n-pentan*

Hergestellt aus 5-[4-Amino-3-brom-5-(N-methylpiperazinomethyl)benzoyloxy]-n-pentylchlorid und 2-[(2,6-Dichlorphenyl)amino]-phenylessigsäurenatriumsalz analog Beispiel 8.
Schmelzpunkt des Hydrochlorids: 193-196°C

*Beispiel 265:*

*1-(4-Amino-3-brom-5-morpholinomethylbenzoyloxy)-5-{2-[(2,6-dichlorphenyl)amino]-phenylacetoxy}-n-pentan*

Hergestellt aus 5-(4-Amino-3-brom-5-mor-

pholinomethylbenzoyloxy)-n-pentylchlorid und 2-[(2,6-Dichlorphenyl)amino]phenylessigsäure-natriumsalz analog Beispiel 8.

Schmelzpunkt des Hydrochlorids: 185-187°C

*Beispiel 266:*

*1-(4-Amino-3-brom-5-pyrrolidinomethylbenzoyloxy)-5-{2-[(2,6-dichlorphenyl)amino]-phenylacetoxy}-n-pentan*

Hergestellt aus 5-(4-Amino-3-brom-5-pyrrolidinomethylbenzoyloxy)-n-pentylchlorid und 2-[(2,6-Dichlorphenyl)amino]phenylessigsäure-natriumsalz analog Beispiel 8.

Schmelzpunkt des Hydrochlorids: 152-153°C

*Beispiel 267:*

*1-(4-Amino-3-brom-5-dimethylaminomethylbenzoyloxy)-5-{2-[(2,6-dichlorphenyl)amino]-phenylacetoxy}-n-pentan*

Hergestellt aus 5-(4-Amino-3-brom-5-dimethylaminomethylbenzoyloxy)-n-pentylchlorid und 2-[(2,6-Dichlorphenyl)amino]phenylessigsäurenatriumsalz analog Beispiel 8.

Schmelzpunkt des Hydrochlorids: 140-144°C

*Beispiel 268:*

*1-[4-Amino-3-brom-5-(N-methylcyclohexylaminomethyl)benzoyloxy]-5-{2-[(2,6-dichlorphenyl)amino]phenylacetoxy}-n-pentan*

Hergestellt aus 5-[4-Amino-3-brom-5-(N-methylcyclohexylaminomethyl)benzoyloxy]-n-pentylchlorid und 2-[(2,6-Dichlorphenyl)-amino]phenylessigsäurenatriumsalz analog Beispiel 8.

Schmelzpunkt des Hydrochlorids: 105-110°C

*Beispiel 269:*

*1-(4-Amino-3-brom-5-tert.-butylaminomethylbenzoyloxy)-5-{2-[(2,6-dichlorphenyl)amino]-phenylacetoxy}-n-pentan*

Hergestellt aus 5-(4-Amino-3-brom-5-tert.-butylaminomethylbenzoyloxy)-n-pentylchlorid und 2-[(2,6-Dichlorphenyl)amino]phenylessigsäurenatriumsalz analog Beispiel 8.

Schmelzpunkt des Hydrochlorids: 109-111°C

*Beispiel 270:*

*1-(4-Amino-3-brom-5-hexamethyleniminomethylbenzoyloxy)-5-{2-[(2,6-dichlorphenyl)-amino]phenylacetoxy}-n-pentan*

Hergestellt aus 5-(4-Amino-3-brom-5-hexamethyleniminomethylbenzoyloxy)-n-pentylchlorid und 2-[(2,6-Dichlorphenyl)amino]-phenylessigsäurenatriumsalz analog Beispiel 8.

Schmelzpunkt des Hydrochlorids: 149-150°C

*Beispiel 271:*

*1-[4-Amino-3-brom-5-(N-cyclohexyl-n-propylaminomethyl)benzoyloxy]-5-{2-[(2,6-dichlorphenyl)amino]phenylacetoxy}-n-pentan*

Hergestellt aus 5-[4-Amino-3-brom-5-(N-cyclohexyl-n-propylaminomethyl)benzoyloxy]-n-pentylchlorid und 2-[(2,6-Dichlorphenyl)-amino]phenylessigsäurenatriumsalz analog Beispiel 8.

Schmelzpunkt des Hydrochlorids: 113-115°C

*Beispiel 272:*

*1-[4-Amino-3-brom-5-(N-äthylcyclohexylaminomethyl)benzamido]-5-{2-[(2,6-dichlorphenyl)amino]phenylacetoxy}-n-pentan*

Hergestellt aus 5-[4-Amino-3-brom-5-(N-äthylcyclohexylaminomethyl)benzamido]-n-pentylchlorid und 2-[(2,6-Dichlorphenyl)-amino]phenylessigsäurenatriumsalz analog Beispiel 8. Amorphes Dihydrochlorid.

IR-Spektrum (Methylenchlorid):
Ester-CO 1720 $cm^{-1}$
Amid-CO 1640 $cm^{-1}$
UV-Spektrum (Äthanol): $\lambda_{max.}$ 275 nm

*Beispiel 273:*

*1-(4-Amino-3-brom-5-diäthylaminomethylbenzoyloxy)-6-{2-[(2,6-dichlorphenyl)amino]-phenylacetoxy}hexan*

Hergestellt aus 6-(4-Amino-3-brom-5-diäthylaminomethylbenzoyloxy)-n-hexylchlorid und 2-[(2,6-Dichlorphenyl)amino]phenylessigsäure-natriumsalz analog Beispiel 8.

Schmelzpunkt des Hydrochlorids: 63-69°C

*Beispiel 274:*

*1-[4-Amino-3-brom-5-(N-äthylcyclohexylaminomethyl)benzamido]-6-{2-[(2,6-dichlorphenyl)amino]phenylacetoxy}-n-hexan*

Hergestellt aus 6-[4-Amino-3-brom-5-(N-äthylcyclohexylaminomethyl)benzamido]-n-hexylchlorid und 2-[(2,6-Dichlorphenyl)amino]-phenylessigsäurenatriumsalz analog Beispiel 8. Amorphes Hydrochlorid.

$C_{36}H_{45}BrCl_2N_4O_3$ (769,1)

| | | | |
|---|---|---|---|
| berechnet: | C 56,22 | H 6,03 | Br 10,39 |
| | Cl 13,83 | N 7,28 | |
| gefunden: | C 56,69 | H 6,23 | Br 10,15 |
| | Cl 13,54 | N 7,25 | |

*Beispiel 275:*

*1-[4-Amino-3-brom-5-(N-äthylcyclohexylaminomethyl)benzoyloxy]-7-{2-[(2,6-dichlorphenyl)amino]phenylacetoxy}-n-heptan*

Hergestellt aus 7-[4-Amino-3-brom-5-(N-äthylcyclohexylaminomethyl)benzoyloxy]-n-heptylchlorid und 2-[(2,6-Dichlorphenyl)-amino]phenylessigsäurenatriumsalz analog Beispiel 8.

Schmelzpunkt des Hydrochlorids: 96-100°C

*Beispiel 276:*

*1-[4-Amino-3-brom-5-(N-äthylcyclohexylaminomethyl)benzoyloxy]-8-{2-[(2,6-dichlorphenyl)amino]phenylacetoxy}-n-octan*

Hergestellt aus 8-[4-Amino-3-brom-5-(N-äthylcyclohexylaminomethylbenzoyloxy]-n-octylchlorid und 2-[(2,6-Dichlorphenyl)amino]-phenylessigsäurenatriumsalz analog Beispiel 8.

Schmelzpunkt des Hydrochlorids: 131-133°C

*Beispiel 277:*

*1-[4-Amino-3-brom-5-(N-äthylcyclohexylaminomethyl)benzoyloxy]-9-{2-[(2,6-dichlorphenyl)amino]phenylacetoxy}-n-nonan*

Hergestellt aus 9-[4-Amino-3-brom-5-(N-äthylcyclohexylaminomethyl)benzoyloxy]-n-nonylchlorid und 2-[(2,6-Dichlorphenyl)amino]-phenylessigsäurenatriumsalz analog Beispiel 8.

Schmelzpunkt des Hydrochlorids: 106-108°C

*Beispiel 278:*

*1-[4-Amino-3-brom-5-(N-äthylcyclohexylaminomethyl)benzoyloxy]-10-{2-[(2,6-dichlorphenyl)amino]phenylacetoxy}-n-decan*

Hergestellt aus 10-[4-Amino-3-brom-5-(N-äthylcyclohexylaminomethyl)benzoyloxy]-n-decylchlorid und 2-[(2,6-Dichlorphenyl)amino]-phenylessigsäurenatriumsalz analog Beispiel 8.

Schmelzpunkt des Hydrochlorids: 125-127°C

*Beispiel 279:*

*2-(4-Amino-3-brom-5-piperidinomethylbenzoyloxy)-2'-{2-[(2,6-dichlorphenyl)amino]-phenylacetoxy}diäthylsulfid*

Hergestellt aus 2-(4-Amino-3-brom-5-piperidinomethylbenzoyloxy)-2'-hydroxydiäthylsulfid und 2-[(2,6-Dichlorphenyl)amino]phenylessigsäure analog Beispiel 4.

Schmelzpunkt des Hydrochlorids: 135-138°C (unter Aufschäumen)

*Beispiel 280:*

*2-(4-Amino-3-brom-5-piperidinomethylbenzoyloxy)-2'-{2-[(2,6-dichlorphenyl)amino]-phenylacetoxy}diäthyloxid*

Hergestellt aus 2-(4-Amino-3-brom-5-piperidinomethylbenzoyloxy)-2'-hydroxydiäthyloxid und 2-[(2,6-Dichlorphenyl)amino]phenylessigsäure analog Beispiel 4. Öl.

$C_{31}H_{34}BrCl_2N_3O_5$ (679,5)

| | | | |
|---|---|---|---|
| berechnet: | C 54,80 | H 5,05 | Br 11,76 |
| | Cl 10,44 | N 6,18 | |
| gefunden: | C 54,50 | H 5,18 | Br 11,75 |
| | Cl 10,44 | N 6,27 | |

*Beispiel 281:*

*2-(4-Amino-3-brom-5-hexamethyleniminomethylbenzoyloxy)-2'-{2-[(2,6-dichlorphenyl)-amino]phenylacetoxy}diäthyloxid*

Hergestellt aus 2-(4-Amino-3-brom-5-hexamethyleniminomethylbenzoyloxy)-2'-hydroxydiäthyloxid und 2-[(2,6-Dichlorphenyl)amino]-phenylessigsäure analog Beispiel 4. Öl.

$C_{32}H_{36}BrCl_{22}N_3O_5$ (693,5)

| | | | |
|---|---|---|---|
| berechnet: | C 55,42 | H 5,23 | Br 11,52 |
| | Cl 10,23 | N 6,06 | |
| gefunden: | C 55,77 | H 5,53 | Br 11,20 |
| | Cl 9,93 | N 5,78 | |

*Beispiel 282:*

*2-(4-Amino-3-brom-5-dimethylaminomethylbenzoyloxy)-2'-{2-[(2,6-dichlorphenyl)amino]-phenylacetoxy}diäthyloxid*

Hergestellt aus 2-(4-Amino-3-brom-5-dimethylaminomethylbenzoyloxy)-2'-hydroxydiäthyloxid und 2-[(2,6-Dichlorphenyl)amino]phenylessigsäure analog Beispiel 4. Öl.

IR-Spektrum (Methylenchlorid):
Ester-CO 1710 $cm^{-1}$
$NH_2$ 3450 $cm^{-1}$
UV-Spektrum (Äthanol): $\lambda_{max.}$ 280 nm

*Beispiel 283:*

*2-(4-Amino-3-brom-5-diäthylaminomethylbenzoyloxy)-2'-{2-[(2,6-dichlorphenyl)amino]-phenylacetoxy}diäthylsulfid*

Hergestellt aus 2-(4-Amino-3-brom-5-diäthylaminomethylbenzoyloxy)-2'-hydroxydiäthylsulfid und 2-[(2,6-Dichlorphenyl)amino]phenylessigsäure analog Beispiel 4. Öl.

IR-Spektrum (Methylenchlorid):
Ester-CO 1710 $cm^{-1}$
$NH_2$ 3450 $cm^{-1}$
UV-Spektrum (Äthanol): $\lambda_{max.}$ 283 nm

*Beispiel 284:*

*N-[2-(4-Amino-3-brom-5-diäthylaminomethylbenzoyloxy)äthyl]-N-[2-{2-[(2,6-dichlorphenyl)amino]phenylacetoxy}äthyl]äthylamin*

Hergestellt aus N-[2-(4-Amino-3-brom-5-diäthylaminomethylbenzoyloxy)äthyl]-N-(2-hydroxyäthyl)äthylamin und 2-[(2,6-Dichlorphenyl)-amino]phenylessigsäure analog Beispiel 4. Öl.

IR-Spektrum (Methylenchlorid):
Ester-CO 1710 $cm^{-1}$
N-Alkyl 2820 $cm^{-1}$
$NH_2$ 3450 $cm^{-1}$
UV-Spektrum (Äthanol): $\lambda_{max.}$ 283 nm

*Beispiel 285:*

*1-(4-Amino-3-brom-5-diäthylaminomethylbenzoyloxy)-6-[(+)-6-methoxy-α-methyl-2-naphthalinacetoxy]-n-hexan*

Hergestellt aus 6-(4-Amino-3-brom-5-diäthylaminomethylbenzoyloxy)-n-hexylchlorid und (+)-6-Methoxy-α-methyl-2-naphthalinessigsäurenatriumsalz analog Beispiel 8. Öl.

IR-Spektrum (Methylenchlorid):
O-$CH_3$ 2830 $cm^{-1}$
Ester-CO 1700 $cm^{-1}$, 1730 $cm^{-1}$
UV-Spektrum (Äthanol): $\lambda_{max.}$ 230 nm, 282 nm

*Beispiel 286:*

*1-[4-Amino-3-chlor-5-(N-äthylcyclohexylaminomethyl)benzoyloxy]-2-[1-(4-chlorbenzoyl)-5-methoxy-2-methyl-[1H]-indol-3-acetoxy]äthan*

Hergestellt aus 4-Amino-3-chlor-5-(N-äthylcyclohexylaminomethyl)benzoylchlorid und 1-[1-(4-Chlorbenzoyl)-5-methoxy-2-methyl-[1H]-indol-3-acetoxy]-2-hydroxyäthan analog Beispiel 7. Amorphes Hydrochlorid.

IR-Spektrum (Methylenchlorid):
Ester-CO 1710 und 1735 $cm^{-1}$

Amid-CO 1680 $cm^{-1}$
UV-Spektrum (Äthanol): $\lambda_{max.}$ 228 nm (Schulter), 276 nm

*Beispiel 287:*

*1-(4-Amino-3-chlor-5-diäthylaminomethylbenzoyloxy)-2-[1-(4-chlorbenzoyl)-5-methoxy-2-methyl-[1H]-indol-3-acetoxy]äthan*

Hergestellt aus 4-Amino-3-chlor-5-diäthylaminomethylbenzoylchlorid und 1-[1-(4-Chlorbenzoyl)-5-methoxy-2-methyl-[1H]-indol-3-acetoxy]-2-hydroxyäthan analog Beispiel 7. Amorphes Hydrochlorid.

IR-Spektrum (Methylenchlorid):
Ester-CO 1710 und 1725 $cm^{-1}$
Amid-CO 1680 $cm^{-1}$
UV-Spektrum (Äthanol): $\lambda_{max.}$ 228 nm (Schulter), 275 nm

*Beispiel 288:*

*1-[4-Amino-3-brom-5-(N-äthylcyclohexylaminomethyl)benzoyloxy]-2-methyl-2-n-propyl-3-[1-(4-chlorbenzoyl)-5-methoxy-2-methyl-[1H]-indol-3-acetoxy]-n-propan*

Hergestellt aus 1-[4-Amino-3-brom-5-(N-äthylcyclohexylaminomethyl)benzoyloxy]-2-methyl-2-n-propyl-3-hydroxy-n-propan und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3450 $cm^{-1}$
N-Alkyl 2940 $cm^{-1}$
$O-CH_3$ 2860 $cm^{-1}$
Ester-CO 1705 und 1735 $cm^{-1}$
Amid-CO 1685 $cm^{-1}$

*Beispiel 289:*

*1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]-2-phenyläthan*

Hergestellt aus 1-[4-Amino-3-brom-5-(N-äthylcyclohexylaminomethyl)benzoyloxy]-2-phenyl-2-hydroxyäthan und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3440 $cm^{-1}$
N-Alkyl 2940 $cm^{-1}$
$O-CH_3$ 2860 $cm^{-1}$
Ester-CO 1710 und 1745 $cm^{-1}$
UV-Spektrum (Äthanol): $\lambda_{max.}$ 230 nm, 295 nm

*Beispiel 290:*

*1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]-1,2-diphenyläthan*

Hergestellt aus 1-[4-Amino-3-brom-5-(N-äthylcyclohexylaminomethyl)benzoyloxy]-1,2-diphenyl-2-hydroxyäthan und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3450 $cm^{-1}$
N-Alkyl 2930 $cm^{-1}$
$O-CH_3$ 2860 $cm^{-1}$
Ester-CO 1710 und 1740 $cm^{-1}$
Amid-CO 1685 $cm^{-1}$
UV-Spektrum (Äthanol): $\lambda_{max.}$ 225 nm, 288 nm

*Beispiel 291:*

*1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]-1-phenyläthan*

Hergestellt aus 1-[4-Amino-3-brom-5-(N-äthylcyclohexylaminomethyl)benzoyloxy]-1-phenyl-2-hydroxyäthan und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3440 $cm^{-1}$
N-Alkyl 2915 $cm^{-1}$
$O-CH_3$ 2850 $cm^{-1}$
Ester-CO 1710 und 1740 $cm^{-1}$
Amid-CO 1685 $cm^{-1}$
UV-Spektrum (Äthanol): $\lambda_{max.}$ 230 nm, 288 nm

*Beispiel 292:*

*2-[4-Amino-3-brom-5-(N-äthylcyclohexylaminomethyl)benzoyloxymethyl]-6-[1-(4-chlorbenzoyl)-5-methoxy-2-methyl-[1H]-indol-3-acetoxymethyl]pyridin*

Hergestellt aus 2-[4-Amino-3-brom-5-(N-äthylcyclohexylaminomethyl)benzoyloxymethyl]-6-hydroxymethylpyridin und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3440 $cm^{-1}$
N-Alkyl 2930 $cm^{-1}$
$O-CH_3$ 2850 $cm^{-1}$
Ester-CO 1710 und 1735 $cm^{-1}$
Amid-CO 1685 $cm^{-1}$
UV-Spektrum (Äthanol): $\lambda_{max.}$ 230 nm, 285 nm

*Beispiel 293:*

*1-[4-Amino-3-brom-5-(N-äthylcyclohexylaminomethyl)benzoyloxymethyl]-4-[1-(4-chlorbenzoyl)-5-methoxy-2-methyl-[1H]-indol-3-acetoxymethyl]benzol*

Hergestellt aus 1-[4-Amino-3-brom-5-(N-äthylcyclohexylaminomethyl)benzoyloxymethyl]-4-hydroxymethylbenzol und 1-(4-Chlorbenzoyl)-5-methoxy-2-methyl-[1H]-indol-3-essigsäure analog Beispiel 4. Öl.

IR-Spektrum (Methylenchlorid):
$NH_2$ 3450 $cm^{-1}$
N-Alkyl 2940 $cm^{-1}$
$O-CH_3$ 2860 $cm^{-1}$
Ester-CO 1705 und 1740 $cm^{-1}$
Amid-CO 1685 $cm^{-1}$
UV-Spektrum (Äthanol): $\lambda_{max.}$ 280 nm

*Beispiel 294:*

*1-[4-Amino-3-brom-5-(N-äthylcyclohexyl-*

*aminomethyl)benzoyloxymethyl]-4-[1-(4-chlorbenzoyl)-5-methoxy-2-methyl-[1H]-indol-3-acetoxymethyl]cyclohexan*

Hergestellt aus 1-[4-Amino-3-brom-5-(N-äthylcyclohexylaminomethyl)benzoyloxymethyl]-4-hydroxymethylcyclohexan und 1-(4-Chlorbenzoyl)-5-methoxy-2-methyl-[1H]-indol-3-essigsäureimidazolid analog Beispiel 1. Öl.

IR-Spektrum (Methylenchlorid):

| | |
|---|---|
| $NH_2$ | 3450 $cm^{-1}$ |
| N-Alkyl | 2940 $cm^{-1}$ |
| $O-CH_3$ | 2860 $cm^{-1}$ |
| Ester-CO | 1705 und 1735 $cm^{-1}$ |
| Amid-CO | 1685 $cm^{-1}$ |

UV-Spektrum (Äthanol): $\lambda_{max.}$ 230 nm, 290 nm

*Beispiel 295:*

*1-[4-Amino-3-brom-5-(N-äthylcyclohexylaminomethyl)benzoyloxy]-2-{2-[(2,6-dichlorphenyl)amino]phenylacetoxy}-2-phenyläthan*

Hergestellt aus 2-[4-Amino-3-brom-5-(N-äthylcyclohexylaminomethyl)benzoyloxy]-1-phenyläthylchlorid und 2-[(2,6-Dichlorphenyl)-amino]phenylessigsäurenatriumsalz analog Beispiel 8. Öl.

IR-Spektrum (Methylenchlorid):

| | |
|---|---|
| $NH_2$ | 3450 $cm^{-1}$ |
| N-Alkyl | 2940 $cm^{-1}$ |
| Ester-CO | 1725 $cm^{-1}$ |

UV-Spektrum (Äthanol): $\lambda_{max.}$ 282 nm

*Beispiel 296:*

*1-[4-Amino-3-brom-5-(N-äthylcyclohexylaminomethyl)benzoyloxymethyl]-4-{2-[(2,6-dichlorphenyl)amino]phenylacetoxymethyl}benzol*

Hergestellt aus 1-[4-Amino-3-brom-5-(N-äthylcyclohexylaminomethyl)benzoyloxymethyl]-4-hydroxymethylbenzol und 2-[(2,6-Dichlorphenyl)amino]phenylessigsäurenatriumsalz analog Beispiel 8. Öl.

IR-Spektrum (Methylenchlorid):

| | |
|---|---|
| $NH_2$ | 3450 $cm^{-1}$ |
| N-Alkyl | 2940 $cm^{-1}$ |
| Ester-CO | 1710 $cm^{-1}$ |

UV-Spektrum (Äthanol): $\lambda_{max.}$ 283 nm

*Beispiel 297:*

*1-[4-Amino-3-brom-5-(N-äthylcyclohexylaminomethyl)benzoyloxymethyl]-4-{2-[(2,6-dichlorphenyl)amino]phenylacetoxymethyl}cyclohexan*

Hergestellt aus 1-[4-Amino-3-brom-5-(N-äthylcyclohexylaminomethyl)benzoyloxymethyl]-4-chlormethylcyclohexan und 2-[(2,6-Dichlorphenyl)amino]phenylessigsäurenatriumsalz analog Beispiel 8. Öl.

IR-Spektrum (Methylenchlorid):

| | |
|---|---|
| $NH_2$ | 3440 $cm^{-1}$ |
| N-Alkyl | 2430 $cm^{-1}$ |
| Ester-CO | 1705 und 1720 $cm^{-1}$ |

UV-Spektrum (Äthanol): $\lambda_{max.}$ 283 nm

*Beispiel A*

*Tabletten mit 100 mg 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid*

*Zusammensetzung:*

| | | |
|---|---|---|
| Wirksubstanzz | (1) | 100,0 mg |
| Milchzucker | (2) | 80,0 mg |
| Maisstärke | (3) | 34,0 mg |
| Polyvinylpyrrolidon | (4) | 4,0 mg |
| Magnesiumstearat | (5) | 2,0 mg |
| | | 220,0 mg |

*Herstellungsverfahren:*

1, 2, 3 und 4 werden gut gemischt und mit Äthanol befeuchtet. Die feuchte Masse wird durch ein Sieb mit 1,6-mm-Maschenweite geschlagen und bei einer Temperatur von 45°C im Trockenschrank getrocknet (exgeschützt). Nach dem Trocknen wird das Granulat durch dasselbe Sieb gerieben und 5 zugesetzt.

Tablettengewicht: 220 mg

Stempel: 9 mm Durchmesser biplan mit beidseitiger Facette und einseitiger Teilkerbe

*Beispiel B*

*Dragées mit 100 mg 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid*

1 Dragéekern enthält:

| | | |
|---|---|---|
| Wirksubstanz | (1) | 100,0 mg |
| Calciumphosphat sekundär | (2) | 110,0 mg |
| Maisstärke | (3) | 40,0 mg |
| Polyvinylpyrrolidon | (4) | 8,0 mg |
| Magnesiumstearat | (5) | 2,0 mg |
| | | 260,0 mg |

*Herstellungsverfahren:*

1, 2, 3 und 4 werden gemischt, mit Äthanol befeuchtet und durch ein Sieb mit 1,6-mm-Maschenweite geschlagen. Die Masse wird auf Horden ausgebreitet bei 45°C getrocknet (exgeschützt) und nochmals durch dasselbe Sieb gegeben, dann wird 5 zugemischt.

Kerngewicht: 260 mg

Stempel: 9 mm ⌀, bikonvex

In einem Dragéekessel werden die Kerne mit einer Schicht, die im wesentlichen aus Zucker und Talkum besteht, überzogen. Zuletzt wird mit Wachs geglänzt.

Dragéegewicht: 300 mg

*Beispiel C*

*Suppositorien mit 200 mg 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid*

1 Zäpfchen enthält:

| | |
|---|---|
| Wirksubstanz | 0,200 g |
| Hartfett (z.B. Witepsol H 19 und Witepsol W 45) | 1,500 g |
| | 1,700 g |

*Herstellungsverfahren:*

Das Hartfett wird geschmolzen. Bei 38° C wird die gemahlene Wirksubstanz in der Schmelze homogen dispergiert. Es wird auf 35° C abgekühlt und in schwach vorgekühlte Suppositorienformen ausgegossen.

Zäpfchengewicht: 1,7 g

*Beispiel D*

*Kapseln mit 200 mg 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid*

1 Kapsel enthält:

| | |
|---|---|
| Wirksubstanz | 200,0 mg |
| Maisstärke getr. | 60,0 mg |
| Milchzucker pulv. | 37,0 mg |
| Magnesiumstearat | 3,0 mg |
| | 300,0 mg |

*Herstellungsverfahren:*

Wirkstoff, Maisstärke und Milchzucker sowie zuvor gesiebtes Magnesiumstearat werden grob vermengt. Diese Mischung wird gesiebt und anschliessend im Pulvermischer homogen vermischt.

Hartgelatinekapsel Grösse 1

*Beispiel E*

*Salbe wasserfrei zu 1% 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthanhydrochlorid*

100 g enthalten:

| | |
|---|---|
| Wirkstoff | 1,0 g |
| Paraffinöl, dünnflüssig | 10,0 g |
| Cetylalkohol | 0,5 g |
| Wollwachs, wasserfrei | 5,0 g |
| Vaseline weiss | 83,5 g |
| | 100,0 g |

*Herstellungsverfahren:*

In einem Schmelzkessel werden Vaseline weiss, Wollfett, Cetylalkohol und Paraffinöl bei 70° C geschmolzen, durch ein 100 µ-Koliersieb in den Ansatzkessel (z.B. Moltomat) gegeben und auf ca. 60° C abgekühlt.

In diese noch flüssige Fettschmelze wird die Wirksubstanz mikronisiert, portionsweise mit dem Homogenisator (Stufe I) eingearbeitet, 10 min weitergerührt und bei Rühren (Stufe II) auf ca. 35°C abgekühlt. Dann lässt man die Salbe ohne Rühren auf Raumtemperatur abkühlen.

*Beispiel F*

*Tabletten mit 100 mg 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid*

Zusammensetzung:

| | | |
|---|---|---|
| Wirksubstanz | (1) | 100,0 mg |
| Milchzucker | (2) | 80,0 mg |
| Maisstärke | (3) | 34,0 mg |
| Polyvinylpyrrolidon | (4) | 4,0 mg |
| Magnesiumstearat | (5) | 2,0 mg |
| | | 220,0 mg |

*Herstellungsverfahren:* siehe analog Beispiel A.

*Beispiel G*

*Dragées mit 100 mg 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid*

1 Drageékern enthält:

| | | |
|---|---|---|
| Wirksubstanz | (1) | 100,0 mg |
| Calciumphosphat sekundär | (2) | 110,0 mg |
| Maisstärke | (3) | 40,0 mg |
| Polyvinylpyrrolidon | (4) | 8,0 mg |
| Magnesiumstearat | (5) | 2,0 mg |
| | | 260,0 mg |

*Herstellungsverfahren:* siehe Analog Beispiel B.

*Beispiel H*

*Suppositorien mit 200 mg 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid*

1 Zäpfchen enthält:

| | |
|---|---|
| Wirksubstanz | 0,200 g |
| Hartfett (z.B. Witepsol H 19 und Witepsol W 45) | 1,500 g |
| | 1,700 g |

*Herstellungverfahren:* siehe analog Beispiel C.

*Beispiel I*

*Kapseln mit 200 mg 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid*

1 Kapsel enthält:

| | |
|---|---|
| Wirksubstanz | 200,0 mg |
| Maisstärke getr. | 60,0 mg |
| Milchzucker pulv. | 37,0 mg |
| Magnesiumstearat | 3,0 mg |
| | 300,0 mg |

*Herstellungsverfahren:* siehe analog Beispiel D.

*Beispiel J*

*Salbe wasserfrei zu 1% 1-[4-Amino-3-brom-5-(N-äthylcyclohexylaminomethyl)benzoyloxy]-5-{2-[(2,6-dichlorphenyl)amino]phenylacetoxy}-n-pentanhydrochlorid*

100 g enthalten:

| | |
|---|---|
| Wirkstoff | 1,0 g |
| Paraffinöl, dünnflüssig | 10,0 g |
| Cetylalkohol | 0,5 g |
| Wollwachs, wasserfrei | 5,0 g |
| Vaseline weiss | 83,5 g |
| | 100,0 g |

*Herstellungsverfahren:* siehe analog Beispiel E.

**Patentansprüche für die Vertragsstaaten:** BE CH LI DE FR GB IT LU NL SE

1. Neue Benzoylderivate der allgemeinen Formel

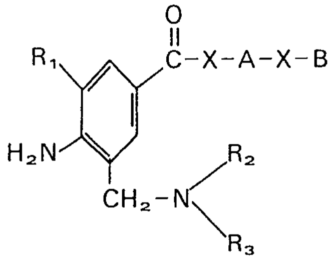

(I)

in der

X ein Sauerstoffatom oder die Iminogruppe, wobei die beiden Reste X gleich oder verschieden sein können,

$R_1$ ein Chlor- oder Bromatom,

$R_2$ und $R_3$, die gleich oder verschieden sein können, Wasserstoffatome, geradkettige oder verzweigte Alkylgruppen mit 1 bis 6 Kohlenstoffatomen, welche durch eine Phenylgruppe oder eine Dialkylaminogruppe mit 1 bis 3 Kohlenstoffatomen in jedem Alkylteil substituiert sein können, Pyridyl- oder Cycloalkylgruppen mit 5 bis 7 Kohlenstoffatomen oder $R_2$ und $R_3$ zusammen mit dem dazwischenliegenden Stickstoffatom eine Pyrrolidino-, Piperidino-, Hexamethylenimino-, Morpholino-, N-Arylpiperazino- oder N-Alkylpiperazinogruppe, wobei die Alkylgruppe 1 bis 3 Kohlenstoffatome enthalten kann,

A eine Cycloalkylengruppe mit 5 bis 7 Kohlenstoffatomen oder eine Alkylengruppe mit 2 bis 10 Kohlenstoffatomen, die durch eine oder zwei Alkylgruppen mit jeweils 1 bis 3 Kohlenstoffatomen, durch eine oder zwei Carbalkoxygruppen mit insgesamt je 2 bis 4 Kohlenstoffatomen, durch eine oder zwei Phenylgruppen, durch 1 bis 4 Hydroxylgruppen, durch eine Halogenmethyl-, Hydroxymethyl-, Alkanoyloxy- oder Alkanoyloxymethylgruppe mit jeweils 1 bis 8 Kohlenstoffatomen im Alkanoylteil oder durch eine Gruppe der Formel

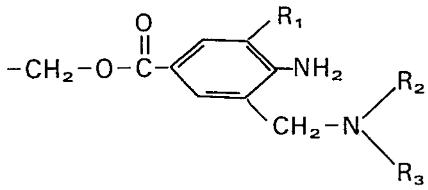

wobei $R_1$, $R_2$ und $R_3$ wie eingangs definiert sind, substituiert oder durch ein Sauerstoff- oder Schwefelatom, durch eine Sulfinyl-, Sulfonyl-, Phenylen-, Cyclohexylen-, Pyridindiyl-, Piperazindiyl- oder Iminogruppe unterbrochen sein kann, wobei die Iminogruppe durch eine Alkylgruppe mit 1 bis 6 Kohlenstoffatomen, durch eine Phenyl- oder Phenylalkylgruppe mit 1 bis 3 Kohlenstoffatomen im Alkylteil substituiert sein kann, und

B die 2-Acetoxybenzoyl-, 2-[(2,6-Dichlorphenyl)amino]phenylacetyl-, 1-(4-Chlorbenzoyl)-5-methoxy-2-methyl-[1H]-indol-3-acetyl-, 3-Benzoyl-α-methylphenylacetyl-, α-Methyl-4-(2-methylpropyl)phenylacetyl-, 2-Fluor-α-methyl-[1,1'-biphenyl]-4-acetyl- und (+)-6-Methoxy-α-methyl-2-naphthalinacetylgruppe bedeuten, und deren physiologisch verträgliche Säureadditionssalze mit anorganischen oder organischen Säuren.

2. Neue Benzoylderivate der allgemeinen Formel I gemäss Anspruch 1, in der

X ein Sauerstoffatom oder die Iminogruppe, wobei die beiden Reste X gleich oder verschieden sein können,

$R_1$ ein Chlor- oder Bromatom, und

$R_2$ und $R_3$, die gleich oder verschieden sein können, Wasserstoffatome, Alkylgruppen mit 1 bis 4 Kohlenstoffatomen, Cycloalkylgruppen mit 5 bis 7 Kohlenstoffatomen, Benzyl- oder 2-Diäthylaminoäthylgruppen oder $R_2$ und $R_3$ zusammen mit dem dazwischenliegenden Stickstoffatom die Pyrrolidino-, Piperidino-, Hexamethylenimino-, Morpholino-, N-Methylpiperazino- oder N-Phenylpiperazinogruppe,

A eine geradkettige Alkylengruppe mit 2 bis 10 Kohlenstoffatomen, eine Äthylengruppe, die durch 1 oder 2 Methylgruppen, durch 1 oder 2 Alkoxycarbonylgruppen mit insgesamt je 2 oder 3 Kohlenstoffatomen, durch eine oder zwei Phenylgruppen, durch eine Hydroxy-, Chlormethyl-, Hydroxymethyl-, 4-Amino-3-brom-5-(N-äthylcyclohexylaminomethyl)benzoyloxymethyl- oder Alkanoyloxymethylgruppe mit 1 bis 18 Kohlenstoffatomen im Alkanoylteil substituiert ist, eine Propylengruppe, die in 2-Stellung durch eine Hydroxygruppe oder eine Alkanoyloxygruppe mit 1 bis 18 Kohlenstoffatomen oder durch zwei Alkylgruppen mit jeweils 1 bis 3 Kohlenstoffatomen substituiert ist, eine durch 4 Hydroxygruppen substituierte n-Hexylengruppe, eine Cyclohexylengruppe oder eine geradkettige Alkylengruppe mit 4 bis 6 Kohlenstoffatomen, welche zwischen den C-Atomen 2 und 3 oder 3 und 4 durch ein Sauerstoff- oder Schwefelatom, durch eine Sulfinyl-, Sulfonyl-, Imino-, Phenylimino-, Benzylimino-, Piperazindiyl- oder eine Alkyliminogruppe mit 1 bis 4 Kohlenstoffatomen unterbrochen ist, eine 1,4-Cyclohexandimethylen-, p-Xylylen- oder 2,6-Pyridindimethylengruppe, und

B die 2-[(2,6-Dichlorphenyl)amino]phenylacetyl-, 1-(4-Chlorbenzoyl)-5-methoxy-2-methyl-[1H]-indol-3-acetyl-, 3-Benzoyl-α-methyl phenylacetyl-, α-Methyl-4-(2-methylpropyl)-phenylacetyl-, 2-Fluor-α-methyl-[1,1'-biphenyl]-4-acetyl- und (+)-6-Methoxy-α-methyl-2-naphthalinacetylgruppe bedeuten, und deren physiologisch verträgliche Säureadditionssalze mit anorganischen oder organischen Säuren.

3. Neue Benzoylderivate der allgemeinen Formel I gemäss Anspruch 1, in der

X je ein Sauerstoffatom,

$R_1$ ein Bromatom,

$R_2$ die Methyl- oder Äthylgruppe,

$R_3$ die Äthyl- oder Cyclohexylgruppe oder $R_2$ und $R_3$ zusammen mit dem Stickstoffatom die Hexamethyleniminogruppe,

A eine geradkettige Alkylengruppe mit 2 bis 6 Kohlenstoffatomen oder eine geradkettige Alkylengruppe mit 4 bis 6 Kohlenstoffatomen, welche zwischen den C-Atomen 2 und 3 oder 3 und 4 durch ein Sauerstoff- oder Schwefelatom, durch eine Äthylimino- oder Propyliminogruppe unterbrochen ist, und

B die 1-(4-Chlorbenzoyl)-5-methoxy-2-methyl-[1H]-indol-3-acetyl-, 2-[(2,6-Dichlorphenyl)amino]phenylacetyl-, 3-Benzoyl-α-methylphenylacetyl-, (+)-6-Methoxy-α-methyl-2-naphthalinacetyl-, α-Methyl-4-(2-methylpropyl)phenylacetyl- oder 2-Fluor-α-methyl-[1,1'-biphenyl]-4-acetylgruppe bedeuten, und deren physiologisch verträgliche Salze mit anorganischen oder organischen Säuren.

4. Neue Benzoylderivate der allgemeinen Formel I gemäss Anspruch 1, in der

$R_1$, $R_2$, $R_3$ und A wie im Anspruch 3 definiert sind, und

B die 1-(4-Chlorbenzoyl)-5-methoxy-2-methyl-[1H]-indol-3-acetyl- oder 2-[(2,6-Dichlorphenyl)amino]phenylacetylgruppe bedeutet, und deren physiologisch verträgliche Salze mit anorganischen oder organischen Säuren.

5. 1-[4-Amino-3-brom-5-(N-äthylcyclohexylaminomethyl)benzoyloxy]-2-[1-(4-chlorbenzoyl)-5-methoxy-2-methyl-[1H]-indol-3-acetoxy]äthan und dessen physiologisch verträgliche Säureadditionssalze.

6. 1-[4-Amino-3-brom-5-(N-äthylcyclohexylaminomethyl)benzoyloxy]-5-{2-[(2,6-dichlorphenyl)amino]phenylacetoxy}-n-pentan und dessen physiologisch verträgliche Säureadditionssalze.

7. 1-(4-Amino-3-brom-5-diäthylaminomethylbenzoyloxy)-2-[1-(4-chlorbenzoyl)-5-methoxy-2-methyl-[1H]-indol-3-acetoxy]äthan und dessen physiologisch verträgliche Säureadditionssalze.

8. Arzneimittel enthaltend eine Verbindung gemäss den Ansprüchen 1 bis 7 neben einem oder mehreren inerten Trägerstoffen und/oder Verdünnungsmitteln.

9. Arzneimittel gemäss Anspruch 8 zur Bekämpfung von entzündlichen Prozessen.

10. Verfahren zur Herstellung von neuen Benzoylderivaten gemäss den Ansprüchen 1 bis 7 und von deren physiologisch verträglichen Säureadditionssalzen mit anorganischen oder organischen Säuren, dadurch gekennzeichnet, dass eine Carbonsäure der allgemeinen Formel

$$B - OH \qquad (II)$$

ihre Salze oder reaktionsfähige Derivate mit einer Verbindung der allgemeinen Formel

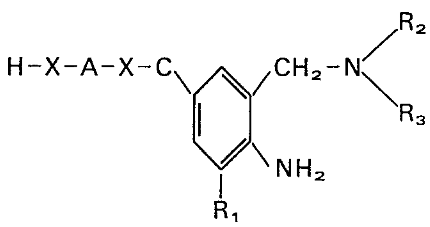

(III)

ihren Alkalisalzen oder reaktionsfähigen Derivaten oder eine Carbonsäure der allgemeinen Formel

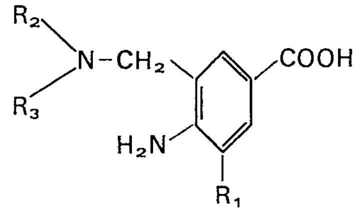

(IV)

ihre Salze oder reaktionsfähige Derivate mit einer Verbindung der allgemeinen Formel

$$H - X - A - X - B \qquad (V)$$

ihren Alkalisalzen oder reaktionsfähigen Derivaten, in denen

$R_1$ bis $R_3$, A, B und X wie eingangs definiert sind und die Gruppe HX zusammen mit den benachbarten α- und β-ständigen C-Atomen des Restes A auch eine Oxiranylgruppe bedeuten kann, umgesetzt wird, und

gewünschtenfalls anschliessend eine erhaltene Verbindung der allgemeinen Formel I, in der A eine Hydroxylgruppe enthält, mittels Acylierung in die entsprechende Acyloxyverbindung der allgemeinen Formel I übergeführt wird oder eine erhaltene Verbindung der allgemeinen Formel I, in der A eine durch ein Schwefelatom unterbrochene Alkylengruppe darstellt, mittels Oxidation in die entsprechende Sulfinyl- oder Sulfonylverbindung übergeführt wird, oder

eine erhaltene Verbindung der allgemeinen Formel I, in der A eine durch eine Sulfinylgruppe unterbrochene Alkylengruppe darstellt, mittels Oxidation in die entsprechende Sulfonylverbindung übergeführt wird, oder

eine erhaltene Verbindung der allgemeinen Formel I in ihre physiologisch verträglichen Säureadditionssalze mit 1 bis 3 Äquivalenten der betreffenden anorganischen oder organischen Säure übergeführt wird.

**Patentansprüche für den Vertragsstaat:** AT

1. Verfahren zur Herstellung von neuen Benzoylderivaten der allgemeinen Formel

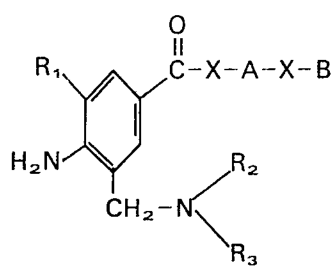

(I)

in der

X ein Sauerstoffatom oder die Iminogruppe, wobei die beiden Reste X gleich oder verschieden sein können,

$R_1$ ein Chlor- oder Bromatom,

$R_2$ und $R_3$, die gleich oder verschieden sein können, Wasserstoffatome, geradkettige oder verzweigte Alkylgruppen mit 1 bis 6 Kohlenstoffatomen, welche durch eine Phenylgruppe oder eine Dialkylaminogruppe mit 1 bis 3 Kohlenstoffatomen in jedem Alkylteil substituiert sein können, Pyridyl- oder Cycloalkylgruppen mit 5 bis 7 Kohlenstoffatomen oder $R_2$ und $R_3$ zusammen mit dem dazwischenliegenden Stickstoffatom eine Pyrrolidino-, Piperidino-, Hexamethylenimino-, Morpholino-, N-Arylpiperazino- oder N-Alkylpiperazinogruppe, wobei die Alkylgruppe 1 bis 3 Kohlenstoffatome enthalten kann,

A eine Cycloalkylengruppe mit 5 bis 7 Kohlenstoffatomen oder eine Alkylengruppe mit 2 bis 10 Kohlenstoffatomen, die durch eine oder zwei Alkylgruppen mit jeweils 1 bis 3 Kohlenstoffatomen, durch eine oder zwei Carbalkoxygruppen mit insgesamt je 2 bis 4 Kohlenstoffatomen, durch eine oder zwei Phenylgruppen, durch 1 bis 4 Hydroxylgruppen, durch eine Halogenmethyl-, Hydroxymethyl-, Alkanoyloxy- oder Alkanoyloxymethylgruppe mit jeweils 1 bis 18 Kohlenstoffatomen im Alkanoylteil oder durch eine Gruppe der Formel

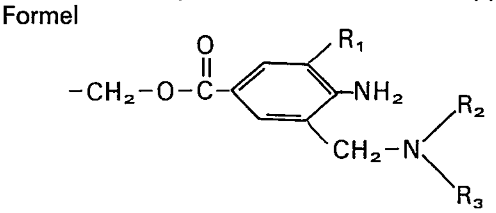

wobei $R_1$, $R_2$ und $R_3$ wie eingangs definiert sind, substituiert oder durch ein Sauerstoff- oder Schwefelatom, durch eine Sulfinyl-, Sulfonyl-, Phenylen-, Cyclohexylen-, Pyridindiyl-, Piperazindiyl- oder Iminogruppe unterbrochen sein kann, wobei die Iminogruppe durch eine Alkylgruppe mit 1 bis 6 Kohlenstoffatomen, durch eine Phenyl- oder Phenylalkylgruppe mit 1 bis 3 Kohlenstoffatomen im Alkylteil substituiert sein kann, und

B die 2-Acetoxybenzoyl-, 2-[(2,6-Dichlorphenyl)amino]phenylacetyl-, 1-(4-Chlorbenzoyl)-5-methoxy-2-methyl-[1H]-indol-3-acetyl-, 3-Benzoyl-α-methylphenylacetyl-, α-Methyl-4-(2-methylpropyl)phenylacetyl-, 2-Fluor-α-methyl-[1,1'-biphenyl]-4-acetyl- und (+)-6-Methoxy-α-methyl-2-naphthalinacetylgruppe bedeuten, und von deren physiologisch verträglichen Säureadditionssalzen mit anorganischen oder organischen Säuren, dadurch gekennzeichnet, dass eine Carbonsäure der allgemeinen Formel

$$B - OH \quad (II)$$

ihre Salze oder reaktionsfähigen Derivate mit einer Verbindung der allgemeinen Formel

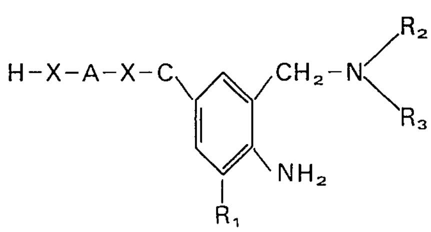

(III)

ihren Alkalisalzen oder reaktionsfähigen Derivaten oder eine Carbonsäure der allgemeinen Formel

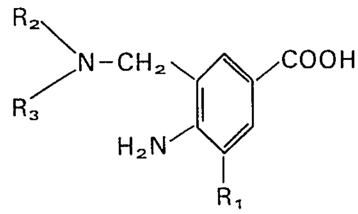

(IV)

ihre Salze oder reaktionsfähigen Derivate mit einer Verbindung der allgemeinen Formel

$$H - X - A - X - B \quad (V)$$

ihren Alkalisalzen oder reaktionsfähigen Derivaten, in denen

$R_1$ bis $R_3$, A, B und X eingangs definiert sind und die Gruppe HX zusammen mit den benachbarten α- und β-ständigen C-Atomen des Restes A auch eine Oxiranylgruppe bedeuten kann, umgesetzt wird, und

gewünschtenfalls anschliessend eine erhaltene Verbindung der allgemeinen Formel I, in der A eine Hydroxylgruppe enthält, mittels Acylierung in die entsprechende Acyloxyverbindung der allgemeinen Formel I übergeführt wird oder eine erhaltene Verbindung der allgemeinen Formel I, in der A eine durch ein Schwefelatom unterbrochene Alkylengruppe darstellt, mittels Oxidation in die entsprechende Sulfinyl- oder Sulfonylverbindung übergeführt wird, oder

eine erhaltene Verbindung der allgemeinen Formel I, in der A eine durch eine Sulfinylgruppe unterbrochene Alkylengruppe darstellt, mittels Oxidation in die entsprechende Sulfonylverbindung übergeführt wird oder eine erhaltene Verbindung der allgemeinen Formel I in ihre physiologisch verträglichen Säureadditionssalze mit 1 bis 3 Äquivalenten der betreffenden anorganischen oder organischen Säure übergeführt wird.

2. Verfahren gemäss Anspruch 1 zur Herstellung von neuen Benzoylderivaten der allgemeinen Formel

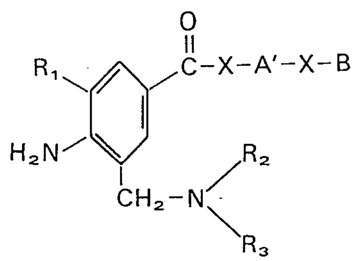

(Ia)

in der

$R_1$ bis $R_3$, B und X wie im Anspruch 1 definiert sind, und

A' eine Cycloalkylengruppe mit 5 bis 7 Kohlenstoffatomen oder eine alkylengruppe mit 2 bis 10 Kohlenstoffatomen, die durch eine oder zwei Alkylgruppen mit 1 bis 3 Kohlenstoffatomen, durch eine oder zwei Carbalkoxygruppen mit insgesamt je 2 bis 4 Kohlenstoffatomen, durch 1 bis 4 Hydroxylgruppen, durch eine Halogenmethyl-, Hydroxymethyl-, Alkanoyloxy- oder Alkanoyloxymethylgruppe mit jeweils 1 bis 18 Kohlenstoffatomen im Alkanoylteil oder durch eine Gruppe der Formel

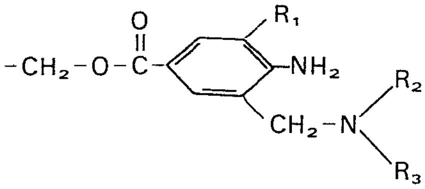

wobei $R_1$, $R_2$ und $R_3$ wie eingangs definiert sind, substituiert oder durch ein Sauerstoff- oder Schwefelatom, durch eine Sulfinyl-, Sulfonyl-, Piperazindiyl- oder Iminogruppe unterbrochen sein kann, wobei die Iminogruppe durch eine Alkylgruppe mit 1 bis 6 Kohlenstoffatomen, durch eine Phenyl- oder Phenylalkylgruppe mit 1 bis 3 Kohlenstoffatomen im alkylteil substituiert sein kann, bedeutet und von deren physiologisch verträglichen Säureadditionssalzen mit anorganischen oder organischen Säuren, dadurch gekennzeichnet, dass eine Carbonsäure der allgemeinen Formel

$$B - OH \qquad (II)$$

ihre Salze oder reaktionsfähigen Derivate mit einer Verbindung der allgemeinen Formel

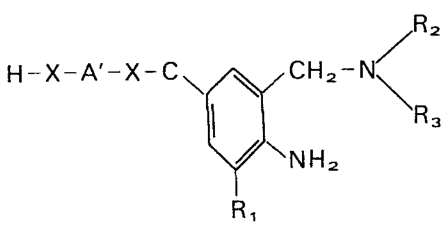

(IIIa)

ihren Alkalisalzen oder reaktionsfähigen Derivaten oder eine Carbonsäure der allgemeinen Formel

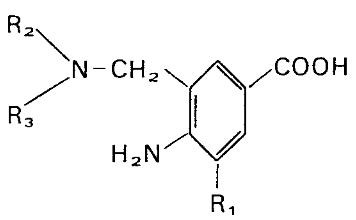

(IV)

ihre Salze oder reaktionsfähigen Derivate mit einer Verbindung der allgemeinen Formel

$$H - X - A' - X - B \qquad (Va)$$

ihren Alkalisalzen oder reaktionsfähigen Derivaten, in denen

$R_1$ bis $R_3$, B und X wie im Anspruch 1 eingangs definiert sind, und

A' wie oben definiert ist, umgesetzt wird, und

gewünschtenfalls anschliessend eine erhaltene Verbindung der allgemeinen Formel Ia, in der A' eine Hydroxygruppe enthält, mittels Acylierung in die entsprechende Acyloxyverbindung der allgemeinen Formel Ia übergeführt wird oder eine erhaltene Verbindung der allgemeinen Formel Ia, in der A' eine durch ein Schwefelatom unterbrochene Alkylengruppe darstellt, mittels Oxidation in die entsprechende Sulfinyl- oder Sulfonylverbindung übergeführt wird, oder

eine erhaltene Verbindung der allgemeinen Formal Ia, in der A' eine durch eine Sulfinylgruppe unterbrochene Alkylengruppe darstellt, mittels Oxidation in die entsprechende Sulfonylverbindung übergeführt wird oder eine erhaltene Verbindung der allgemeinen Formel Ia in ihre physiologisch verträglichen Säureadditionssalze mit 1 bis 3 Äquivalenten der betreffenden anorganischen oder organischen Säure übergeführt wird.

3. Verfahren gemäss den Ansprüchen 1 und 2, dadurch gekennzeichnet, dass die Umsetzung einer Verbindung der allgemeinen Formel III oder V oder ihrer Alkalisalze mit einer Carbonsäure der allgemeinen Formel II oder IV oder ihren funktionellen Derivaten gegebenenfalls in Gegenwart eines säureaktivierenden und/oder wasserentziehenden Mittels bei Temperaturen zwischen −25 und 250°C durchgeführt wird.

4. Verfahren gemäss den Ansprüchen 1 und 2, dadurch gekennzeichnet, dass die Umsetzung einer Carbonsäure der allgemeinen Formel II oder IV oder ihrer Salze mit einem reaktionsfähigen Derivat einer Verbindung der allgemeinen Formel III (oder V) bei Temperaturen zwischen −25 und 250°C durchgeführt wird.

5. Verfahren gemäss Anspruch 4, dadurch gekennzeichnet, dass als funktionelles Derivat einer Carbonsäure der allgemeinen Formel II oder IV deren Ester, Imidazolid, Säurehalogenid, deren Anhydrid oder deren gemischtes Anhydrid, deren Acyloxytriphenylphosphoniumsalz, deren N-Acyloxyimid oder auch deren Isatosäureanhydrid, wenn die Aminogruppe in 2-Stellung einer Carbonsäure der allgemeinen Formel IV steht, verwendet wird.

6. Verfahren gemäss Anspruch 5, dadurch gekennzeichnet, dass als reaktionsfähiges Derivat einer Verbindung der allgemeinen Formel III oder V deren Phosphazoderivat, falls X die Iminogruppe oder ein Sauerstoffatom darstellt, deren Epoxid, deren Halogenid oder deren Ester mit einer Sulfon- oder Carbonsäure verwendet wird.

7. Verfahren gemäss Anspruch 4, dadurch gekennzeichnet, dass als Salz einer Carbonsäure der allgemeinen Formel II oder IV das Alkali-, Erdalkali- oder Silbersalz verwendet wird.

8. Verfahren gemäss den Ansprüchen 1 bis 7, dadurch gekennzeichnet, dass die Umsetzung in einem Lösungsmittel und bei temperaturen zwischen −10°C und der Siedetemperatur des verwendeten Lösungsmittels durchgeführt wird.

9. Verfahren gemäss den Ansprüchen 1 bis 8, dadurch gekennzeichnet, dass die Umsetzung in Gegenwart einer anorganischen oder tertiären organischen Base durchgeführt wird.

10. Verfahren gemäss den Ansprüchen 1 bis 9, dadurch gekennzeichnet, dass die Umsetzung in Gegenwart eines Reaktionsbeschleunigers wie Natriumhydrid, 4-Dimethylaminopyridin, Natriumjodid oder Kaliumjodid oder in Gegenwart eines Phasentransferkatalysators wie einem Kronenäther durchgeführt wird.

**Claims for the Contracting States:** BE CH LI DE FR GB IT LU NL SE

1. New benzoyl derivatives of general formula:

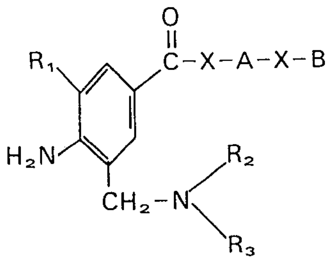

(I)

wherein:

X represents an oxygen atom or the imino group, the two groups X being the same or different,

$R_1$ represents a chlorine or bromine atom,

$R_2$ and $R_3$, which may be the same or different, represent hydrogen atoms, straight-chained or branched alkyl groups with 1 to 6 carbon atoms which may be substituted by a phenyl group or a dialkylamino group with 1 to 3 carbon atoms in each alkyl moiety, or they may represent pyridyl or cycloalkyl groups with 5 to 7 carbon atoms, or $R_2$ and $R_3$ together with the nitrogen atom between them may represent a pyrrolidino, piperidino, hexamethyleneimino, morpholino, N-arylpiperazino or N-alkylpiperazino group, wherein the alkyl group may contain 1 to 3 carbon atoms,

A represents a cycloalkylene group with 5 to 7 carbon atoms or an alkylene group with 2 to 10 carbon atoms which may be substituted by one or two alkyl groups each having 1 to 3 carbon atoms, or by one or two carbalkoxy groups each having a total of 2 to 4 carbon atoms, or by one or two phenyl groups, by 1 to 4 hydroxyl groups, by a halomethyl, hydroxymethyl, alkanoyloxy or alkanoyloxymethyl group each with 1 to 18 carbon atoms in the alkanoyl moiety or by a group of formula:

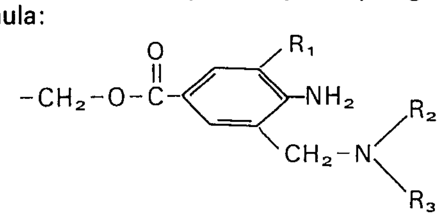

wherein $R_1$, $R_2$ and $R_3$ are as hereinbefore defined, or the alkylene group may be interrupted by an oxygen or sulphur atom, by a sulfinyl, sulfonyl, phenylene, cyclohexylene, pyridindiyl, piperazindiyl or imino group, whilst the imino group may be substituted by an alkyl group with 1 to 6 carbon atoms, or by a phenyl or phenylalkyl group with 1 to 3 carbon atoms in the alkyl moiety, and

B represent the 2-acetoxybenzoyl, 2-[(2,6-dichlorophenyl)amino]phenylacetyl, 1-(4-chlorobenzoyl)-5-methoxy-2-methyl-1H-indol-3-acetyl, 3-benzoyl-α-methylphenylacetyl, α-methyl-4-(2-methylpropyl)phenylacetyl, 2-fluoro-α-methyl-[1,1'-biphenyl]-4-acetyl, and (+)-6-methoxy-α-methyl-2-naphthaleneacetyl group, and the physiologically acceptable acid addition salts thereof with inorganic or organic acids.

2. New benzoyl derivatives of general formula I according to claim 1, wherein:

X represents an oxygen atom or the imino group, the two groups X being the same or different,

$R_1$ represents a chlorine or bromine atom,

$R_2$ and $R_3$, which may be the same or different, represent hydrogen atoms, alkyl groups with 1 to 4 carbon atoms, cycloalkyl groups with 5 to 7 carbon atoms, benzyl or 2-diethylaminoethyl groups, or $R_2$ and $R_3$ together with the nitrogen atom between them represent the pyrrolidino, piperidino, hexamethyleneimino, morpholino, N-methylpiperazino or N-phenylpiperazino group,

A represents a straight-chained alkylene group with 2 to 10 carbon atoms, an ethylene group which is substituted by one or two methyl groups, by 1 or 2 alkoxycarbonyl groups each with a total of 2 or 3 carbon atoms, by one or two phenyl groups, by a hydroxy, chloromethyl, hydroxymethyl, 4-amino-3-bromo-5-(N-ethylcyclohexylaminomethyl)benzoyloxymethyl or alkanoyloxymethyl group with 1 to 18 carbon atoms in the alkanoyl moiety, a propylene group which is substituted in the 2-position by a hydroxy group or an alkanoyloxy group with 1 to 18 carbon atoms or by two alkyl groups each with 1 to 3 carbon atoms, an n-hexylene group substituted by four hydroxy groups, a cyclohexylene group or a straight-chained alkylene group with 4 to 6 carbon atoms, which is interrupted between carbon atoms 2 and 3 or 3 and 4 by an oxygen or sulphur atom, or by a sulfinyl, sulfonyl, imino, phenylimino, benzylimino, piperazindiyl or an alkylimino group with 1 to 4 carbon atoms, or a 1,4-cyclohexanedimethylene, p-xylylene or 2,6-pyridinedimethylene group, and

B represents the 2-[(2,6-dichlorophenyl)-amino]phenylacetyl, 1-(4-chlorobenzoyl)-5-methoxy-2-methyl-1H-indol-3-acetyl, 3-benzoyl-α-methylphenylacetyl, α-methyl-4-(2-methylpropyl)phenylacetyl, 2-fluoro-α-methyl-[1,1'-biphenyl]-4-acetyl and (+)-6-methoxy-α-methyl-2-naphthaleneacetyl group, and the physiologically acceptable acid addition salt thereof with inorganic or organic acids.

3. New benzoyl derivatives of general formula I according to claim 1, wherein:

X in each case represents an oxygen atom,

$R_1$ represents a bromine atom,

$R_2$ represents the methyl or ethyl group,

$R_3$ represents the ethyl or cyclohexyl group, or $R_2$ and $R_3$ together with the nitrogen atom represent the hexamethyleneimino group,

A represents a straight-chained alkylene group with 2 to 6 carbon atoms or a straight-chained alkylene group with 4 to 6 carbon atoms which is interrupted between carbon atoms 2 and 3 or 3 and 4 by an oxygen or sulphur atom or by an ethylimino or propylimino group, and

B represents the 1-(4-chlorobenzoyl)-5-methoxy-2-methyl-1H-indol-3-acetyl, 2-[(2,6-dichlorophenyl)amino]phenylacetyl, 3-benzoyl-α-methylphenylacetyl, (+)-6-methoxy-α-methyl-2-naphthaleneacetyl, α-methyl-4-(2-methylpropyl)phenylacetyl or 2-fluoro-α-methyl-[1,1'-biphenyl]-4-acetyl group, and the physiologically acceptable salts thereof with inorganic or organic acids.

4. New benzoyl derivatives of general formula I according to claim 1, wherein:

$R_1$, $R_2$, $R_3$ and A are defined as in claim 3, and

B represents the 1-(4-chlorobenzoyl)-5-methoxy-2-methyl-1H-indol-3-acetyl or 2-[(2,6-dichlorophenyl)amino]phenylacetyl group, and the physiologically acceptable salts thereof with inorganic or organic acids.

5. 1-[4-Amino-3-bromo-5-(N-ethylcyclohexylaminomethyl)benzoyloxy]-2-[1-(4-chlorobenzoyl)-5-methoxy-2-methyl-1H-indol-3-acetoxy]ethane and the physiologically acceptable acid addition salts thereof.

6. 1-[4-Amino-3-bromo-5-(N-ethylcyclohexylaminomethyl)benzoyloxy]-5-{2-[(2,6-dichlorophenyl)amino]phenylacetoxy}-n-pentan and the physiologically acceptable acid addition salts thereof.

7. 1-(4-Amino-3-bromo-5-diethylaminomethylbenzoyloxy)-2-[1-(4-chlorobenzoyl)-5-methoxy-2-methyl-1H-indol-3-acetoxy]ethane and the physiologically acceptable acid addition salts thereof.

8. Pharmaceutical compositions containing a compound as claimed in claims 1 to 7 together with one or more inert carriers and/or diluents.

9. Pharmaceutical compositions as claimed in claim 8 for controlling inflammatory processes.

10. Process for the preparation of new benzoyl derivatives as claimed in claims 1 to 7 and of the physiologically acceptable acid addition salts thereof with inorganic or organic acids, characterised in that a carboxylic acid of general formula

$$B - OH \qquad (II)$$

or a salt or reactive derivative thereof, is reacted with a compound of general formula:

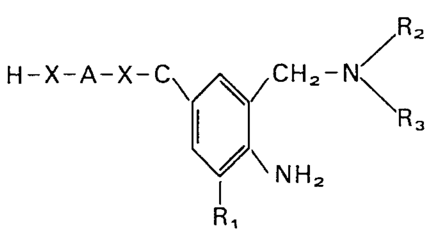

(III)

or an alkali metal salt or reactive derivative thereof, or a carboxylic acid of general formula:

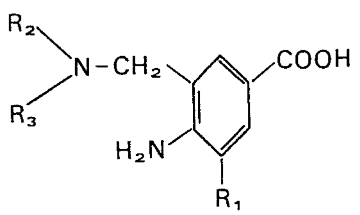

(IV)

or a salt or reactive derivative thereof is reacted with a compound of general formula:

$$H - X - A - X - B \qquad (V)$$

or an alkali metal salt or reactive derivative thereof wherein:

$R_1$ to $R_3$, A, B and X are as hereinbefore defined and the group HX together with the adjacent carbon atoms at the α- and β-positions of the group A may also represent an oxiranyl group,

and subsequently, if desired, a compound of general formula I obtained wherein A contains a hydroxyl group is converted by acylation into the corresponding acyloxy compound of general formula I or a compound of general formula I obtained wherein A represents an alkylene group interrupted by a sulphur atom is converted by oxidation into the corresponding sulfinyl or sulfonyl compound, or

a compound of general formula I obtained wherein A represents an alkylene group interrupted by a sulfinyl group is converted by oxidation into the corresponding sulfonyl compound, or

a compound of general formula I obtained is converted into the physiologically acceptable acid addition salts thereof with 1 to 3 equivalents of the appropriate inorganic or organic acid.

**Claims for the Contracting State:** AT

1. Process for the preparation of new benzoyl derivatives of general formula:

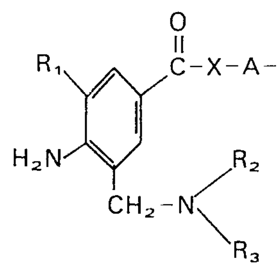

(I)

wherein:

X represents an oxygen atom or the imino group, the two groups X being the same or different,

$R_1$ represents a chlorine or bromine atom,

$R_2$ and $R_3$, which may be the same or different, represent hydrogen atoms, straight-chained or branched alkyl groups with 1 to 6 carbon atoms which may be substituted by a phenyl group or a dialkylamino group with 1 to 3 carbon atoms in each alkyl moiety, or they may represent pyridyl or cycloalkyl groups with 5 to 7 carbon atoms or $R_2$ and $R_3$ together with the nitrogen atom between them may represent a pyrrolidino, piperidino, hexamethyleneimino, morpholino, N-arylpiperazino or N-alkylpiperazino group, wherein the alkyl group may contain 1 to 3 carbon atoms,

A represents a cycloalkylene group with 5 to 7 carbon atoms or an alkylene group with 2 to 10 carbon atoms which may be substituted by one or two alkyl groups each having 1 to 3 carbon atoms, or by one or two carbalkoxy groups each having a total of 2 to 4 carbon atoms, or by one or two phenyl groups, by 1 to 4 hydroxyl groups, by a halomethyl, hydroxymethyl, alkanoyloxy or alkanoyloxymethyl group each with 1 to 18 carbon

atoms in the alkanoyl moiety, or by a group of formula:

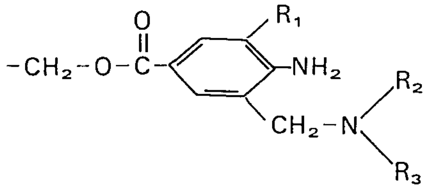

wherein $R_1$, $R_2$ and $R_3$ are as hereinbefore defined, or the alkylene group may be interrupted by an oxygen or sulphur atom, by a sulfinyl, sulfonyl, phenylene, cyclohexylene, pyridindiyl, piperazindiyl or imino group, whilst the imino group may be substituted by an alkyl group with 1 to 6 carbon atoms, or by a phenyl or phenylalkyl group with 1 to 3 carbon atoms in the alkyl moiety, and

B represents the 2-acetoxybenzoyl, 2-[(2,6-dichlorophenyl)amino]phenylacetyl, 1-(4-chlorobenzoyl)-5-methoxy-2-methyl-1H-indol-3-acetyl, 3-benzoyl-α-methylphenylacetyl, α-methyl-4-(2-methylpropyl)phenylacetyl, 2-fluoro-α-methyl-[1,1'-biphenyl]-4-acetyl, and (+)-6-methoxy-α-methyl-2-naphthaleneacetyl group, and the physiologically acceptable acid addition salts thereof with inorganic or organic acids, characterised in that a carboxylic acid of general formula:

$$B - OH \qquad (II)$$

or a salt or reactive derivative thereof, is reacted with a compound of general formula:

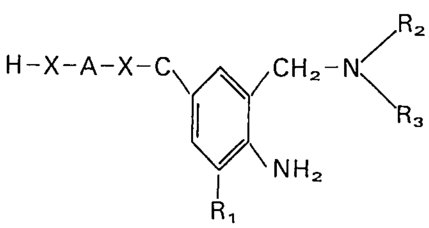

(III)

or an alkali metal salt or reactive derivative thereof, or a carboxylic acid of general formula:

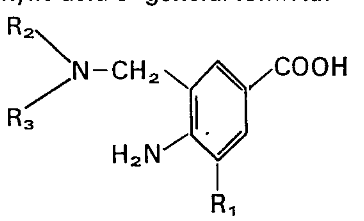

(IV)

or a salt or reactive derivative thereof is reacted with a compound of general formula:

$$H - X - A - X - B \qquad (V)$$

or an alkali metal salt or reactive derivative thereof wherein:

$R_1$ to $R_3$, A, B and X are as hereinbefore defined and the group HX together with the adjacent carbon atoms at the α- and β-positions of the group A may also represent an oxiranyl group,

and subsequently, if desired, a compound of general formula I obtained wherein A contains a hydroxyl groups is converted by acylation into the corresponding acyloxy compound of general formula I, or a compound of general formula I obtained wherein A represents an alkylene group interrupted by a sulphur atom is converted by oxidation into the corresponding sulfinyl or sulfonyl compound, or

a compound of general formula I obtained wherein A represents an alkylene group interrupted by a sulfinyl group is converted by oxidation into the corresponding sulfonyl compound, or

a compound of general formula I obtained is converted into the physiologically acceptable acid addition salts thereof with 1 to 3 equivalents of the appropriate inorganic or organic acid.

2. Process according to claim 1 for the preparation of new benzoyl derivatives of general formula:

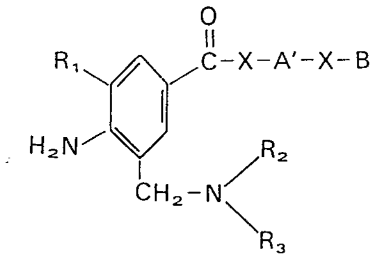

(Ia)

wherein:

$R_1$ to $R_3$, B and X are defined as in claim 1, and A' represents a cycloalkylene group with 5 to 7 carbon atoms or an alkylene group with 2 to 10 carbon atoms which may be substituted by one or two alkyl groups with 1 to 3 carbon atoms or by one or two carbalkoxy groups each with a total of 2 to 4 carbon atoms, or by 1 to 4 hydroxyl groups, or by a halomethyl, hydroxymethyl, alkanoyloxy or alkanoyloxymethyl group each with 1 to 18 carbon atoms in the alkanoyl moiety or by a group of formula:

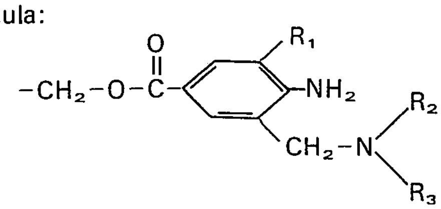

wherein $R_1$, $R_2$ and $R_3$ are as hereinbefore defined, or the alkylene may be interrupted by an oxygen or sulphur atom or by a sulfinyl, sulfonyl, piperazindiyl or imino group, whilst the imino group may be substituted by an alkyl group with 1 to 6 carbon atoms, or by a phenyl or phenylalkyl group with 1 to 3 carbon atoms in the alkyl moiety, and of the physiologically acceptable acid addition salts thereof with inorganic or organic acids, characterised in that a carboxylic acid of general formula:

$$B - OH \qquad (II)$$

or a salt or reactive derivative thereof, is reacted with a compound of general formula:

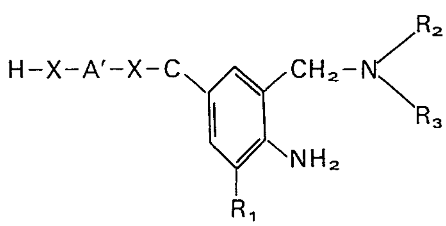

(IIIa)

or an alkali metal salt or reactive derivative thereof, or a carboxylic acid of general formula:

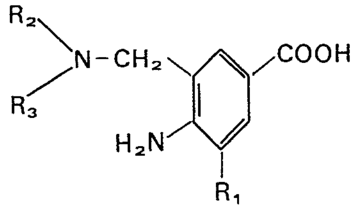

(IV)

or a salt or reactive derivative thereof, is reacted with a compound of general formula:

$$H - X - A' - X - B \qquad (Va)$$

or an alkali metal salt or reactive derivative thereof wherein:

$R_1$ to $R_3$, B and X are as defined in claim 1 hereinbefore, and

A′ is as hereinbefore defined,

and subsequently, if desired, a compound of general formula Ia wherein A′ contains a hydroxy group is converted by acylation into the corresponding acyloxy compound of general formula Ia, or a compound of general formula Ia obtained wherein A′ represents an alkylene group interrupted by a sulphur atom is converted by oxidation into the corresponding sulfinyl or sulfonyl compound, or

a compound of general formula Ia obtained wherein A′ represents an alkylene group interrupted by a sulfinyl group is converted by oxidation into the corresponding sulfonyl compound, or

a compound of general formula Ia obtained is converted into the physiologically acceptable acid addition salts thereof with 1 to 3 equivalents of the appropriate inorganic or organic acid.

3. Process as claimed in claims 1 and 2, characterised in that the reaction of a compound of general formula III or V or of the alkali metal salts thereof with a carboxylic acid of general formula II or IV or the functional derivatives thereof is carried out optionally in the presence of an acid-activating and/or dehydrating agent at temperatures of between −25 and 250°C.

4. Process as claimed in claims 1 and 2, characterised in that the reaction of a carboxylic acid of general formula II or IV or of the salts thereof with a reactive derivative of a compound of general formula III or V is effected at temperatures of between −25 and 250°C.

5. Process as claimed in claim 4, characterised in that the functional derivative of a carboxylic acid of general formula II or IV used is the ester, imidazolide, acid halide, anhydride or mixed anhydride thereof, the acyloxytriphenylphosphonium salt, N-acyloxyimide of isato acid anhydride thereof if the amino group is at the 2-position of a carboxylic acid of general formula IV.

6. Process as claimed in claim 5, characterised in that the reactive derivative of a compound of general formula III or V used is the phosphazo derivative, if X represents the imino group, or, if X represents an oxygen atom, the epoxide, halide or ester thereof with a sulphonic or carboxylic acid.

7. Process as claimed in claim 4, characterised in that the salt of a carboxylic acid of general formula II or IV used is the alkali metal salt, alkaline earth metal salt or silver salt.

8. Process as claimed in claims 1 to 7, characterised in that the reaction is effected in a solvent and at temperatures of between −10°C and the boiling point of the solvent used.

9. Process as claimed in claims 1 to 8, characterised in that the reaction is effected in the presence of an inorganic or tertiary organic base.

10. Process as claimed in claims 1 to 9, characterised in that the reaction is effected in the presence of a reaction accelerator such as sodium hydride, 4-dimethylaminopyridine, sodium iodide or potassium iodide or in the presence of a phase transfer catalyst such as a crown ether.

A′ is as hereinbefore defined,

and subsequently, if desired, a compound of general formula Ia wherein A′ contains a hydroxy group is converted by acylation into the corresponding acyloxy compound of general formula Ia, or a compound of general formula Ia obtained wherein A′ represents an alkylene group interrupted by a sulphur atom is converted by oxidation into the corresponding sulfinyl or sulfonyl compound, or

a compound of general formula Ia obtained wherein A′ represents an alkylene group interrupted by a sulfinyl group is converted by oxidation into the corresponding sulfonyl compound, or

a compound of general formula Ia obtained is converted into the physiologically acceptable acid addition salts thereof with 1 to 3 equivalents of the appropriate inorganic or organic acid.

3. Process as claimed in claims 1 and 2, characterised in that the reaction of a compound of general formula III or V or of the alkali metal salts thereof with a carboxylic acid of general formula II or IV or the functional derivatives thereof is carried out optionally in the presence of an acid-activating and/or dehydrating agent at temperatures of between −25 and 250°C.

4. Process as claimed in claims 1 and 2, characterised in that the reaction of a carboxylic acid of general formula II or IV or of the salts thereof with a reactive derivative of a compound of general formula III or V is effected at temperatures of between −25 and 250°C.

5. Process as claimed in claim 4, characterised in that the functional derivative of a carboxylic acid of general formula II or IV used is the ester, imidazolide, acid halide, anhydride or mixed anhydride thereof, the acyloxytriphenylphosphonium salt, N-acyloxyimide of isato acid anhydride thereof if the amino group is at the 2-position of a carboxylic acid of general formula IV.

6. Process as claimed in claim 5, characterised in that the reactive derivative of a compound of general formula III or V used is the phosphazo derivative, if X represents the imino group, or, if X represents an oxygen atom, the epoxide, halide or ester thereof with a sulphonic or carboxylic acid.

7. Process as claimed in claim 4, characterised in that the salt of a carboxylic acid of general

formula II or IV used is the alkali metal salt, alkaline earth metal salt or silver salt.

8. Process as claimed in claims 1 to 7, characterised in that the reaction is effected in a solvent and at temperatures of between −10°C and the boiling point of the solvent used.

9. Process as claimed in claims 1 to 8, characterised in that the reaction is effected in the presence of an inorganic or tertiary organic base.

10. Process as claimed in claims 1 to 9, characterised in that the reaction is effected in the presence of a reaction accelerator such as sodium hydride, 4-dimethylaminopyridine, sodium iodide or potassium iodide or in the presence of a phase transfer catalyst such as a crown ether.

**Revendications pour les Etats contractants:**
BE CH LI DE FR GB IT LU NL SE

1. Nouveaux dérivés benzoylés de formule générale:

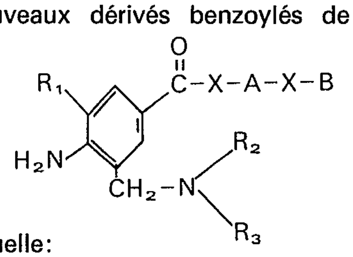

(I)

dans laquelle:

X représente un atome d'oxygène ou le groupe imino, les deux radicaux X pouvant être identiques ou différents,

$R_1$ représente un atome de chlore ou de brome,

$R_2$ et $R_3$, qui peuvent être identiques ou différents, représentent des atomes d'hydrogène, des groupes alcoyle rectilignes ou ramifiés avec 1 à 6 atomes de carbone, lesquels peuvent être substitués par un groupe phényle ou un groupe dialcoylamino avec 1 à 3 atomes de carbone dans chaque partie alcoyle, des groupes pyridyle ou cycloalcoyle avec 5 à 7 atomes de carbone, ou $R_2$ et $R_3$ représentent ensemble avec l'atome d'azote intermédiaire un groupe pyrrolidino, pipéridino, hexaméthylène-imino, morpholino, N-arylpipérazino ou N-alcoylpipérazino, le groupe alcoyle pouvant contenir 1 à 3 atomes de carbone,

A représente un groupe cycloalcoylène avec 5 à 7 atomes de carbone ou un groupe alcoylène avec 2 à 10 atomes de carbone qui peut être substitué par un ou deux groupes alcoyle avec à chaque fois 1 à 3 atomes de carbone, par un ou deux groupes carbalcoxy avec en tout dans chaque cas 2 à 4 atomes de carbone, par un ou deux groupes phényle, par 1 à 4 groupes hydroxyle, par un groupe halogénométhyle, hydroxyméthyle, alcanoyloxy ou alcanoyloxyméthyle avec à chaque fois 1 à 18 atomes de carbone dans la partie alcanoyle ou par un groupe de formule:

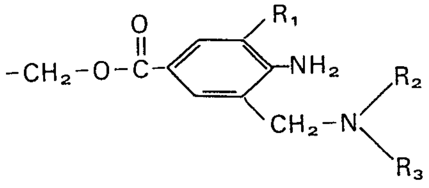

$R_1$, $R_2$ et $R_3$ étant définis comme au début, ou qui peut être interrompu par un atome d'oxygène ou de soufre, par un groupe sulfinyle, sulfonyle, phénylène, cyclohexylène, pyridinediyle, pipérazinediyle ou imino, le groupe imino pouvant être substitué par un groupe alcoyle avec 1 à 6 atomes de carbone, par un groupe phényle ou phénylalcoyle avec 1 à 3 atomes de carbone dans la partie alcoyle, et

B représente le groupe 2-acétoxybenzoyle, 2-[(2,6-dichlorophényl)amino]phénylacétyle, 1-(4-chlorobenzoyl)-5-méthoxy-2-méthyl-1H-indole-3-acétyle, 3-benzoyl-α-méthylphénylacétyle, α-méthyl-4-(2-méthylpropyl)phénylacétyle, 2-fluoro-α-méthyl-[1,1'-biphényl]-4-acétyle et (+)-6-méthoxy-α-méthyl-2-naphthalène-acétyle, et leurs sels d'addition d'acide physiologiquement supportables avec des acides minéraux ou organiques.

2. Nouveaux dérivés benzoylés de formule générale I selon la revendication 1, dans laquelle:

X représente un atome d'oxygène ou le groupe imino, les deux radicaux X pouvant être identiques ou différents,

$R_1$ représente un atome de chlore ou de brome,

$R_2$ et $R_3$, qui peuvent être identiques ou différents, représentent des atomes d'hydrogène, des groupes alcoyle avec 1 à 4 atomes de carbone, des groupes cycloalcoyle avec 5 à 7 atomes de carbone, des groupes benzyle ou 2-diéthylaminoéthyle, ou $R_2$ et $R_3$ représentent ensemble avec l'atome d'azote intermédiaire le groupe pyrrolidino, pipéridino, hexaméthylène-imino, morpholino, N-méthylpipérazino ou N-phénylpipérazino,

A représente un groupe alcoylène rectiligne avec 2 à 10 atomes de carbone, un groupe éthylène qui est substitué par un ou deux groupes méthyle, par un ou deux groupes alcoxycarbonyle avec en tout à chaque fois 2 ou 3 atomes de carbone, par un ou deux groupes phényle, par un groupe hydroxy, chlorométhyle, hydroxyméthyle, 4-amino-3-bromo-5-(N-éthylcyclohexylaminométhyl)benzoyloxyméthyle ou alcanoyloxyméthyle avec 1 à 18 atomes de carbone dans la partie alcanoyle ou représente un groupe propylène qui est substitué en position 2 par un groupe hydroxy ou un groupe alcanoyloxy avec 1 à 18 atomes de carbone ou par deux groupes alcoyle avec chacun 1 à 3 atomes de carbone, ou représente un groupe n-hexylène substitué par 4 groupes hydroxy, ou représente un groupe cyclohexylène ou un groupe alcoylène rectiligne avec 4 à 6 atomes de carbone, lequel est interrompu entre les atomes de carbone 2 et 3 ou 3 et 4 par un atome d'oxygène ou de soufre, par un groupe sulfinyle, sulfonyle, imino, phénylimino, benzylimino, pipérazinediyle ou un groupe alcoylimino avec 1 à 4 atomes de carbone, ou représente un groupe 1,4-cyclohexanediméthylène, p-xylylène ou 2,6-pyridinediméthylène, et

B représente le groupe 2-[(2,6-dichlorophényl)amino]phénylacétyle, 1-(4-chlorobenzoyl)-5-méthoxy-2-méthyl-1H-indole-3-acétyle, 3-benzoyl-α-méthylphénylacétyle, α-méthyl-4-(2-méthylpropyl)phénylacétyle, 2-fluoro-α-mé-

thyl-[1,1'-biphényl]-4-acétyle et (+)-6-méthoxy-α-méthyl-2-naphthalène-acétyle, et leurs sels d'addition d'acide physiologiquement supportables avec des acides minéraux ou organiques.

3. Nouveaux dérivés benzoylés de formule générale I selon la revendication 1, dans laquelle:

X représente dans chaque cas un atome d'oxygène,

$R_1$ représente un atome de brome,

$R_2$ représente le groupe méthyle ou éthyle,

$R_3$ représente le groupe éthyle ou cyclohexyle ou $R_2$ et $R_3$ représentent ensemble avec l'atome d'azote le groupe hexaméthylène-imino,

A représente un groupe alcoylène rectiligne avec 2 à 6 atomes de carbone ou un groupe alcoylène rectiligne avec 4 à 6 atomes de carbone qui est interrompu entre les atomes de carbone 2 et 3 ou 3 et 4 par un atome d'oxygène ou de soufre, par un groupe éthylimino ou propylimino, et

B représente le groupe 1-(4-chlorobenzoyl)-5-méthoxy-2-méthyl-1H-indole-3-acétyle, 2-[(2,6-dichlorophényl)amino]phénylacétyle, 3-benzoyl-α-méthylphénylacétyle, (+)-6-méthoxy-α-méthyl-2-naphthalène-acétyle, α-méthyl-4-(2-méthylpropyl)phénylacétyle ou 2-fluoro-α-méthyl-[1,1'-biphényl]-4-acétyle, et leurs sels physiologiquement supportables avec des acides minéraux ou organiques.

4. Nouveaux dérivés benzoylés de formule générale I selon la revendication 1, dans laquelle:

$R_1$, $R_2$, $R_3$ et A sont définis comme dans la revendication 3, et

B représente le groupe 1-(4-chlorobenzoyl)-5-méthoxy-2-méthyl-1H-indole-3-acétyle ou 2-[(2,6-dichlorophényl)amino]phénylacétyle, et leurs sels physiologiquement supportables avec des acides minéraux ou organiques.

5. Le 1-[4-amino-3-bromo-5-(N-éthylcyclohexylaminométhyl)benzoyloxy]-2-[1-(4-chlorobenzoyl)-5-méthoxy-2-méthyl-1H-indole-3-acétoxy]éthane et ses sels physiologiquement supportables d'addition d'acide.

6. Le 1-[4-amino-3-bromo-5-(N-éthylcyclohexylaminométhyl)benzoyloxy]-5-{2-[(2,6-dichlorophényl)amino]phénylacétoxy}-n-pentane et ses sels physiologiquement supportables d'addition d'acide.

7. Le 1-(4-amino-3-bromo-5-diéthylaminométhylbenzoyloxy)-2-[1-(4-chlorobenzoyl)-5-méthoxy-2-méthyl-1H-indole-3-acétoxy]éthane et ses sels physiologiquement supportables d'addition d'acide.

8. Médicament contenant un composé selon les revendications 1 à 7 conjointement à un ou plusieurs excipients ou diluants inertes.

9. Médicament selon la revendication 8 pour lutter contre les processus inflammatoires.

10. Procédé de préparation de nouveaux dérivés benzoylés selon les revendications 1 à 7 et de leurs sels d'addition d'acide physiologiquement supportables avec des acides minéraux ou organiques, caractérisé en ce que l'on fait réagir un acide carboxylique de formule générale:

$$B - OH \qquad (II)$$

ses sels ou dérivés réactifs avec un composé de formule générale:

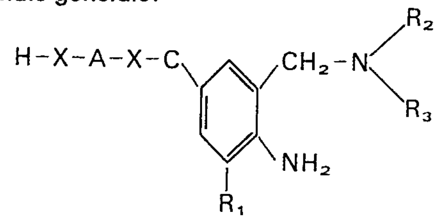

(III)

ses sels alcalins ou dérivés réactifs ou un acide carboxylique de formule générale:

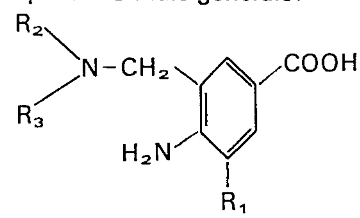

(IV)

ses sels ou dérivés réactifs avec un composé de formule générale:

$$H - X - A - X - B \qquad (V)$$

ses sels alcalins ou dérivés réactifs, dans lesquels $R_1$ à $R_3$, A, B et X sont définis comme au début, et le groupe HX peut représenter ensemble avec les atomes de carbone voisins en positions α et β du radical A également un groupe oxirannyle, et

en ce que, si on le désire, on transforme ensuite un composé obtenu de formule générale I, dans laquelle A contient un groupe hydroxyle, au moyen d'une acylation en le composé acyloxy correspondant de formule générale I ou en ce que l'on transforme un composé obtenu de formule générale I, dans laquelle A représente un groupe alcoylène interrompu par un atome de soufre, au moyen d'une oxydation en le composé sulfinylé ou sulfonylé correspondant, ou

en ce que l'on transforme un composé obtenu de formule générale I, dans laquelle A représente un groupe alcoylène interrompu par un groupe sulfinyle, au moyen d'une oxydation en le composé sulfonylé correspondant ou en ce que l'on transforme un composé obtenu de formule générale I en ses sels d'addition d'acide physiologiquement supportables avec 1 à 3 équivalents de l'acide minéral ou organique correspondant.

**Revendications pour l'Etat contractant:** AT

1. Procédé de préparation de nouveaux dérivés benzoylés de formule générale:

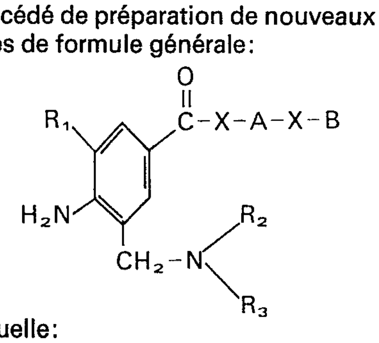

(I)

dans laquelle:

X représente un atome d'oxygène ou le groupe

imino, les deux radicaux X pouvant être identiques ou différents,

$R_1$ représente un atome de chlore ou de brome,

$R_2$ et $R_3$, qui peuvent être identiques ou différents, représentent des atomes d'hydrogène, des groupes alcoyle rectilignes ou ramifiés avec 1 à 6 atomes de carbone lesquels peuvent être substitués par un groupe phényle ou un groupe dialcoylamino avec 1 à 3 atomes de carbone dans chaque partie alcoyle, des groupes pyridyle ou cycloalcoyle avec 5 à 7 atomes de carbone, ou $R_2$ et $R_3$ représentent ensemble avec l'atome d'azote intermédiaire un groupe pyrrolidino, pipéridino, hexaméthylène-imino, morpholino, N-arylpipérazino ou N-alcoylpipérazino, le groupe alcoyle pouvant contenir 1 à 3 atomes de cabone,

A représente un groupe cycloalcoylène avec 5 à 7 atomes de carbone ou un groupe alcoylène avec 2 à 10 atomes de carbone qui peut être substitué par un ou deux groupes alcoyle avec à chaque fois 1 à 3 atomes de carbone, par un ou deux groupes carbalcoxy avec en tout à chaque fois 2 à 4 atomes de carbone, par un ou deux groupes phényle, par 1 à 4 groupes hydroxyle, par un groupe halogénométhyle, hydroxyméthyle, alcanoyloxy ou alcanoyloxyméthyle avec à chaque fois 1 à 18 atomes de carbone dans la partie alcanoyle ou par un groupe de formule:

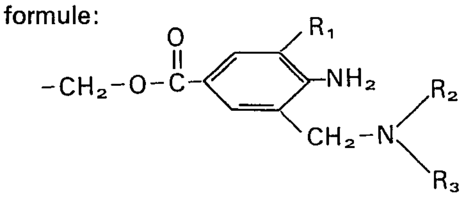

$R_1$, $R_2$ et $R_3$ étant définis comme au début, ou qui peut être interrompu par un atome d'oxygène ou de soufre, par un groupe sulfinyle, sulfonyle, phénylène, cyclohexylène, pyridinediyle, pipérazinediyle ou imino, le groupe imino pouvant être substitué par un groupe alcoyle avec 1 à 6 atomes de carbone, par un groupe phényle ou phénylalcoyle avec 1 à 3 atomes de carbone dans la partie alcoyle, et

B représente le groupe 2-acétoxybenzoyle, 2-[(2,6-dichlorophényl)amino]phénylacétyle, 1-(4-chlorobenzoyl)-5-méthoxy-2-méthyle-1H-indole-3-acétyle, 3-benzoyl-α-méthylphénylacétyle, α-méthyl-4-(2-méthylpropyl)phénylacétyle, 2-fluoro-α-méthyl-[1,1'-biphényl]-4-acétyle et (+)-6-méthoxy-α-méthyl-2-naphthalène-acétyle, et de leurs sels d'addition d'acide physiologiquement supportables avec des acides minéraux ou organiques, caractérisé en ce que l'on fait réagir un acide carboxylique de formule générale:

$$B - OH \qquad (II)$$

ses sels ou dérivés réactifs avec un composé de formule générale:

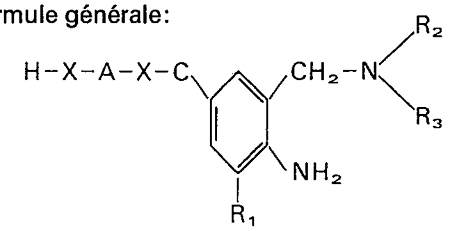

(III)

ses sels alcalins ou dérivés réactifs ou un acide carboxylique de formule générale:

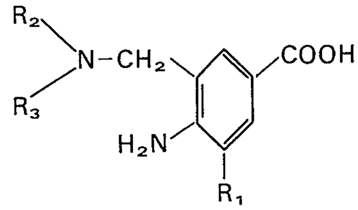

(IV)

ses sels ou dérivés réactifs avec un composé de formule générale:

$$H - X - A - X - B \qquad (V)$$

ses sels alcalins ou dérivés réactifs, dans lesquels $R_1$ à $R_3$, A, B et X sont définis comme au début et le groupe HX peut représenter ensemble avec les atomes de carbone voisins en positions α et β du radical A également un groupe oxirannyle,

et en ce qu'éventuellement on transforme ensuite un composé obtenu de formule générale I, dans laquelle A contient un groupe hydroxyle, au moyen d'une acylation en le composé acyloxy correspondant de formule générale I, ou on transforme un composé obtenu de formule générale I, dans laquelle A représente un groupe alcoylène interrompu par un atome de soufre, au moyen d'une oxydation en le composé sulfinylé ou sulfonylé correspondant, ou

on transforme un composé obtenu de formule générale I, dans laquelle A représente un groupe alcoylène interrompu par un groupe sulfinyle, au moyen d'une oxydation en le composé sulfonylé correspondant, ou on transforme un composé obtenu de formule générale I en ses sels d'addition d'acide physiologiquement supportables avec 1 à 3 équivalents de l'acide minéral ou organique correspondant.

2. Procédé selon la revendication 1 de préparation de nouveaux dérivés benzoylés de formule générale:

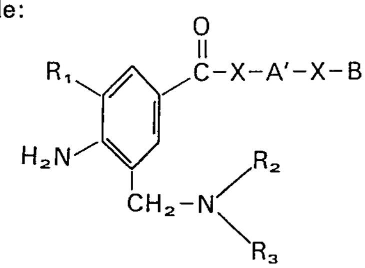

(Ia)

dans laquelle:

$R_1$ à $R_3$, B et X sont définis comme dans la revendication 1, et

A' représente un groupe cycloalcoylène avec 5 à 7 atomes de carbone ou un groupe alcoylène avec 2 à 10 atomes de carbone qui peut être substitué par un ou deux groupes alcoyle avec 1 à 3 atomes de carbone, par un ou deux groupes carbalcoxy avec en tout dans chaque cas 2 à 4 atomes de carbone, par 1 à 4 groupes hydroxyle, par un groupe halogénométhyle, hydroxyméthyle, alcanoyloxy ou alcanoyloxyméthyle avec dans chaque cas 1 à 18 atomes de carbone dans la partie alcanoyle, ou par un groupe de formule:

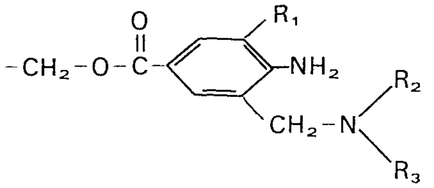

dans laquelle $R_1$, $R_2$ et $R_3$ sont définis comme au début, ou qui peut être interrompu par un atome d'oxygène ou de soufre, par un groupe sulfinyle, sulfonyle, pipérazinediyle ou imino, le groupe imino pouvant être substitué par un groupe alcoyle avec 1 à 6 atomes de carbone, par un groupe phényle ou phénylalcoyle avec 1 à 3 atomes de carbone dans la partie alcoyle, et de leurs sels d'addition d'acide physiologiquement supportables avec des acides minéraux ou organiques, caractérisé en ce que l'on fait réagir un acide carboxylique de formule générale:

$$B - OH \qquad (II)$$

ses sels ou dérivés réactifs avec un composé de formule générale:

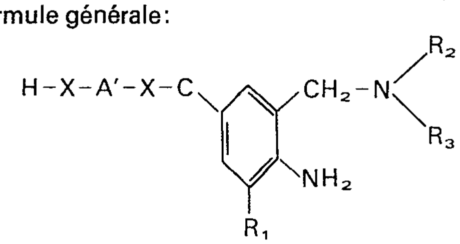

(IIIa)

ses sels alcalins ou dérivés réactifs ou un acide carboxylique de formule générale:

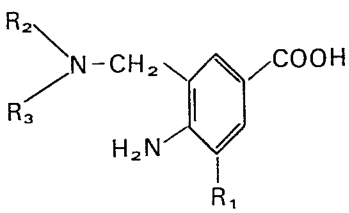

(IV)

ses sels ou dérivés réactifs avec un composé de formule générale:

$$H - X - A' - X - B \qquad (Va)$$

ses sels alcalins ou dérivés réactifs, dans lesquels $R_1$ à $R_3$, B et X sont définis comme dans la revendication 1 au début, et

A' est défini comme plus haut,

et en ce que, si on le désire, on transforme ensuite un composé obtenu de formule générale Ia, dans laquelle A' contient un groupe hydroxyle, au moyen d'une acylation en le composé acyloxy correspondant de formule générale Ia ou on transforme un composé obtenu de formule générale Ia, dans laquelle A' représente un groupe alcoylène interrompu par un atome de soufre, au moyen d'une oxydation en le composé sulfinylé ou sulfonylé correspondant, ou on transforme un composé obtenu de formule générale Ia, dans laquelle A' représente un groupe alcoylène interrompu par un groupe sulfinyle, au moyen d'une oxydation en le composé sulfonylé correspondant, ou en ce que l'on transforme un composé obtenu de formule générale Ia en ses sels d'addition d'acide physiologiquement supportables au moyen de 1 à 3 équivalents de l'acide minéral ou organique correspondant.

3. Procédé selon les revendications 1 et 2, caractrisé en ce que la réaction d'un composé de formule générale III ou V ou de ses sels alcalins avec un acide carboxylique de formule générale II ou IV ou ses dérivés fonctionnels a lieu à des températures comprises entre −25 et 250° C et éventuellement en présence d'un agent d'activation des acides et/ou d'élimination de l'eau.

4. Procédé selon les revendications 1 et 2, caractérisé en ce que la réaction d'un acide carboxylique de formule générale II ou IV ou de ses sels avec un dérivé réactif d'un composé de formule générale III ou V a lieu à des températures comprises entre −25 et 250° C.

5. Procédé selon la revendication 4, caractérisé en ce que l'on utilise en tant que dérivé fonctionnel d'un acide carboxylique de formule générale II ou IV ses ester, imidazolide, halogénure d'acide, son anhydride ou son anhydride mixte, son sel d'acyloxytriphénylphosphonium, son N-acyloxyimide ou, également, son anhydride d'acide isatoïque, lorsque le groupe amino est situé en position 2 d'un acide carboxylique de formule générale IV.

6. Procédé selon la revendication 5, caractérisé en ce que l'on utilise en tant que dérivé réactif d'un composé de formule générale III ou V son dérivé phosphazo, dans le cas où X représente le groupe imino ou dans le cas où X représente un atome d'oxygène son époxyde, son halogénure ou son ester avec un acide sulfonique ou carboxylique.

7. Procédé selon la revendication 4, caractérisé en ce que l'on utilise en tant que sel d'un acide carboxylique de formule générale II ou IV le sel alcalin, alcalino-terreux ou d'argent.

8. Procédé selon les revendications 1 à 7, caractérisé en ce que la réaction a lieu dans un solvant et à des températures comprises entre −10° C et le point d'ébullition du solvant utilisé.

9. Procédé selon les revendications 1 à 8, caractérisé en ce que la réaction a lieu en présence d'une base minérale ou organique tertiaire.

10. Procédé selon les revendications 1 à 9, caractérisé en ce que la réaction a lieu en présence d'un accélérateur de réaction tel que l'hydrure de sodium, la 4-diméthylaminopyridine, l'iodure de sodium ou l'iodure de potassium ou en présence d'un catalyseur de transfert de phases tel qu'un éther couronne.